# EUROPEAN PATENT APPLICATION

(11) **EP 2 407 548 A1**
(43) Date of publication of application: **18.01.2012**
(21) Application number: 11179956.5
(22) Date of filing: 16.10.2007
(51) Int. Cl.: C12P 21/08

(54) **Molecules with reduced half-lives, compositions and uses thereof**

(30) Priority: 16.10.2006 US 851739 P
(62) Divisional of application: 07852754.6
(71) Applicant: MedImmune, LLC, Gaithersburg, MD 20878 (US)
(72) Inventor: Dall'Acqua, William, Gaithersburg, MD 20878 (US); Wu, Herren, Boyds, MD 20841 (US)
(74) Representative: Myers, Jonathan David

(57) **Abstract**

The present invention provides polypeptides containing at least the FcRn binding portion of an Fc region of an immunoglobulin molecule and that have altered amino acid sequences relative to wild type immunoglobulin molecules. The polypeptides have decreased in vivo serum half-lives and can be employed in various methods.

## Description

### 1. FIELD OF THE INVENTION

The present invention relates to immunoglobulin polypeptides with reduced serum half lives. The immunoglobulin polypeptides contain a portion of an immunoglobulin that binds to FcRn and that is modified to have at least one amino acid substitution relative to a wild type counterpart immunoglobulin.

### 2. BACKGROUND OF THE INVENTION

The present invention encompasses polypeptides that comprise at least a portion of an immunoglobulin constant that binds to an FcRn and that contain one or more amino acid modifications relative to a wild type immunoglobulin constant domain. The at least one amino acid modification decreases the affinity of the immunoglobulin constant domain, or fragment thereof, for the FcRn and reduces the serum half life of the polypeptides. The polypeptides have particular use in, e.g., therapy, prophylaxis, diagnosis, and prognosis of diseases or disorders.

### 3. SUMMARY OF THE INVENTION

One embodiment of the invention is polypeptide containing at least an FcRn binding portion of an Fc region of an immunoglobulin molecule. The polypeptide contains at least one amino acid modification relative to a wild type immunoglobulin molecule.

Another embodiment of the invention is a method of diagnosing, prognosing, monitoring, or treating a disease or disorder. The method includes a step of administering the polypeptide that contains at least an FcRn binding portion of an Fc region of an immunoglobulin molecule and that contains at least one amino acid modification relative to a wild type immunoglobulin molecule.

### 4. BRIEF DESCRIPTION OF THE FIGURES

The patent or application file contains at least one drawing executed in color. Copies of this patent or patent application publication with color drawings will be provided by the Office upon request and payment of the necessary fee.

Figure 1: MEDI-524 constant region amino acid sequence (SEQ ID NO:1) showing location of amino acid substitutions. Amino acid substitutions were made in the constant region at positions L251, M252, S254, R255, T256, V308, L309, Q311, D312, G385, Q386, P387, N389, H433, N434, and Y436 (shown in gray and underlined).

Figure 2: Comparative binding of deoptimized Fc mutants to immobilized human FcRn at pH 6.0. This is a representative sensorgram showing select mutants and repetitive wt (MEDI-524) injections.

Figure 3: Human FcRn binding of deoptimized IgG mutants at pH 6.0. RUs were determined for each mutant as it was flowed over immobilized human FcRn.

Figure 4: Binding of G385P, P387V, N389G, N389S, and G385S+Q3861 to human FcRn surface at pH 7.4. Binding of wild type (MEDI-524) at pH 6 is shown for comparison.

Figure 5: Binding of N434L, Y436T, L251S, M252T, and M252S to human FcRn surface at pH 7.4. Binding of wild type (MEDI-524) at pH 6 is shown for comparison.

Figure 6: Binding of S254W, S254R, R255V, T256L to human FcRn surface at pH 7.4. Binding of wild type (MEDI-524) at pH 6 is shown for comparison.

Figure 7: Binding of L309W, L309N, Q311G, D312G, and D312I to human FcRn surface at pH 7.4. Binding of wild type (MEDI-524) at pH 6 is shown for comparison.

Figure 8: Binding of L309F+Q311L, L309E+Q311V, L309R+Q311W, G385W, T256W to human FcRn surface at pH 7.4. Binding of wild type (MEDI-524) at pH 6 is shown for comparison.

Figure 9: Binding of L309W, Q386V, Q386L, P387G, H433L to human FcRn surface at pH 7.4. Binding of wild type (MEDI-524) at pH 6 is shown for comparison.

Figure 10: Binding of Y436I, R255T, T256H, N434S+Y436S, M252W, and N434G to human FcRn surface at pH 7.4. Binding of wild type (MEDI-524) at pH 6 is shown for comparison.

Figure 11: Binding of varying concentrations of human FcRn to MEDI-524 (wt) surface at pH 6.0.

Figure 12: Binding of human FcRn and ovalbumin to MEDI-524 (wt) surface at pH 6.0 and 7.4.

Figure 13: Binding of varying concentrations of human FcRn to mutant G385S + Q386I surface at pH 6.0.

Figure 14: Binding of human FcRn and ovalbumin to mutant G385S + Q386I surface at pH 6.0 and 7.4.

Figure 15: Binding of varying concentrations of human FcRn to mutant L309N surface at pH 6.0.

Figure 16: Binding of human FcRn and ovalbumin to mutant L309N surface at pH 6.0 and 7.4.

Figure 17: Binding of human FcRn and ovalbumin to mutant L251S surface at pH 6.0 and 7.4.

Figure 18: Binding of human FcRn and ovalbumin to mutant N434L surface at pH 6.0 and 7.4.

Figure 19: Binding of human FcRn and ovalbumin to mutant M252T surface at pH 6.0 and 7.4

Figure 20: Binding of human FcRn and ovalbumin to mutant S254W surface at pH 6.0 and 7.4.

Figure 21: Binding of human FcRn and ovalbumin to mutant S254R surface at pH 6.0 and 7.4.

Figure 22: ELISA data for the binding of mutants N434L (D), G385S + Q386I (A), and M252T (◊) to human FcRn at acidic pH. All mutants show reduced binding when compared to Numax ((MEDI-524) (●).

Figure 23: ELISA data for the binding of mutants S254W (■), S254R (▲), and L309N (◆) to human FcRn at acidic pH. All mutants show reduced binding when compared to Numax ((MEDI-524) (●).

### 5. DETAILED DESCRIPTION

### 5.1 DEFINITIONS

The term "IgG Fc region" as used herein refers the portion of an IgG molecule that correlates to a crystallizable fragment obtained by papain digestion of an IgG molecule. The Fc region consists of the C-terminal half of the two heavy chains of an IgG molecule that are linked by disulfide bonds. It has no antigen binding activity but contains the carbohydrate moiety and the binding sites for complement and Fc receptors, including the FcRn receptor. The Fc fragment is the portion of a heavy chain constant region of an antibody beginning N-terminal of the hinge region at the papain cleavage site, at about position 216 according to the EU index as in Kabat (*see* Kabat al., Sequences of Proteins of Immunological Interest, 5th ed., 1991 NIH Pub. No. 91-3242) and including the hinge, CH2, and CH3 domains.

The term "IgG hinge-Fc region" or "hinge-Fc fragment" as used herein refers to a region of an IgG molecule consisting of the Fc region (residues 231-446) and a hinge region (residues 216-230) extending from the N-terminus of the Fc region.

The "CH2 domain" includes the portion of a heavy chain molecule that extends, e.g., from about EU positions 231-340. the CH2 domain is unique in that it is not closely paired with another domain. Rather, two N-linked branched carbohydrate chains are interposed between the two CH2 domains of an intact native IgG molecule.

The "CH3 domain" includes the portion of a heavy chain molecule that extends approximately 110 residues from the C-terminus of the CH2 domain, e.g., from about residue 341-446, EU numbering system). The CH3 domain typically forms the C-terminal portion of an antibody. In some immunoglobulins, however, additional domains may extend from CH3 domain to form the C-terminal portion of the molecule (e.g., the CH4 domain in the µ chain of IgM and the ε chain of IgE).

The term "constant domain" refers to the portion of an immunoglobulin molecule having a more conserved amino acid sequence relative to the other portion of the immunoglobulin, the variable domain, which contains the antigen binding site. The constant domain contains the CH1, CH2 and CH3 domains of the heavy chain and the CHL domain of the light chain.

A "fusion protein" refers to a chimeric polypeptide which comprising a first polypeptide linked to a second polypeptide with which it is not naturally linked in nature. For example, a fusion protein may comprise an amino acid sequence encoding at least a portion of an Fc region (e.g., the portion of the Fc region that confers binding to FcR) and an amino acid sequence encoding a non-immunoglobulin polypeptide, e.g., a ligand binding domain of a receptor or a receptor binding domain of a ligand. The amino acid sequence may normally exist in separate protein that are brought together in the fusion polypeptide or they may normally exist in the same protein but are placed in a new arrangement in the fusion polypeptide. A fusion protein may be created, for example, by chemical synthesis, or by creating and translating a polynucleotide in which the peptide regions are encoded in the desired relationship.

"Linked," "fused," or "fusion" are used interchangeably. These terms refer to the joining together of two or more elements or components, by whatever means, including chemical conjugation or recombinant means. An "in-frame fusion" or "operably linked" refers to the joining of two or more open reading frames (ORFs) to form a continuous longer ORF, in a manner that maintains the correct reading frame of the original ORFs. Thus, the resulting recombinant fusion protein is a single protein containing two or more segments that correspond to polypeptides encoded by the original ORFs (which segments are not normally so joined in nature). Although the reading frame is thus made continuous throughout the fused segments, the segments may be physically or spatially separated by, for example, an in-frame linker sequence.

The term "FcRn receptor" or "FcRn" as used herein refers to an Fc receptor ("n" indicates neonatal) which is known to be involved in transfer of maternal IgGs to a fetus through the human or primate placenta, or yolk sac (rabbits) and to a neonate from the colostrum through the small intestine. It is also known that FcRn is involved in the maintenance of constant serum IgG levels by binding the IgG molecules and recycling them into the serum. The binding of FcRn to IgG molecules is strictly pH-dependent with optimum binding at pH 6.0. FcRn comprises a heterodimer of two polypeptides, whose molecular weights are approximately 50 kD and 15 kD, respectively. The extracellular domains of the 50 kD polypeptide are related to major histocompatibility complex (MHC) class I α-chains and the 15 kD polypeptide was shown to be the non-polymorphic β₂-microglobulin (β₂-microglobulin). In addition to placenta and neonatal intestine, FcRn is also expressed in various tissues across species as well as various types of endothelial cell lines. It is also expressed in human adult vascular endothelium, muscle vasculature and hepatic sinusoids and it is suggested that the endothelial cells may be most responsible for the maintenance of serum IgG levels in humans and mice.

The term "in vivo half-life" as used herein refers to a biological half-life of a particular type of IgG molecule or its fragments containing FcRn-binding sites in the circulation of a given animal and is represented by a time required for half the quantity administered in the animal to be cleared from the circulation and/or other tissues in the animal. When a clearance curve of a given IgG is constructed as a function of time, the curve is usually biphasic with a rapid α-phase which represents an equilibration of the injected IgG molecules between the intra- and extra-vascular space and which is, in part, determined by the size of molecules, and a longer β-phase which represents the catabolism of the IgG molecules in the intravascular space. The term "in vivo half-life" practically corresponds to the half life of the IgG molecules in the β-phase.

An "isolated" or "purified" polypeptide comprising at least an FcRn binding portion of an Fc region of an IgG molecule is substantially free of cellular material or other contaminating proteins from the cell or tissue source from which the protein is derived, or substantially free of chemical precursors or other chemicals when chemically synthesized. The language "substantially free of cellular material" includes preparations of the polypeptide in which the polypeptide is separated from cellular components of the cells from which it is isolated or recombinantly produced. Thus, a polypeptide that is substantially free of cellular material includes preparations of antibody, antibody fragment, or antibody or antibody fragment fusion proteins having less than about 30%, 20%, 10%, or 5% (by dry weight) of contaminating protein. When the polypeptide is recombinantly produced, it is also preferably substantially free of culture medium, i.e., culture medium represents less than about 20%, 10%, or 5% of the volume of the protein preparation. When the polypeptide is produced by chemical synthesis, it is preferably substantially free of chemical precursors or other chemicals, i.e., it is separated from chemical precursors or other chemicals which are involved in the synthesis of the protein. Accordingly such preparations of the polypeptide have less than about 30%, 20%, 10%, 5% (by dry weight) of chemical precursors or compounds other than the polypeptide of interest. The polypeptides encompassed by the invention may be isolated or purified.

An "isolated" nucleic acid molecule is one which is separated from other nucleic acid molecules which are present in the natural source of the nucleic acid molecule. Moreover, an "isolated" nucleic acid molecule, such as a cDNA molecule, can be substantially free of other cellular material, or culture medium when produced by recombinant techniques, or substantially free of chemical precursors or other chemicals when chemically synthesized. An "isolated" nucleic acid molecule does not include cDNA molecules within a cDNA library. Nucleic acid molecules encoding antibodies may be isolated or purified. Nucleic acid molecules encoding fusion proteins may be isolated or purified.

The term "host cell" as used herein refers to the particular subject cell transfected with a nucleic acid molecule or infected with phagemid or bacteriophage and the progeny or potential progeny of such a cell. Progeny of such a cell may not be identical to the parent cell transfected with the nucleic acid molecule due to mutations or environmental influences that may occur in succeeding generations or integration of the nucleic acid molecule into the host cell genome.

The names of amino acids referred to herein are abbreviated either with three-letter or one-letter symbols.

To determine the percent identity of two amino acid sequences or of two nucleic acid sequences, the sequences are aligned for optimal comparison purposes (e.g., gaps can be introduced in the sequence of a first amino acid or nucleic acid sequence for optimal alignment with a second amino acid or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (i.e., % identity=number of identical overlapping positions/total number of positionsx100%). In one embodiment, the two sequences are the same length.

The determination of percent identity between two sequences can also be accomplished using a mathematical algorithm. A preferred, non-limiting example of a mathematical algorithm utilized for the comparison of two sequences is the algorithm of Karlin and Altschul, 1990, Proc. Natl. Acad. Sci. U.S.A. 87:2264-2268, modified as in Karlin and Altschul, 1993, Proc. Natl. Acad. Sci. U.S.A. 90:5873-5877. Such an algorithm is incorporated into the NBLAST and XBLAST programs of Altschul et al., 1990, J. Mol. Biol. 215:403. BLAST nucleotide searches can be performed with the NBLAST nucleotide program parameters set, e.g., for score=100, wordlength=12 to obtain nucleotide sequences homologous to a nucleic acid molecules of the present invention. BLAST protein searches can be performed with the XBLAST program parameters set, e.g., to score-50, wordlength=3 to obtain amino acid sequences homologous to a protein molecule of the present invention. To obtain gapped alignments for comparison purposes, Gapped BLAST can be utilized as described in Altschul et al., 19 97, Nucleic Acids Res. 25:3389-3402. Alternatively, PSI-BLAST can be used to perform an iterated search which detects distant relationships between molecules (Id.). When utilizing BLAST, Gapped BLAST, and PSI-Blast programs, the default parameters of the respective programs (e.g., of XBLAST and NBLAST) can be used (see, e.g., http://www.ncbi.nlm.nih.gov). Another preferred, non-limiting example of a mathematical algorithm utilized for the comparison of sequences is the algorithm of Myers and Miller, 1988, CABIOS 4:11-17. Such an algorithm is incorporated in the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used.

The percent identity between two sequences can be determined using techniques similar to those described above, with or without allowing gaps. In calculating percent identity, typically only exact matches are counted.

### 5.2 POLYPEPTIDES WITH DECREASED IN VIVO HALF-LIVES

The invention encompasses polypeptides with reduced in vivo half-lives. The polypeptides contain at least an FcRn binding portion of an Fc region of an immunoglobulin G (IgG) molecule and further contain at least amino acid residue substitution relative to a wild type IgG molecule. The polypeptides may be IgG antibodies, a constant domain of an IgG antibody, a portion of an IgG antibody constant domain, e.g., about amino acid residues 216-446 or about amino acid residues 231-446 according to the EU numbering system as in Kabat (*see* Kabat et al., Sequences of Proteins of Immunological Interest, 5th ed., 1991 NIH Pub. No. 91-3242), or any other fragment of an IgG molecule capable of binding FcRn. These molecules can be used to diagnose, monitor, or prognose a disease or disorder. If the molecules are conjugated to a detectable moiety for an imaging procedure, shorter serum half life may improve the quality of any of the image obtained from the procedure. Additionally, use of these molecules will reduce exposure of the individual subjected to the imaging procedure to the polypeptide and the detectable moiety. These molecules may also be desirable to shorten the serum half life of therapeutics having less than optimal toxicity profiles. Furthermore, these molecules may be preferable for treatment during pregnancy or to alter the biodistribution of the molecule. The polypeptides with reduced serum half life may preferentially distribute to the kidney or liver.

The polypeptides contain portions of an IgG constant domain that interact with FcRn and that are modified, relative to a corresponding IgG constant domain or portion thereof, to have decreased affinity for FcRn. The one or more amino acid modifications can be made in one or more of residues 251, 252, 254, 255, 309, 312, 386, 434, or the combination of 385 and 386. In one embodiment, the amino acid modifications are made in a human IgG constant domain, or FcRn-binding domain thereof.

The amino acid modifications may be any modification, including at one or more of residues 251, 252, 254, 255, 309, 312, 386, 434, or the combination of 385 and 386, that decreases the in vivo half-life of the IgG constant domain, or FcRn-binding fragment thereof (e.g., Fc or hinge-Fc domain), and any molecule attached thereto, and decreases the affinity of the IgG, or fragment thereof, for FcRn. In other embodiments, the modifications may alter (i.e., increase or decrease) bioavailability of the molecule, or may alter (i.e., increase or decrease) transport (or concentration or half-life) of the molecule to mucosal surfaces (e.g., of the lungs) or other portions of a target tissue. The amino acid modifications may further alter transport or concentration or half-life of the molecule to the lungs. In other embodiments, the amino acid modifications may alter transport (or concentration or half-life) of the molecule to the heart, pancreas, liver, kidney, bladder, stomach, large or small intestine, respiratory tract, lymph nodes, nervous tissue (central and/or peripheral nervous tissue), muscle, epidermis, bone, cartilage, joints, blood vessels, bone marrow, prostate, ovary, uterine, tumor or cancer tissue, etc. where the molecule may not be needed, e.g., due to toxicity. In another embodiment, the modifications decrease the bioavailability of the molecule to the heart, pancreas, liver, kidney, bladder, stomach, large or small intestine, respiratory tract, lymph nodes, nervous tissue (central and/or peripheral nervous tissue), muscle, epidermis, bone, cartilage, joints, blood vessels, bone marrow, prostate, ovary, uterine, tumor or cancer tissue. In another embodiment, the amino acid modifications do not abolish, or do not alter, other immune effector or receptor binding functions of the constant domain, for example, but not limited to complement fixation, ADCC and binding to FcγRI, FcγRII, and FcγRIII, as can be determined by methods well-known and routine in the art. In another embodiment, the modified FcRn binding fragment of the constant domain does not contain sequences that mediate immune effector functions or other receptor binding. In yet another embodiment, the effector functions are selectively altered (e.g., to reduce or increase effector functions).

In some embodiments, the amino acid modifications are substitutions at one or more of residue 251, or residue 252, or residue 254, or residue 255, or residue 309, or residue 312, or residue 386, or residue 434, or both residues 385 and 386. Amino acid residue 251 may be substituted to be serine, amino acid residue 252 may be substituted to be threonine, amino acid residue 254 may be substituted to be tryptophan or arginine, amino acid residue 255 may be substituted to be valine, amino acid residue 309 may be substituted to be arginine, amino acid residue 312 may be substituted to be isoleucine, amino acid residue 386 may be substituted to be leucine, amino acid residue 434 may be substituted to be leucine, and amino acid residues 385 and 386 may be substituted to be serine and isoleucine, respectively.

In another embodiment, the amino acid modifications are at all of residues 251, 252, 254, 255, 309, 312, 385, 386, and 434. Amino acid residue 251 may be substituted to be serine, amino acid residue 252 may be substituted to be threonine, amino acid residue 254 may be substituted to be tryptophan or an arginine, amino acid residue 255 may be substituted to be valine, amino acid residue 309 may be substituted to be arginine, amino acid residue 312 may be substituted to be isoleucine, amino acid residue 434 may be substituted to be leucine, amino acid residue 386 may be substituted to be leucine. A further amino acid residue substitution may be made at amino acid residue 385, for a serine.

In yet another embodiment, the molecule of the invention contains a Fc region, or FcRn-binding domain thereof, having substitutions at one or more of residue 251, or residue 252, or residue 254, or residue 255, or residue 309, or residue 312, or residue 386, or residue 434, or both residues 385 and 386. Amino acid residue 251 may be substituted to be serine, amino acid residue 252 may be substituted to be threonine, amino acid residue 254 may be substituted to be tryptophan or arginine, amino acid residue 255 may be substituted to be valine, amino acid residue 309 may be substituted to be arginine, amino acid residue 312 may be substituted to be isoleucine, amino acid residue 386 may be substituted to be leucine, amino acid residue 434 may be substituted to be leucine, and amino acid residues 385 and 386 may be substituted to be serine and isoleucine, respectively. The FcRn binding domain may have amino acid substitutions at 1, 2. 3, 4, 5, 6, 7, 8, or all 9 of residues 251, 252, 254, 255, 309, 312, 434, 385, or 386.

Amino acid modifications can be made by any method known in the art and many such methods are well known and routine for the skilled artisan. For example, but not by way of limitation, amino acid substitutions, deletions and insertions may be accomplished using any well-known PCR-based technique. Amino acid substitutions may be made by site-directed mutagenesis (see, for example, Zoller and Smith, Nucl. Acids Res. 10:6487-6500, 1982; Kunkel, Proc. Natl. Acad. Sci USA 82:488, 1985, which are hereby incorporated by reference in their entireties). Mutants that result in decreased affinity for FcRn and decreased in vivo half-life may readily be screened using well-known and routine assays. By way of example, amino acid substitutions can be introduced at one or more residues in the IgG constant domain or FcRn-binding fragment thereof and the mutated constant domains or fragments are expressed on the surface of bacteriophage which are then screened for decreased FcRn binding affinity.

The amino acid residues to be modified may be surface exposed residues. In making amino acid substitutions, the amino acid residue to be substituted may or may not be a conservative amino acid substitution, for example, a polar residue is substituted with a polar residue, a hydrophilic residue with a hydrophilic residue, hydrophobic residue with a hydrophobic residue, a positively charged residue with a positively charged residue, or a negatively charged residue with a negatively charged residue.

In one embodiment, the invention provides modified immunoglobulin molecules (e.g., various antibodies) that have decreased in vivo half-life and affinity for FcRn relative to unmodified molecules (and, in some embodiments, altered bioavailabilty such as increased or decreased transport to mucosal surfaces or other target tissues). Such immunoglobulin molecules include IgG molecules that naturally contain an FcRn binding domain and other non-IgG immunoglobulins (e.g., IgE, IgM, IgD, IgA and IgY) or fragments of immunoglobulins that have been engineered to contain an FcRn-binding fragment (i.e., fusion proteins comprising non-IgG immunoglobulin or a portion thereof and an FcRn binding domain). In both cases the FcRn-binding domain has one or more amino acid modifications that decrease the affinity of the constant domain fragment for FcRn.

The modified immunoglobulins include any immunoglobulin molecule that binds (preferably, immunospecifically, i.e., competes off non-specific binding), as determined by immunoassays well known in the art for assaying specific antigen-antibody binding) an antigen and contains an FcRn-binding fragment. Such antibodies include, but are not limited to, polyclonal, monoclonal, bi-specific, multi-specific, human, humanized, chimeric antibodies, single chain antibodies, Fab fragments, F(ab')2 fragments, disulfidelinked Fvs, and fragments containing either a VL or VH domain or even a complementary determining region (CDR) that specifically binds an antigen, in certain cases, engineered to contain or fused to an FcRn binding domain.

The IgG molecules of the invention, and FcRn-binding fragments thereof, may be IgG1 subclass of IgGs, but may also be any other IgG subclasses of given animals. For example, in humans, the IgG class includes IgG1, IgG2, IgG3, and IgG4; and mouse IgG includes IgG1, IgG2a, IgG2b, IgG2c and IgG3. It is known that certain IgG subclasses, for example, mouse IgG2b and IgG2c, have higher clearance rates than, for example, IgG1 (Medesan et al., Eur. J. Immunol., 28:2092-2100, 1998). Thus, when using IgG subclass IgG1, it may be advantageous to substitute one or more of the IgG1 residues, particularly in the CH2 and CH3 domains, with residues in the other IgG subtypes to decrease the in vivo half-life of IgG1.

The immunoglobulins, and portions thereof that bind to FcRn, may be from any animal origin including birds and mammals. The immunoglobulins may be human, rodent (e.g., mouse and rat), donkey, sheep, rabbit, goat, guinea pig, camel, horse, or chicken. As used herein, "human" immunoglobulins include antibodies having the amino acid sequence of a human immunoglobulin and include antibodies isolated from human immunoglobulin libraries or from animals transgenic for one or more human immunoglobulin and that do not express endogenous immunoglobulins, as described infra and, for example, in U.S. Pat. No. 5,939,598 by Kucherlapati et al.

If the polypeptide is an antibody, the antibody may be monospecific, bispecific, trispecific or of greater multi specificity. Multispecific antibodies may be specific for different epitopes of a polypeptide or may be specific for heterologous epitopes, such as a heterologous polypeptide or solid support material. See, e.g., PCT publications WO 93/17715, WO 92/08802; WO 91/00360; WO 92/05793; Tutt, et al., J. Immunol., 147:60-69, 1991; U.S. Pat. Nos. 4,474,893; 4,714,681; 4,925,648; 5,573,920; 5,601,819; Kostelny et al., J. Immunol., 148:1547-1553, 1992.

Antibodies include antibody derivatives that are otherwise modified, i.e., by the covalent attachment of any type of molecule to the antibody such that covalent attachment does not prevent the antibody from binding antigen and/or generating an anti-idiotypic response. For example, but not by way of limitation, the antibody derivatives include antibodies that have been modified, e.g., by glycosylation, acetylation, pegylation, phosphorylation, amidation, derivatization by known protecting/blocking groups, proteolytic cleavage, linkage to a cellular ligand or other protein, etc. Any of numerous chemical modifications may be carried out by known techniques, including, but not limited to, specific chemical cleavage, acetylation, formylation, metabolic synthesis of tunicamycin, etc. Additionally, the derivative may contain one or more non-classical amino acids.

Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al., Antibodies: A Laboratory Manual, (Cold Spring Harbor Laboratory Press, 2nd ed. 1988); Hammering, et al., in: Monoclonal Antibodies and T-Cell Hybridomas, pp. 563-681 (Elsevier, N.Y., 1981) (both of which are incorporated herein by reference in their entireties). The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced.

Methods for producing and screening for specific antibodies using hybridoma technology are routine and well known in the art. In a non-limiting example, mice can be immunized with an antigen of interest or a cell expressing such an antigen. Once an immune response is detected, e.g., antibodies specific for the antigen are detected in the mouse serum, the mouse spleen is harvested and splenocytes isolated. The splenocytes are then fused by well known techniques to any suitable myeloma cells. Hybridomas are selected and cloned by limiting dilution. The hybridoma clones are then assayed by methods known in the art for cells that secrete antibodies capable of binding the antigen. Ascites fluid, which generally contains high levels of antibodies, can be generated by inoculating mice intraperitoneally with positive hybridoma clones.

Antibody fragments which recognize specific epitopes may be generated by known techniques. For example, Fab and F(ab')₂ fragments may be produced by proteolytic cleavage of immunoglobulin molecules, using enzymes such as papain (to produce Fab fragments) or pepsin (to produce F(ab')₂ fragments). F(ab')₂ fragments contain the complete light chain, and the variable region, the CH1 region and the hinge region of the heavy chain.

For example, antibodies can also be generated using various phage display methods known in the art. In phage display methods, functional antibody domains are displayed on the surface of phage particles which carry the polynucleotide sequences encoding them. In a particular embodiment, such phage can be utilized to display antigen binding domains, such as Fab and Fv or disulfide-bond stabilized Fv, expressed from a repertoire or combinatorial antibody library (e.g., human or murine). Phage expressing an antigen binding domain that binds the antigen of interest can be selected or identified with antigen, e.g., using labeled antigen or antigen bound or captured to a solid surface or bead. Phage used in these methods are typically filamentous phage, including fd and M13. The antigen binding domains are expressed as a recombinantly fused protein to either the phage gene III or gene VIII protein. Alternatively, the modified FcRn binding portion of immunoglobulins of the present invention can be also expressed in a phage display system. Examples of phage display methods that can be used to make the immunoglobulins, or fragments thereof, of the present invention include those disclosed in Brinkman et al., J. Immunol. Methods, 182:41-50, 1995; Ames et al., J. Immunol. Methods, 184:177-186, 1995; Kettleborough et al., Eur. J. Immunol., 24:952-958,1994; Persic et al., Gene, 187:9-18, 1997; Burton et al., Advances in Immunology, 57:191-280, 1994; PCT application No. PCT/GB91/01134; PCT publications WO 90/02809; WO 91/10737; WO 92/01047; WO 92/18619; WO 93/1 1236; WO 95/15982; WO 95/20401; and U.S. Pat. Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108; each of which is incorporated herein by reference in its entirety.

As described in the above references, after phage selection, the antibody coding regions from the phage can be isolated and used to generate whole antibodies, including human antibodies, or any other desired fragments, and expressed in any desired host, including mammalian cells, insect cells, plant cells, yeast, and bacteria, e.g., as described in detail below. For example, techniques to recombinantly produce Fab, Fab' and F(ab')₂ fragments can also be employed using methods known in the art such as those disclosed in PCT publication WO 92/22324; Mullinax et al., BioTechniques, 12(6):864-869, 1992; and Sawai et al., AJRI, 34:26-34, 1995; and Better et al., Science, 240:1041-1043, 1988 (each of which is incorporated by reference in its entirety). Examples of techniques which can be used to produce single-chain Fvs and antibodies include those described in U.S. Pat. Nos. 4,946,778 and 5,258,498; Huston et al Methods in Enzymology, 203:46-88,1991; Shu et al., PNAS, 90:7995-7999,1993; and Skerra et al., Science, 240:1038-1040, 1988.

For some uses, including in vivo use of antibodies in humans and in vitro detection assays, it may be preferable to use chimeric, humanized, or human antibodies. A chimeric antibody is a molecule in which different portions of the antibody are derived from different animal species, such as antibodies having a variable region derived from a murine monoclonal antibody and a constant region derived from a human immunoglobulin. Methods for producing chimeric antibodies are known in the art. See e.g., Morrison, Science, 229:1202, 1985; Oi et al., BioTechniques, 4:214 1986; Gillies et al., J. Immunol. Methods, 125:191-202, 1989; U.S. Pat. Nos. 5,807,715; 4,816,567; and 4,816,397, which are incorporated herein by reference in their entireties. Humanized antibodies are antibody molecules from non-human species that bind the desired antigen having one or more complementarity determining regions (CDRs) from the non-human species and framework regions from a human immunoglobulin molecule. Often, framework residues in the human framework regions will be substituted with the corresponding residue from the CDR donor antibody to alter, preferably improve, antigen binding. These framework substitutions are identified by methods well known in the art, e.g., by modeling of the interactions of the CDR and framework residues to identify framework residues important for antigen binding and sequence comparison to identify unusual framework residues at particular positions. See, e.g., Queen et al., U.S. Pat. No. 5,585,089; Riechmann et al., Nature, 332:323, 1988, which are incorporated herein by reference in their entireties. Antibodies can be humanized using a variety of techniques known in the art including, for example, CDR-grafting (EP 239,400; PCT publication WO 91/09967; U.S. Pat. Nos. 5,225,539; 5,530,101 and 5,585,089), veneering or resurfacing (EP 592,106; EP 519,596; Padlan, Molecular Immunology, 28(4/5):489-498, 1991; Studnicka et al., Protein Engineering, 7(6):805-814,1994; Roguska et al., Proc Natl. Acad. Sci. USA, 91:969-973, 1994), and chain shuffling (U.S. Pat. No. 5,565,332), all of which are hereby incorporated by reference in their entireties.

Completely human antibodies are desirable for therapeutic treatment of human patients. Human antibodies can be made by a variety of methods known in the art including phage display methods described above using antibody libraries derived from human immunoglobulin sequences. See U.S. Pat. Nos. 4,444,887 and 4,716,111; and PCT publications WO 98/46645; WO 98/50433; WO 98/24893; WO 98/16654; WO 96/34096; WO 96/33735; and WO 91/10741, each of which is incorporated herein by reference in its entirety.

Human antibodies can also be produced using transgenic mice which are incapable of expressing functional endogenous immunoglobulins, but which can express human immunoglobulin genes. For an overview of this technology for producing human antibodies, see Lonberg and Huszar, Int. Rev. Immunol., 13:65-93, 1995. For a detailed discussion of this technology for producing human antibodies and human monoclonal antibodies and protocols for producing such antibodies, see, e.g., PCT publications WO 98/24893; WO 92/01047; WO 96/34096; WO 96/33735; European Patent No.0 598 877; U.S. Pat. Nos. 5,413,923; 5,625,126; 5,633,425; 5,569,825; 5,661,016; 5,545,806; 5,814,318; 5,885,793; 5,916,771; and 5,939,598, which are incorporated by reference herein in their entireties. In addition, companies such as Abgenix, Inc. (Freemont, Calif.), Medarex (NJ) and Genpharm (San Jose, Calif.) can be engaged to provide human antibodies directed against a selected antigen using technology similar to that described above.

Completely human antibodies which recognize a selected epitope can be generated using a technique referred to as "guided selection." In this approach a selected non-human monoclonal antibody, e.g., a mouse antibody, is used to guide the selection of a completely human antibody recognizing the same epitope. (Jespers et al., Bio/technology, 12:899-903,1988).

In some embodiments, the modified antibodies have in vivo therapeutic and/or prophylactic uses. Examples of therapeutic and prophylactic antibodies which may be so modified include, but are not limited to, SYNAGIS® (MedImmune, MD) which is a humanized anti-respiratory syncytial virus (RSV) monoclonal antibody for the prevention or treatment of RSV infection; NUMAX™ (MedImmune) which is also a humanized anti-RSV monoclonal antibody for the prevention and treatment of RSV infection; HERCEPTIN™ (Trastuzumab) (Genentech, CA) which is a humanized anti-HER2 monoclonal antibody for the treatment of patients with metastatic breast cancer; REMICADE™ (infliximab) (Centocor, PA) which is a chimeric anti-TNFα monoclonal antibody for the treatment of patients with Crone's disease; REOPRO™ (abciximab) (Centocor) which is an anti-glycoprotein IIb/IIIa receptor F(ab) fragment recognizing the alpha-11b/beta-3 integrin on platelets for the prevention of clot formation; CNTO95 (Centocor) which is an alpha-v integrin monoclonal antibody; ZENAPAX™(daclizumab) (Roche Pharmaceuticals, Switzerland) which is an immunosuppressive, humanized anti-CD25 monoclonal antibody for the prevention of acute renal allograft rejection. Other examples are a humanized anti-CD 18 F(ab')2 (Genentech); CDP860 which is a humanized anti-CD18 F(ab')2 (Celltech, UK); PRO542 which is an anti-HIV gp120 antibody fused with CD4 (Progenics/Genzyme Transgenics); Ostavir which is a human anti Hepatitis B virus antibody (Protein Design Lab/Novartis); PROTOVIR™ which is a humanized anti-CMV IgG1 antibody (Protein Design Lab/Novartis); MAK-195 (SEGARD) which is a murine anti-TNF-α F(ab')2 (Knoll Pharma/BASF); IC14 which is an anti-CD14 antibody (ICOS Pharm); a humanized anti-VEGF IgG1 antibody (Genentech); OVAREX™ which is a murine anti-CA 125 antibody (Altarex); PANOREX™ which is a murine anti-17-IA cell surface antigen IgG2a antibody (Glaxo Wellcome/Centocor); BEC2 which is a murine anti-idiotype (GD3 epitope) IgG antibody (ImClone System); IMC-C225 which is a chimeric anti-EGFR IgG antibody (ImClone System); VITAXIN™ which is a humanized anti-αVβ3 integrin antibody (Applied Molecular Evolution/MedImmune); Campath 1H/LDP-03 which is a humanized anti-CD52 IgG1 antibody (Leukosite); Smart M195 which is a humanized anti-CD33 IgG antibody (Protein Design Lab/Kanebo); RITUXAN™ which is a chimeric anti-CD20 IgG1 antibody (IDEC Pharm/Genentech, Roche/Zettyaku); LYMPHOCIDE™ which is a humanized anti-CD22 IgG antibody (Immunomedics); Smart ID10 which is a humanized anti-HLA antibody (Protein Design Lab); ONCOLYM™ (Lym-1) is a radiolabelled murine anti-HLA DIAGNOSTIC REAGENT antibody (Techniclone); ABX-IL8 is a human anti-IL8 antibody (Abgenix); anti-IL9 monoclonal antibodies such as 7Fcom3-2H2 (and other antibodies disclosed in U.S. patent application publication no. 2005-0002934) and MH9D1 (and other antibodies disclosed in U.S. patent application publication no. 2003-0219439) (MedImmune); anti-EphA2 monoclonal antibody as disclosed in U.S. patent application publication number 2006/0039904 (MedImmune); anti-EphA4 monoclonal antibody as disclosed in U.S. patent application number 2005/0059592 (MedImmune); anti-HMGB1 Monoclonal antibodies disclosed in US patent application publication 2006-0099207 and U.S. application serial number 60/822,044 filed August 10, 2006 (MedImmune); IFNα monoclonal antibody as disclosed in WO 05/059106, IFNAR monoclonal antibodies as disclosed in U.S. patent application publication number 2006/0029601 (MedImmune); anti-staphylococcal monoclonal antibodies for the prevention of serious bloodstream infections, such as for example, BSYX-A110 (Biosynexus, MD); anti-CD11a is a humanized IgG1 antibody (Genetech/Xoma); ICM3 is a humanized anti-ICAM3 antibody (ICOS Pharm); IDEC-114 is a primatized anti-CD80 antibody (IDEC Pharm/Mitsubishi); ZEVALIN™ is a radiolabelled murine anti-CD20 antibody (IDEC/Schering AG); IDEC-131 is a humanized anti-CD40L antibody (IDEC/Eisai); IDEC-151 is a primatized anti-CD4 antibody (IDEC); IDEC-152 is a primatized anti-CD23 antibody (IDEC/Seikagaku); SMART anti-CD3 is a humanized anti-CD3 IgG (Protein Design Lab); 5G1.1 is a humanized anti-complement factor 5 (C5) antibody (Alexion Pharm); D2E7 is a humanized anti-TNF-α antibody (CAT/BASF); CDP870 is a humanized anti-TNF-α Fab fragment (Celltech); IDEC-151 is a primatized anti-CD4 IgG1 antibody (IDEC Pharm/SmithKline Beecham); MDX-CD4 is a human anti-CD4 IgG antibody (Medarex/Eisai/Genmab); CDP571 is a humanized anti-TNF-α IgG4 antibody (Celltech); LDP-02 is a humanized anti-α4β7 antibody (LeukoSite/Genentech); OrthoClone OKT4A is a humanized anti-CD4 IgG antibody (Ortho Biotech); ANTOVA™ is a humanized anti-CD40L IgG antibody (Biogen); ANTEGREN™ is a humanized anti-VLA-4 IgG antibody (Elan); MDX-33 is a human anti-CD64 (FcγR) antibody (Medarex/Centeon); SCH55700 is a humanized anti-IL-5 IgG4 antibody (Celltech/Schering); SB-240563 and SB-240683 are humanized anti-IL-5 and IL-4 antibodies, respectively, (SmithKline Beecham); rhuMab-E25 is a humanized anti-IgE IgG1 antibody (Genentech/Norvartis/Tan- ox Biosystems); IDEC-152 is a primatized anti-CD23 antibody (IDEC Pharm); ABX-CBL is a murine anti CD-147 IgM antibody (Abgenix); BTI-322 is a rat anti-CD2 IgG antibody (Medimmune/Bio Transplant); Orthoclone/OKT3 is a murine anti-CD3 IgG2a antibody (ortho Biotech); SIMULECT™ is a chimeric anti-CD25 IgG1 antibody (Novartis Pharm); LDP-01 is a humanized anti-β2-integrin IgG antibody (LeukoSite); Anti-LFA-1 is a murine anti CD18 F(ab')2 (Pasteur-Merieux/Immunotech); CAT-152 is a human anti-TGF-β2 antibody (Cambridge Ab Tech); and Corsevin M is a chimeric anti-Factor VII antibody (Centocor).

In other embodiments, the antibody is further modified at its antigen-binding sites, Fc-receptor binding sites, or complement-binding sites, by genetic engineering to increase or reduce such activities compared to the wild type.

The present invention also provides polypeptides containing at least an FcRn binding portion of an Fc region of an IgG molecule and that contain amino acid alterations. The portion of the Fc region of the IgG molecule may comprise from about amino acid residues 231-446 of an IgG molecule, according to EU numbering. Alternatively, the portion of the Fc region of the IgG molecule may comprise from about amino acid residues 216-446 of an IgG molecule, according to EU numbering. The polypeptides containing at least an FcRn binding portion of an Fc region of the IgG molecule may comprise a CH2 domain having one or more amino acid residue substitutions in amino acid residues 251, 252, 254, 255, 309, and/or amino acid residue 312, and/or to a CH3 domain having one or more modifications in amino acid residues 386 and/or 385 and 386 and/or 434.

The polypeptide containing at least an FcRn binding portion of an Fc region of an IgG molecule may be fused to a second polypeptide molecule or be conjugated to a toxic moiety. The second polypeptide molecule or toxic moiety may be referred to as a bioactive molecule.

A bioactive molecule can be any polypeptide or synthetic drug known to one of skill in the art. A bioactive molecule may be a polypeptide consisting of at least 5, at least 10, at least 20, at least 30, at least 40, at least 50, at least 60, at least 70, at least 80, at least 90 or at least 100 amino acid residues. Examples of bioactive polypeptides include, but are not limited to, various types of antibodies, cytokines (e.g., IL-2, IL-3, IL-4, IL-5, IL-6, IL-7, IL-10, IL-12, IL-15, IFN-γ, IFN-α and IFN-β), cell adhesion molecules (e.g., CTLA4, CD2, and CD28), ligands (e.g., TNF-α, TNF-β, and an anti-angiogenic factor such as endostatin), receptors, growth factors (e.g., PDGF, EGF, NGF, and KGF).

A bioactive molecule may be a molecule that binds to a tumor associated antigen, for example, pan B antigens (e.g., CD20), pan T cell antigens (e.g, CD2, CD3, CD5, CD6, CD7), MAGE-1, MAGE-3, MUC-1, HPV 16, HPV E6, HPV E7, TAG-72, CEA, α-Lewis^{y}, L6-Antigen, CD19, CD22, CD25, CD30, CD33, CD37, CD44, CD52, CD56, mesothelin, PSMA, HLA-DR, EGF receptor, VEGF receptor, and HER2 receptor.

A bioactive molecule may be an adhesion molecule. Adhesion molecules are membrane bound proteins that allow cells to interact with one another. Various adhesion proteins, including leukocyte homing receptors and cellular adhesion molecules, of receptor binding portions thereof, can be incorporated in a fusion protein of the invention. Leukocyte homing receptors are expressed on leukocyte cell surfaces during inflammation and include the β1-integrins (e.g., VLA-1, 2, 3, 4, 5, and 6) which mediate binding to extracellular matrix components, and the β2-integrins (e.g., LF-1, LPAM-1, CR3, and CR4) which bind cellular adhesion molecules (CAMs) on vascular endothelium. CAMs include ICAM-1, ICAM-2, VCAM-1, MadCAM-1, E-selectin, L-selectin, and P-selectin.

A bioactive molecule can also be a therapeutic moiety such as a cytotoxin (e.g., a cytostatic or cytocidal agent), a therapeutic agent or a radioactive element (e.g., alpha-emitters, gamma-emitters, etc.). Examples of cytostatic or cytocidal agents include, but are not limited to, paclitaxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents include, but are not limited to, antimetabolites (e.g., methotrexate, 6-mercaptopurine, 6-thioguanine, cytarabine, 5-fluorouracil decarbazine), alkylating agents (e.g., mechlorethamine, thioepa chlorambucil, melphalan, carmustine (BSNU) and lomustine (CCNU), cyclothosphamide, busulfan, dibromomannitol, streptozotocin, mitomycin C, and cisdichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine and vinblastine).

The present invention also provides polynucleotides comprising a nucleotide sequence encoding a modified IgG of the invention and fragments thereof which contain modified FcRn binding sites with decreased affinity and vectors comprising said polynucleotides. Furthermore, the invention includes polynucleotides that hybridize under stringent or lower stringent hybridization conditions to polynucleotides encoding modified IgGs of the present invention.

The nucleotide sequence of modified IgGs and the polynucleotides encoding the same may be obtained by any methods known in the art, including general DNA sequencing method, such as dideoxy chain termination method (Sanger sequencing), and oligonucleotide priming in combination with PCR, respectively.

### 5.3 IDENTIFICATION OF MUTATIONS WITHIN THE HINGE-FC REGION OF IMMUNOGLOBULIN MOLECULES

One or more modifications in amino acid residues 251, 252, 254, 255, 309, 312, 386, 434, or 385 and 386 of the constant domain may be introduced utilizing any technique known to those of skill in the art. The constant domain or fragment thereof having one or more modifications in amino acid 251, 252, 254, 255, 309, 312, 386, 434, or 385 and 386 may be screened by, for example, a binding assay to identify the constant domain or fragment thereof with decreased affinity for the FcRn receptor. Those modifications in the hinge-Fc domain or the fragments thereof which decrease the affinity of the constant domain or fragment thereof for the FcRn receptor can be introduced into antibodies to decrease the in vivo half-lives of said antibodies. Further, those modifications in the constant domain or the fragment thereof which decrease the affinity of the constant domain or fragment thereof for the FcRn can be fused to bioactive molecules to decrease the in vivo half-lives of said bioactive molecules and preferably alter (increase or decrease) the bioavailability of the molecule, for example, to increase or decrease transport to mucosal surfaces (or other target tissue) (e.g., the lungs) or other portions of a target tissue. The amino acid modifications may alter transport or concentration or half-life of the molecule to the lungs, may alter transport (or concentration or half-life) of the molecule to the heart, pancreas, liver, kidney, bladder, stomach, large or small intestine, respiratory tract, lymph nodes, nervous tissue (central and/or peripheral nervous tissue), muscle, epidermis, bone, cartilage, joints, blood vessels, bone marrow, prostate, ovary, uterine, tumor or cancer tissue, etc. In one embodiment, the amino acid modifications do not abolish, or do not alter, other immune effector or receptor binding functions of the constant domain, for example, but not limited to complement fixation, ADCC and binding to FcγRI, FcγRII, and FcγRIII, as can be determined by methods well-known and routine in the art. In another embodiment, the modified FcRn binding fragment of the constant domain does not contain sequences that mediate immune effector functions or other receptor binding. In yet another embodiment, the effector functions are selectively altered (e.g., to reduce or increase effector functions).

### 5.3.1. MUTAGENESIS

Mutagenesis may be performed in accordance with any of the techniques known in the art including, but not limited to, synthesizing an oligonucleotide having one or more modifications within the sequence of the constant domain of an antibody or a fragment thereof (e.g., the CH2 or CH3 domain) to be modified. Site-specific mutagenesis allows the production of mutants through the use of specific oligonucleotide sequences which encode the DNA sequence of the desired mutation, as well as a sufficient number of adjacent nucleotides, to provide a primer sequence of sufficient size and sequence complexity to form a stable duplex on both sides of the deletion junction being traversed. Typically, a primer of about 17 to about 75 nucleotides or more in length is preferred, with about 10 to about 25 or more residues on both sides of the junction of the sequence being altered. A number of such primers introducing a variety of different mutations at one or more positions may be used to generated a library of mutants.

The technique of site-specific mutagenesis is well known in the art, as exemplified by various publications (see, e.g.,. Kunkel et al., Methods Enzymol., 154:367-82, 1987, which is hereby incorporated by reference in its entirety). In general, site-directed mutagenesis is performed by first obtaining a single-stranded vector or melting apart of two strands of a double stranded vector which includes within its sequence a DNA sequence which encodes the desired peptide. An oligonucleotide primer bearing the desired mutated sequence is prepared, generally synthetically. This primer is then annealed with the single-stranded vector, and subjected to DNA polymerizing enzymes such as T7 DNA polymerase, in order to complete the synthesis of the mutation-bearing strand. Thus, a heteroduplex is formed wherein one strand encodes the original non-mutated sequence and the second strand bears the desired mutation. This heteroduplex vector is then used to transform or transfect appropriate cells, such as E. coli cells, and clones are selected which include recombinant vectors bearing the mutated sequence arrangement. As will be appreciated, the technique typically employs a phage vector which exists in both a single stranded and double stranded form. Typical vectors useful in site-directed mutagenesis include vectors such as the M13 phage. These phage are readily commercially available and their use is generally well known to those skilled in the art. Double stranded plasmids are also routinely employed in site directed mutagenesis which eliminates the step of transferring the gene of interest from a plasmid to a phage.

Alternatively, the use of PCR™ with commercially available thermostable enzymes such as Taq DNA polymerase may be used to incorporate a mutagenic oligonucleotide primer into an amplified DNA fragment that can then be cloned into an appropriate cloning or expression vector. See, e.g., Tomic et al., Nucleic Acids Res., 18(6):1656, 1987, and Upender et al., Biotechniques, 18(1):29-30, 32, 1995, for PCR™-mediated mutagenesis procedures, which are hereby incorporated in their entireties. PCR™ employing a thermostable ligase in addition to a thermostable polymerase may also be used to incorporate a phosphorylated mutagenic oligonucleotide into an amplified DNA fragment that may then be cloned into an appropriate cloning or expression vector (see e.g., Michael, Biotechniques, 16(3):410-2,1994, which is hereby incorporated by reference in its entirety).

Other methods known to those of skill in art of producing sequence variants of the Fc domain of an antibody or a fragment thereof can be used. For example, recombinant vectors encoding the amino acid sequence of the constant domain of an antibody or a fragment thereof may be treated with mutagenic agents, such as hydroxylamine, to obtain sequence variants.

### 5.3.2. PANNING

Vectors, in particular, phage, expressing constant domains or fragments thereof having one or more modifications in amino acid residues 251, 252, 254, 255, 309, 312, 386, 434, and/or 385 + 386 can be screened to identify constant domains or fragments thereof having decreased affinity for FcRn to select out the lowest affinity binders from a population of phage. Immunoassays which can be used to analyze binding of the constant domain or fragment thereof having one or more modifications in amino acid residues 251, 252, 254, 255, 309, 312, 386, 434, and/or 385 + 386 to the FcRn include, but are not limited to, radioimmunoassays, ELISA (enzyme linked immunosorbent assay), "sandwich" immunoassays, and fluorescent immunoassays. Such assays are routine and well known in the art (see, e.g., Ausubel et al., eds, 1994, Current Protocols in Molecular Biology, Vol. 1, John Wiley & Sons, Inc., New York, which is incorporated by reference herein in its entirety). Exemplary immunoassays are described briefly herein below (but are not intended by way of limitation). BIAcore kinetic analysis can also be used to determine the binding on and off rates of a constant domain or a fragment thereof having one or more modifications in amino acid residues 251, 252, 254, 255, 309, 312, 386, 434, and/or 385 + 386 to the FcRn. BIAcore kinetic analysis comprises analyzing the binding and dissociation of a constant domain or a fragment thereof having one or more modifications in amino acid residues 251, 252, 254, 255, 309, 312, 386, 434, and/or 385 + 386 from chips with immobilized FcRn on their surface.

### 5.3.3. SEQUENCING

Any of a variety of sequencing reactions known in the art can be used to directly sequence the nucleotide sequence encoding constant domains or fragments thereof having one or more modifications in amino acid residues 251, 252, 254, 255, 309, 312, 386, 434, and/or 385 + 386. Examples of sequencing reactions include those based on techniques developed by Maxim and Gilbert (Proc. Natl. Acad. Sci. USA, 74:560, 1977) or Sanger (Proc. Natl. Acad. Sci. USA, 74:5463, 1977). It is also contemplated that any of a variety of automated sequencing procedures can be utilized (Bio/Techniques, 19:448, 1995), including sequencing by mass spectrometry (see, e.g., PCT Publication No. WO 94/16101, Cohen et al., Adv. Chromatogr., 36:127-162, 1996, and Griffin et al., Appl. Biochem. Biotechnol., 38:147-159, 1993).

### 5.4. RECOMBINANT METHODS OF PRODUCING ANTIBODIES

The polypeptides of the invention, which include antibodies or fragments thereof can be produced by any method known in the art for the synthesis of antibodies, e.g., by chemical synthesis or recombinant expression techniques.

The nucleotide sequence encoding an antibody may be obtained from any information available to those of skill in the art (i.e., from Genbank, the literature, or by routine cloning). If a clone containing a nucleic acid encoding a particular antibody or an epitope-binding fragment thereof is not available, but the sequence of the antibody molecule or epitope-binding fragment thereof is known, a nucleic acid encoding the immunoglobulin may be chemically synthesized or obtained from a suitable source (e.g., an antibody cDNA library, or a cDNA library generated from, or nucleic acid, preferably poly A.+ RNA, isolated from any tissue or cells expressing the antibody, such as hybridoma cells selected to express an antibody) by PCR amplification using synthetic primers hybridizable to the 3' and 5' ends of the sequence or by cloning using an oligonucleotide probe specific for the particular gene sequence to identify, e.g., a cDNA clone from a cDNA library that encodes the antibody. Amplified nucleic acids generated by PCR may then be cloned into replicable cloning vectors using any method well known in the art.

Once the nucleotide sequence of the antibody is determined, the nucleotide sequence of the antibody may be manipulated using methods well known in the art for the manipulation of nucleotide sequences, e.g., recombinant DNA techniques, site directed mutagenesis, PCR, etc. (see, for example, the techniques described in Sambrook et al., 1990, Molecular Cloning, A Laboratory Manual, 2d Ed., Cold Spring Harbor Laboratory, Cold Spring Harbor, N.Y.; and Ausubel et al., eds., 1998, Current Protocols in Molecular Biology, John Wiley & Sons, NY, which are both incorporated by reference herein in their entireties), to generate antibodies having a different amino acid sequence by, for example, introducing amino acid substitutions, deletions, and/or insertions into the epitope-binding domain regions of the antibodies and preferably, into the hinge-Fc regions of the antibodies which are involved in the interaction with the FcRn. In a preferred embodiment, antibodies having one or more modifications in amino acid residues 251, 252, 254, 255, 309, 312, 386, 434, and/or 385 + 386 are generated.

Recombinant expression of an antibody requires construction of an expression vector containing a nucleotide sequence that encodes the antibody. Once a nucleotide sequence encoding an antibody molecule or a heavy or light chain of an antibody, or portion thereof (preferably, but not necessarily, containing the heavy or light chain variable region) has been obtained, the vector for the production of the antibody molecule may be produced by recombinant DNA technology using techniques well known in the art. Thus, methods for preparing a protein by expressing a polynucleotide containing an antibody encoding nucleotide sequence are described herein. Methods which are well known to those skilled in the art can be used to construct expression vectors containing antibody coding sequences and appropriate transcriptional and translational control signals. These methods include, for example, in vitro recombinant DNA techniques, synthetic techniques, and in vivo genetic recombination. The invention, thus, provides replicable vectors comprising a nucleotide sequence encoding the constant region of the antibody molecule with one or more modifications in the amino acid residues involved in the interaction with the FcRn (see, e.g., PCT Publication WO 86/05807; PCT Publication WO 89/01036; and U.S. Pat. No. 5,122,464). The nucleotide sequence encoding the heavy-chain variable region, light-chain variable region, both the heavy-chain and light-chain variable regions, an epitope-binding fragment of the heavy- and/or light-chain variable region, or one or more complementarity determining regions (CDRs) of an antibody may be cloned into such a vector for expression.

The expression vector is transferred to a host cell by conventional techniques and the transfected cells are then cultured by conventional techniques to produce an antibody having an decreased affinity for the FcRn and an decreased in vivo half-life. Thus, the invention encompasses host cells containing a polynucleotide encoding an antibody, a constant domain or a FcRn binding fragment thereof having one or more modifications in amino acid residues 251, 252, 254, 255, 309, 312, 386, 434, and/or 385 + 386, that may be, operably linked to a heterologous promoter.

A variety of host-expression vector systems may be utilized to express the antibody molecules of the invention. Such host-expression systems represent vehicles by which the coding sequences of interest may be produced and subsequently purified, but also represent cells which may, when transformed or transfected with the appropriate nucleotide coding sequences, express an antibody molecule of the invention in situ. These include, but are not limited to, microorganisms such as bacteria (e.g., E. coli and B. subtilis) transformed with recombinant bacteriophage DNA, plasmid DNA or cosmid DNA expression vectors containing antibody coding sequences; yeast (e.g., Saccharomyces and Pichia) transformed with recombinant yeast expression vectors containing antibody coding sequences; insect cell systems infected with recombinant virus expression vectors (e.g., baculovirus) containing antibody coding sequences; plant cell systems infected with recombinant virus expression vectors (e.g., cauliflower mosaic virus, CaMV; and tobacco mosaic virus, TMV) or transformed with recombinant plasmid expression vectors (e.g., Ti plasmid) containing antibody coding sequences; and mammalian cell systems (e.g., COS, CHO, BHK, 293, 3T3 and NSO cells) harboring recombinant expression constructs containing promoters derived from the genome of mammalian cells (e.g., metallothionein promoter) or from mammalian viruses (e.g., the adenovirus late promoter; the vaccinia virus 7.5K promoter). Bacterial cells such as Escherichia coli, or eukaryotic cells, for the expression of whole recombinant antibody molecule, can be used for the expression of a recombinant antibody molecule. For example, mammalian cells such as Chinese hamster ovary cells (CHO), in conjunction with a vector such as the major intermediate early gene promoter element from human cytomegalovirus is an effective expression system for antibodies (Foecking et al., Gene, 45:101, 1986, and Cockett et al., Bio/Technology, 8:2, 1990).

In bacterial systems, a number of expression vectors may be advantageously selected depending upon the use intended for the antibody molecule being expressed. For example, when a large quantity of such a protein is to be produced, for the generation of pharmaceutical compositions of an antibody molecule, vectors which direct the expression of high levels of fusion protein products that are readily purified may be desirable. Such vectors include, but are not limited to, the E. coli expression vector pUR278 (Ruther et al., EMBO, 12:1791, 1983), in which the antibody coding sequence may be ligated individually into the vector in frame with the lacZ coding region so that a fusion protein is produced; and pIN vectors (Inouye & Inouye, Nucleic Acids Res., 13:3101-3109, 1985 and Van Heeke & Schuster, J. Biol. Chem., 24:5503-5509, 1989).

In an insect system, Autographa californica nuclear polyhedrosis virus (AcNPV) may be used as a vector to express foreign genes. The virus grows in Spodoptera frugiperda cells. The antibody coding sequence may be cloned individually into non-essential regions (for example the polyhedrin gene) of the virus and placed under control of an AcNPV promoter (for example the polyhedrin promoter).

In mammalian host cells, a number of viral-based expression systems may be utilized to express an antibody molecule of the invention. In cases where an adenovirus is used as an expression vector, the antibody coding sequence of interest may be ligated to an adenovirus transcription/translation control complex, e.g., the late promoter and tripartite leader sequence. This chimeric gene may then be inserted in the adenovirus genome by in vitro or in vivo recombination. Insertion in a non-essential region of the viral genome (e.g., region E1 or E3) will result in a recombinant virus that is viable and capable of expressing the antibody molecule in infected hosts (e.g., see Logan & Shenk, Proc. Natl. Acad. Sci. USA, 81:355-359, 1984). Specific initiation signals may also be required for efficient translation of inserted antibody coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (see, e.g., Bitter et al., Methods in Enzymol., 153:516-544,1987).

In addition, a host cell strain may be chosen which modulates the expression of the antibody sequences, or modifies and processes the antibody in the specific fashion desired. Such modifications (e.g., glycosylation) and processing (e.g., cleavage) of protein products may be important for the function of the antibody. Different host cells have characteristic and specific mechanisms for the post-translational processing and modification of proteins and gene products. Appropriate cell lines or host systems can be chosen to ensure the correct modification and processing of the antibody expressed. To this end, eukaryotic host cells which possess the cellular machinery for proper processing of the primary transcript, glycosylation, and phosphorylation of the gene product may be used. Such mammalian host cells include but are not limited to CHO, VERO, BHK, HeLa, COS, MDCK, 293, 3T3, W138, and in particular, myeloma cells such as NS0 cells, and related cell lines, see, for example, Morrison et al., U.S. Pat. No. 5,807,715, which is hereby incorporated by reference in its entirety.

For long-term, high-yield production of recombinant antibodies, stable expression is preferred. For example, cell lines which stably express the antibody molecule may be engineered. Rather than using expression vectors which contain viral origins of replication, host cells can be transformed with DNA controlled by appropriate expression control elements (e.g., promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the antibody molecule. Such engineered cell lines may be particularly useful in screening and evaluation of compositions that interact directly or indirectly with the antibody molecule.

A number of selection systems may be used, including but not limited to, the herpes simplex virus thymidine kinase (Wigler et al., Cell, 11:223, 1977), hypoxanthine-guanine phosphoribosyltransferase (Szybalska & Szybalski, Proc. Natl. Acad. Sci. USA, 48:202, 1992), and adenine phosphoribosyltransferase (Lowy et al., Cell, 22:8-17, 1980) genes can be employed in tk⁻, hgprt⁻ or aprt⁻ cells, respectively. Also, antimetabolite resistance can be used as the basis of selection for the following genes: dhfr, which confers resistance to methotrexate (Wigler et al., Natl. Acad. Sci. USA, 77:357, 1980 and O'Hare et al., Proc. Natl. Acad. Sci. USA, 78:1527, 1981); gpt, which confers resistance to mycophenolic acid (Mulligan & Berg, Proc. Natl. Acad. Sci. USA, 78:2072, 1981); neo, which confers resistance to the aminoglycoside G-418 (Wu and Wu, Biotherapy, 3:87-95, 1991; Tolstoshev, Ann. Rev. Pharmacol. Toxicol., 32:573-596, 1993; Mulligan, Science, 260:926-932, 1993; and Morgan and Anderson, Ann. Rev. Biochem., 62: 191-217, 1993; and May, TIB TECH, 11(5):155-2 15, 1993); and hygro, which confers resistance to hygromycin (Santerre et al., Gene, 30:147, 1984). Methods commonly known in the art of recombinant DNA technology may be routinely applied to select the desired recombinant clone, and such methods are described, for example, in Ausubel et al. (eds.), 1993, Current Protocols in Molecular Biology, John Wiley & Sons, NY; Kriegler, 1990, Gene Transfer and Expression, A Laboratory Manual, Stockton Press, NY; in Chapters 12 and 13, Dracopoli et al. (eds), 1994, Current Protocols in Human Genetics, John Wiley & Sons, NY; and Colberre-Garapin et al., J. Mol. Biol., 150:1, 1981, which are incorporated by reference herein in their entireties.

The expression levels of an antibody molecule can be increased by vector amplification (for a review, see Bebbington and Hentschel, 1987, The use of vectors based on gene amplification for the expression of cloned genes in mammalian cells in DNA cloning, Vol.3. Academic Press, New York). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., Mol., Cell. Biol., 3:257, 1983).

The host cell may be co-transfected with two expression vectors of the invention, the first vector encoding a heavy chain derived polypeptide and the second vector encoding a light chain derived polypeptide. The two vectors may contain identical selectable markers which enable equal expression of heavy and light chain polypeptides or different selectable markers to ensure maintenance of both plasmids. Alternatively, a single vector may be used which encodes, and is capable of expressing, both heavy and light chain polypeptides. In such situations, the light chain should be placed before the heavy chain to avoid an excess of toxic free heavy chain (Proudfoot, Nature, 322:52, 1986; and Kohler, Proc. Natl. Acad. Sci. USA, 77:2 197, 1980). The coding sequences for the heavy and light chains may comprise cDNA or genomic DNA.

Once an antibody molecule encompassed by the invention has been produced by recombinant expression, it may be purified by any method known in the art for purification of an immunoglobulin molecule, for example, by chromatography (e.g., ion exchange, affinity, particularly by affinity for the specific antigen after Protein A purification, and sizing column chromatography), centrifugation, differential solubility, or by any other standard techniques for the purification of proteins. Further, the antibodies of the present invention or fragments thereof may be fused to heterologous polypeptide sequences described herein or otherwise known in the art to facilitate purification.

### 5.4.1. ANTIBODY CONJUGATES

The present invention encompasses antibodies or fragments thereof recombinantly fused or chemically conjugated (including both covalently and non-covalently conjugations) to heterologous polypeptides (i.e., an unrelated polypeptide; or portion thereof, preferably at least 10, at least 20, at least 30, at least 40, at least 50, at least 60, at least 70, at least 80, at least 90 or at least 100 amino acids of the polypeptide) to generate fusion proteins. The fusion does not necessarily need to be direct, but may occur through linker sequences. Antibodies fused or conjugated to heterologous polypeptides may also be used in in vitro immunoassays and purification methods using methods known in the art. See e.g., PCT Publication No. WO 93/21232; EP 439,095; Naramura et al., Immunol. Lett., 39:91-99,1994; U.S. Pat. No. 5,474,981; Gillies et al., PNAS, 89:1428-1432, 1992; and Fell et al., J. Immunol., 146:2446-2452, 1991, which are incorporated herein by reference in their entireties.

Antibodies can be fused to marker sequences, such as a peptide to facilitate purification. In preferred embodiments, the marker amino acid sequence is a hexa-histidine peptide, such as the tag provided in a pQE vector (QIAGEN, Inc., 9259 Eton Avenue, Chatsworth, Calif., 91311), among others, many of which are commercially available. As described in Gentz et al., Proc. Natl. Acad. Sci. USA, 86:821-824, 1989, for instance, hexa-histidine provides for convenient purification of the fusion protein. Other peptide tags useful for purification include, but are not limited to, the hemagglutinin "HA" tag, which corresponds to an epitope derived from the influenza hemagglutinin protein (Wilson et al., Cell, 37:767 1984) and the "flag" tag (Knappik et al., Biotechniques, 17(4):754-761, 1994).

The present invention also encompasses antibodies conjugated to a diagnostic or therapeutic agent or any other molecule for which in vivo half-life is desired to be decreased, e.g., an agent which increases toxicity of the antibody molecule or an agent conjugated to the antibody for imaging. Antibodies conjugated to agents that are used for imaging may be used, for example, to monitor the development or progression of a disease, disorder or infection or as part of a clinical testing procedure to, e.g., determine the efficacy of a given treatment regimen. Such an agent may be referred to as a detectable substance. Examples of detectable substances include various enzymes, prosthetic groups, fluorescent materials, luminescent materials, bioluminescent materials, radioactive materials, positron emitting metals, and nonradioactive paramagnetic metal ions. The detectable substance may be coupled or conjugated either directly to the antibody or indirectly, through an intermediate (such as, for example, a linker known in the art) using techniques known in the art. See, for example, U.S. Pat. No. 4,741,900 for metal ions which can be conjugated to antibodies for use as diagnostics according to the present invention. Examples of suitable enzymes include horseradish peroxidase, alkaline phosphatase, β-galactosidase, or acetylcholinesterase; examples of suitable prosthetic group complexes include streptavidin/biotin and avidinibiotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin; and examples of suitable radioactive material include ¹²⁵I, ¹³¹I, ¹¹¹In or ^{99m}Tc.

An antibody may be conjugated to a therapeutic moiety such as a cytotoxin (e.g., a cytostatic or cytocidal agent), a therapeutic agent or a radioactive element (e.g., alpha-emitters, gamma-emitters, etc.), e.g., a toxic agent which, if has the antibody or portion thereof has decreased half-life will decrease side effects caused by administration of the antibody. Cytotoxins or cytotoxic agents include any agent that is detrimental to cells. Examples include paclitaxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents include, but are not limited to, antimetabolites (e.g., methotrexate, 6-mercaptopurine, 6-thioguanine, cytarabine, 5-fluorouracil decarbazine), alkylating agents (e.g, mechlorethamine, thioepa chlorambucil, melphalan, carmustine (BSNU) and lomustine (CCNU), cyclothosphamide, busulfan, dibromomannitol, streptozotocin, mitomycin C, and cisdichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine and vinblastine).

Further, an antibody may be conjugated to a therapeutic agent or drug moiety that modifies a given biological response. Therapeutic agents or drug moieties are not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, α-interferon (IFN-α), β-interferon (IFN-β), nerve growth factor (NGF), platelet derived growth factor (PDGF), tissue plasminogen activator (TPA), an apoptotic agent (e.g., TNF-α, TNF-β, AIM I as disclosed in PCT Publication No. WO 97/33899), AIM II (see, PCT Publication No. WO 97/34911), Fas Ligand (Takahashi et al., J. Immunol., 6:1567-1574, 1994), and VEGI (PCT Publication No. WO 99/23105), a thrombotic agent or an anti-angiogenic agent (e.g., angiostatin or endostatin); or a biological response modifier such as, for example, a lymphokine (e.g., interleukin-1 ("IL-1"), interleukin-2 ("IL-2"), interleukin-6 ("IL-6"), granulocyte macrophage colony stimulating factor ("GM-CSF"), and granulocyte colony stimulating factor ("G-CSF")), or a growth factor (e.g., growth hormone ("GH")).

Techniques for conjugating such therapeutic moieties to antibodies are well known; see, e.g., Amon et al., "Monoclonal Antibodies For Immunotargeting Of Drugs In Cancer Therapy", in Monoclonal Antibodies And Cancer Therapy, Reisfeld et al. (eds.), 1985, pp. 243-56, Alan R. Liss, Inc.); Hellstrom et al., "Antibodies For Drug Delivery", in Controlled Drug Delivery (2nd Ed.), Robinson et al. (eds.), 1987, pp. 623-53, Marcel Dekker, Inc. ); Thorpe, "Antibody Carriers Of Cytotoxic Agents In Cancer Therapy: A Review", in Monoclonal Antibodies '84:Biological And Clinical Applications, Pinchera et al. (eds.), 1985, pp. 475-506); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), 1985, pp. 303-16, Academic Press; and Thorpe et al., Immunol. Recombinant expression vector., 62:119-58, 1982.

An antibody or fragment thereof, with or without a therapeutic moiety conjugated to it, administered alone or in combination with cytotoxic factor(s) and/or cytokine(s) can be used as a therapeutic.

Alternatively, an antibody can be conjugated to a second antibody to form an antibody heteroconjugate as described by Segal in U.S. Pat. No. 4,676,980, which is incorporated herein by reference in its entirety.

Antibodies may also be attached to solid supports, which are particularly useful for immunoassays or purification of the target antigen. Such solid supports include, but are not limited to, glass, cellulose, polyacrylamide, nylon, polystyrene, polyvinyl chloride or polypropylene.

### 5.5 METHODS OF PRODUCING FUSION PROTEINS

Fusion proteins can be produced by standard recombinant DNA techniques or by protein synthetic techniques, e.g., by use of a peptide synthesizer. For example, a nucleic acid molecule encoding a fusion protein can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence (see, e.g., Current Protocols in Molecular Biology, Ausubel et al., eds., John Wiley & Sons, 1992). Moreover, a nucleic acid encoding a bioactive molecule can be cloned into an expression vector containing an IgG Fc domain or a fragment thereof such that the bioactive molecule is linked in-frame to the constant domain or fragment thereof.

Methods for fusing or conjugating polypeptides to the constant regions of antibodies are known in the art. See, e.g., U.S. Pat. Nos. 5,336,603, 5,622,929, 5,359,046, 5,349,053, 5,447,851, 5,723,125, 5,783,181, 5,908,626, 5,844,095, and 5,112,946; EP 307,434; EP 367,166; EP 394,827; PCT publications WO 91/06570, WO 96/04388, WO 96/22024, WO 97/34631, and WO 99/04813; Ashkenazi et al., Proc. Natl. Acad. Sci. USA, 88: 10535-10539, 1991; Traunecker et al., Nature, 331:84-86, 1988; Zheng et al., J. Immunol., 154:5590-5600,1995; and Vil et al., Proc. Natl. Acad. Sci. USA, 89:11337-11341, 1992, which are incorporated herein by reference in their entireties.

The nucleotide sequence encoding a bioactive molecule may be obtained from any information available to those of skill in the art (e.g., from Genbank, the literature, or by routine cloning), and the nucleotide sequence encoding a constant domain or a fragment thereof with decreased affinity for the FcRn may be determined by sequence analysis of mutants produced using techniques described herein, or may be obtained from Genbank or the literature. The nucleotide sequence coding for a fusion protein can be inserted into an appropriate expression vector, i.e., a vector which contains the necessary elements for the transcription and translation of the inserted protein-coding sequence. A variety of host-vector systems may be utilized in the present invention to express the protein-coding sequence. These include but are not limited to mammalian cell systems infected with virus (e.g., vaccinia virus, adenovirus, etc.); insect cell systems infected with virus (e.g., baculovirus); microorganisms such as yeast containing yeast vectors; or bacteria transformed with bacteriophage, DNA, plasmid DNA, or cosmid DNA. The expression elements of vectors vary in their strengths and specificities. Depending on the host-vector system utilized, any one of a number of suitable transcription and translation elements may be used.

The expression of a fusion protein may be controlled by any promoter or enhancer element known in the art. Promoters which may be used to control the expression of the gene encoding fusion protein include, but are not limited to, the SV40 early promoter region (Bemoist and Chambon, Nature, 290:304-310, 1981), the promoter contained in the 3' long terminal repeat of Rous sarcoma virus (Yamamoto, et al., Cell, 22:787-797, 1980), the herpes thymidine kinase promoter (Wagner et al., Proc. Natl. Acad. Sci. U.S.A., 78:1441-1445, 1981), the regulatory sequences of the metallothionein gene (Brinster et al., Nature, 296:39-42, 1982), the tetracycline (Tet) promoter (Gossen et al., Proc. Nat. Acad. Sci. USA, 89:5547-5551, 1995); prokaryotic expression vectors such as the β-lactamase promoter (Villa-Kamaroff, et al., Proc. Natl. Acad. Sci. U.S.A., 75:3727-3731, 1978), or the tac promoter (DeBoer, et al., Proc. Natl. Acad. Sci. U.S.A., 80:21-25, 1983; see also "Useful proteins from recombinant bacteria" in Scientific American, 242:74-94, 1980); plant expression vectors comprising the nopaline synthetase promoter region (Herrera-Estrella et al., Nature, 303:209-213, 1983) or the cauliflower mosaic virus 35S RNA promoter (Gardner, et al., Nucl. Acids Res., 9:2871, 1981), and the promoter of the photosynthetic enzyme ribulose biphosphate carboxylase (Herrera-Estrella et al., Nature, 310:115-120, 1984); promoter elements from yeast or other fungi such as the Gal 4 promoter, the ADC (alcohol dehydrogenase) promoter, PGK (phosphoglycerol kinase) promoter, alkaline phosphatase promoter, and the following animal transcriptional control regions, which exhibit tissue specificity and have been utilized in transgenic animals: elastase I gene control region which is active in pancreatic acinar cells (Swift et al., Cell 38:639-646, 1984; Omitz et al., 50:399-409, Cold Spring Harbor Symp. Quant. Biol., 1986; MacDonald, Hepatology 7:425-515, 1987); insulin gene control region which is active in pancreatic beta cells (Hanahan, Nature 315:115-122, 1985), immunoglobulin gene control region which is active in lymphoid cells (Grosschedl et al., Cell, 38:647-658, 1984; Adames et al., Nature 318:533-538, 1985; Alexander et al., Mol. Cell. Biol., 7:1436-1444, 1987), mouse mammary tumor virus control region which is active in testicular, breast, lymphoid and mast cells (Leder et al., Cell, 45:485-495, 1986), albumin gene control region which is active in liver (Pinkert et al., Genes and Devel., 1:268-276, 1987), α-fetoprotein gene control region which is active in liver (Krumlauf et al., Mol. Cell. Biol., 5:1639-1648, 1985; Hammer et al., Science, 235:53-58, 1987; α 1-antitrypsin gene control region which is active in the liver (Kelsey et al., Genes and Devel., 1:161-171, 1987), beta-globin gene control region which is active in myeloid cells (Mogram et al., Nature, 315:338-340, 1985; Kollias et al., Cell, 46:89-94, 1986; myelin basic protein gene control region which is active in oligodendrocyte cells in the brain (Readhead et al., Cell, 48:703-712, 1987); myosin light chain-2 gene control region which is active in skeletal muscle (Sani, Nature, 314:283-286, 1985); neuronal-specific enolase (NSE) which is active in neuronal cells (Morelli et al., Gen. Virol., 80:571-83, 1999); brain-derived neurotrophic factor (BDNF) gene control region which is active in neuronal cells (Tabuchi et al., Biochem. Biophysic. Res. Comprising., 253:818-823, 1998); glial fibrillary acidic protein (GFAP) promoter which is active in astrocytes (Gomes et al., Braz. J. Med. Biol. Res., 32(5):619-631, 1999; Morelli et al., Gen. Virol., 80:571-83,1999) and gonadotropic releasing hormone gene control region which is active in the hypothalamus (Mason et al., Science, 234:1372-1378, 1986).

In a specific embodiment, the expression of a fusion protein is regulated by a constitutive promoter. In another embodiment, the expression of a fusion protein is regulated by an inducible promoter. In accordance with these embodiments, the promoter may be a tissue-specific promoter.

In a specific embodiment, a vector is used that comprises a promoter operably linked to a fusion protein-encoding nucleic acid, one or more origins of replication, and, optionally, one or more selectable markers (e.g., an antibiotic resistance gene).

In mammalian host cells, a number of viral-based expression systems may be utilized. In cases where an adenovirus is used as an expression vector, the fusion protein coding sequence may be ligated to an adenovirus transcription/translation control complex, e.g., the late promoter and tripartite leader sequence. This chimeric gene may then be inserted in the adenovirus genome by in vitro or in vivo recombination. Insertion in a non-essential region of the viral genome (e.g., region E1 or E3) will result in a recombinant virus that is viable and capable of expressing the antibody molecule in infected hosts (e.g., see Logan & Shenk, Proc. Natl. Acad. Sci. USA, 81:355-359,1984). Specific initiation signals may also be required for efficient translation of inserted fusion protein coding sequences. These signals include the ATG initiation codon and adjacent sequences. Furthermore, the initiation codon must be in phase with the reading frame of the desired coding sequence to ensure translation of the entire insert. These exogeneous translation control signals and initiation codons can be of a variety of origins, both natural and synthetic. The efficiency of expression may be enhanced by the inclusion of appropriate transcription enhancer elements, transcription terminators, etc. (see Bitter et al., Methods in Enzymol., 153:516-544,1987).

Expression vectors containing inserts of a gene encoding a fusion protein can be identified by three general approaches: (a) nucleic acid hybridization, (b) presence or absence of "marker" gene functions, and (c) expression of inserted sequences. In the first approach, the presence of a gene encoding a fusion protein in an expression vector can be detected by nucleic acid hybridization using probes comprising sequences that are homologous to an inserted gene encoding the fusion protein. In the second approach, the recombinant vector/host system can be identified and selected based upon the presence or absence of certain "marker" gene functions (e.g. thymidine kinase activity, resistance to antibiotics, transformation phenotype, occlusion body formation in baculovirus, etc.) caused by the insertion of a nucleotide sequence encoding a fusion protein in the vector. For example, if the nucleotide sequence encoding the fusion protein is inserted within the marker gene sequence of the vector, recombinants containing the gene encoding the fusion protein insert can be identified by the absence of the marker gene function. In the third approach, recombinant expression vectors can be identified by assaying the gene product (i.e., fusion protein) expressed by the recombinant. Such assays can be based, for example, on the physical or functional properties of the fusion protein in in vitro assay systems, e.g., binding with anti-bioactive molecule antibody.

In addition, a host cell strain may be chosen which modulates the expression of the inserted sequences, or modifies and processes the gene product in the specific fashion desired. Expression from certain promoters can be elevated in the presence of certain inducers; thus, expression of the genetically engineered fusion protein may be controlled. Furthermore, different host cells have characteristic and specific mechanisms for the translational and post-translational processing and modification (e.g., glycosylation, phosphorylation of proteins). Appropriate cell lines or host systems can be chosen to ensure the desired modification and processing of the foreign protein expressed. For example, expression in a bacterial system will produce an unglycosylated product and expression in yeast will produce a glycosylated product. Eukaryotic host cells which possess the cellular machinery for proper processing of the primary transcript, glycosylation, and phosphorylation of the gene product may be used. Such mammalian host cells include but are not limited to CHO, VERY, BHK, HeLa, COS, MDCK, 293, 3T3, WI38, and in particular, neuronal cell lines such as, for example, SK-N-AS, SK-N-FJ, SK-N-DZ human neuroblastomas (Sugimoto et al., J. Natl. Cancer Inst., 73: 51-57, 1984), SK-N-SH human neuroblastoma (Biochim. Biophys. Acta, 704: 450-460, 1982), Daoy human cerebellar medulloblastoma (He et al., Cancer Res., 52: 1144-1148, 1992) DBTRG-05MG glioblastoma cells (Kruse et al., 1992, In Vitro Cell. Dev. Biol., 28A:609-614, 1992), IMR-32 human neuroblastoma (Cancer Res., 30: 2110-2118, 1970), 1321N1 human astrocytoma (Proc. Natl Acad. Sci. USA, 74: 4816,1997), MOG-G-CCM human astrocytoma (Br. J. Cancer, 49: 269, 1984), U87MG human glioblastoma-astrocytoma (Acta Pathol. Microbiol. Scand., 74: 465-486, 1968), A172 human glioblastoma (Olopade et al., Cancer Res., 52: 2523-2529, 1992), C6 rat glioma cells (Benda et al., Science, 161: 370-371, 1968), Neuro-2a mouse neuroblastoma (Proc. Natl. Acad. Sci. USA, 65: 129-136, 1970), NB41A3 mouse neuroblastoma (Proc. Natl. Acad. Sci. USA, 48: 1184-1190, 1962), SCP sheep choroid plexus (Bolin et al., J. Virol. Methods, 48: 211-221, 1994), G355-5, PG-4 Cat normal astrocyte (Haapala et al., J. Virol., 53: 827-833, 1985), Mpf ferret brain (Trowbridge et al., In Vitro, 18: 952-960, 1982), and normal cell lines such as, for example, CTX TNA2 rat normal cortex brain (Radany et al., Proc. Natl. Acad. Sci. USA, 89: 6467-6471, 1992) such as, for example, CRL7030 and Hs578Bst. Furthermore, different vector/host expression systems may effect processing reactions to different degrees.

For long-term, high-yield production of recombinant proteins, stable expression may be desirable. For example, cell lines which stably express the fusion protein may be engineered. Rather than using expression vectors which contain viral origins of replication, host cells can be transformed with DNA controlled by appropriate expression control elements (e.g., promoter, enhancer, sequences, transcription terminators, polyadenylation sites, etc.), and a selectable marker. Following the introduction of the foreign DNA, engineered cells may be allowed to grow for 1-2 days in an enriched medium, and then are switched to a selective medium. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines that express the differentially expressed or pathway gene protein. Such engineered cell lines may be particularly useful in screening and evaluation of compounds that affect the endogenous activity of the differentially expressed or pathway gene protein.

A number of selection systems may be used, including but not limited to the herpes simplex virus thymidine kinase (Wigler, et al., Cell, 11:223, 1997), hypoxanthineguanine phosphoribosyltransferase (Szybalska & Szybalski, Proc. Natl. Acad. Sci. USA, 48:2026, 1962), and adenine phosphoribosyltransferase (Lowy, et al., 1980, Cell, 22:817, 1980) genes can be employed in tk-, hgprt- or aprt- cells, respectively. Also, antimetabolite resistance can be used as the basis of selection for dhfr, which confers resistance to methotrexate (Wigler, et al., Natl. Acad. Sci. USA, 77:3567, 1980; O'Hare, et al., Proc. Natl. Acad. Sci. USA, 78:1527, 1981); gpt, which confers resistance to mycophenolic acid (Mulligan & Berg, Proc. Natl. Acad. Sci. USA, 78:2072,1981); neo, which confers resistance to the aminoglycoside G-418 (Colberre-Garapin, et al., J. Mol. Biol., 150:1, 1981); and hygro, which confers resistance to hygromycin (Santerre, et al., Gene, 30:147, 1984) genes.

Once a fusion protein of the invention has been produced by recombinant expression, it may be purified by any method known in the art for purification of a protein, for example, by chromatography (e.g., ion exchange, affinity, particularly by affinity for the specific antigen after Protein A, and sizing column chromatography), centrifugation, differential solubility, or by any other standard technique for the purification of proteins.

### 5.6 PROPHYLACTIC AND THERAPEUTIC USES OF ANTIBODIES

The present invention encompasses antibody-based therapies which involve administering antibodies, or portions thereof to an animal, such as a mammal, e.g., a human, for preventing, treating, or ameliorating symptoms associated with a disease, disorder, or infection. Prophylactic and therapeutic compounds of the invention include, but are not limited to, antibodies and nucleic acids encoding antibodies. Antibodies and portions thereof may be provided in pharmaceutically acceptable compositions as known in the art or as described herein.

Antibodies and portions thereof that function as antagonists of a disease, disorder, or infection can be administered to an animal, preferably a mammal and most preferably a human, to treat, prevent or ameliorate one or more symptoms associated with the disease, disorder, or infection. For example, antibodies which disrupt or prevent the interaction between a viral antigen and its host cell receptor may be administered to an animal, preferably a mammal and most preferably a human, to treat, prevent or ameliorate one or more symptoms associated with a viral infection. Antibodies can also recognize and bind to target antigens found on the surface of diseased cells and tissues, such as, for example, cancerous cells and/or tumors or inflamed tissues and activate an immune response to treat, prevent or ameliorate the diseases caused by said cancerous cells and/or tumors or said inflamed tissues.

In a specific embodiment, an antibody or fragment thereof prevents a viral or bacterial antigen from binding to its host cell receptor by at least 99%, at least 95%, at least 90%, at least 85%, at least 80%, at least 75%, at least 70%, at least 60%, at least 50%, at least 45%, at least 40%, at least 45%, at least 35%, at least 30%, at least 25%, at least 20%, or at least 10% relative to antigen binding to its host cell receptor in the absence of said antibodies. In another embodiment, a combination of antibodies and/or fragments thereof prevent a viral or bacterial antigen from binding to its host cell receptor by at least 99%, at least 95%, at least 90%, at least 85%, at least 80%, at least 75%, at least 70%, at least 60%, at least 50%, at least 45%, at least 40%, at least 45%, at least 35%, at least 30%, at least 25%, at least 20%, or at least 10% relative to antigen binding to its host cell receptor in the absence of said antibodies. In one embodiment, the antibody is used to treat or prevent RSV infection. In another embodiment, the antibody is used to treat, prevent or ameliorate tumor growth and/or metastasis and/or cancerous cells and tissues. In yet another embodiment, the antibody is used to treat, prevent or ameliorate inflamed tissues.

Antibodies and portions thereof which do not prevent a viral or bacterial antigen from binding its host cell receptor but inhibit or downregulate viral or bacterial replication can also be administered to an animal to treat, prevent or ameliorate one or more symptoms associated with a viral or bacterial infection. The ability of an antibody to inhibit or downregulate viral or bacterial replication may be determined by techniques described herein or otherwise known in the art. For example, the inhibition or downregulation of viral replication can be determined by detecting the viral titer in the animal.

In a specific embodiment, an antibody or portion thereof inhibits or downregulates viral or bacterial replication by at least 99%, at least 95%, at least 90%, at least 85%, at least 80%, at least 75%, at least 70%, at least 60%, at least 50%, at least 45%, at least 40%, at least 45%, at least 35%, at least 30%, at least 25%, at least 20%, or at least 10% relative to viral or bacterial replication in absence of said antibody. In another embodiment, a combination of antibodies inhibit or downregulate viral or bacterial replication by at least 99%, at least 95%, at least 90%, at least 85%, at least 80%, at least 75%, at least 70%, at least 60%, at least 50%, at least 45%, at least 40%, at least 45%, at least 35%, at least 30%, at least 25%, at least 20%, or at least 10% relative to viral or bacterial replication in absence of said antibodies.

Antibodies can also be used to prevent, inhibit or reduce the growth or metastasis of cancerous cells. In a specific embodiment, an antibody inhibits or reduces the growth or metastasis of cancerous cells by at least 99%, at least 95%, at least 90%, at least 85%, at least 80%, at least 75%, at least 70%, at least 60%, at least 50%, at least 45%, at least 40%, at least 45%, at least 35%, at least 30%, at least 25%, at least 20%, or at least 10% relative to the growth or metastasis in absence of said antibody. In another embodiment, a combination of antibodies inhibits or reduces the growth or metastasis of cancer by at least 99%, at least 95%, at least 90%, at least 85%, at least 80%, at least 75%, at least 70%, at least 60%, at least 50%, at least 45%, at least 40%, at least 45%, at least 35%, at least 30%, at least 25%, at least 20%, or at least 10% relative to the growth or metastasis in absence of said antibodies. Examples of cancers include, but are not limited to, leukemia (e.g, acute leukemia such as acute lymphocytic leukemia and acute myelocytic leukemia), neoplasms, tumors (e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, and retinoblastoma), heavy chain disease, metastases, or any disease or disorder characterized by uncontrolled cell growth.

Antibodies and fragments thereof can also be used to reduce the inflammation experienced by animals, particularly mammals, with inflammatory disorders. In a specific embodiment, an antibody reduces the inflammation in an animal by at least 99%, at least 95%, at least 90%, at least 85%, at least 80%, at least 75%, at least 70%, at least 60%, at least 50%, at least 45% at least 40%, at least 45%, at least 35%, at least 30%, at least 25%, at least 20%, or at least 10% relative to the inflammation in an animal in the not administered said antibody. In another embodiment, a combination of antibodies reduce the inflammation in an animal by at least 99%, at least 95%, at least 90%, at least 85%, at least 80%, at least 75%, at least 70%, at least 60%, at least 50%, at least 45%, at least 40%, at least 45%, at least 35%, at least 30%, at least 25%, at least 20%, or at least 10% relative to the inflammation in an animal in not administered said antibodies. Examples of inflammatory disorders include, but are not limited to, rheumatoid arthritis, spondyloarthropathies, inflammatory bowel disease and asthma.

In certain embodiments, the antibody used for treatment of inflammation (or cancer) is a modified anti-αvβ3 antibody, preferably a Vitaxin antibody (see, PCT publications WO 98/33919 and WO 00/78815, both by Huse et al., and both of which are incorporated by reference herein in their entireties).

Antibodies can also be used to prevent the rejection of transplants. Antibodies can also be used to prevent clot formation. Further, antibodies that function as agonists of the immune response can also be administered to an animal, preferably a mammal, and most preferably a human, to treat, prevent or ameliorate one or more symptoms associated with the disease, disorder, or infection.

One or more antibodies that immunospecifically bind to one or more antigens may be used locally or systemically in the body as a therapeutic. The antibodies of this invention may also be advantageously utilized in combination with other monoclonal or chimeric antibodies, or with lymphokines or hematopoietic growth factors (such as, e.g., IL-2, IL-3 and IL-7), which, for example, serve to increase the number or activity of effector cells which interact with the antibodies. The antibodies of this invention may also be advantageously utilized in combination with other monoclonal or chimeric antibodies, or with lymphokines or hematopoietic growth factors (such as, e.g., IL-2, IL-3 and IL-7), which, for example, serve to increase the immune response. The antibodies of this invention may also be advantageously utilized in combination with one or more drugs used to treat a disease, disorder, or infection such as, for example anti-cancer agents, anti-inflammatory agents or anti-viral agents. Examples of anti-cancer agents include, but are not limited to, isplatin, ifosfamide, paclitaxel, taxanes, topoisomerase I inhibitors (e.g. CPT-11, topotecan, 9-AC, and GG-211), gemcitabine, vinorelbine, oxaliplatin, 5-fluorouracil (5-FU), leucovorin, vinorelbine, temodal, and taxol. Examples of anti-viral agents include, but are not limited to, cytokines (e.g., IFN-α, IFN-β, IFN-.gamma.), inhibitors of reverse transcriptase (e.g., AZT, 3TC, D4T, ddC, ddI, d4T, 3TC, adefovir, efavirenz, delavirdine, nevirapine, abacavir, and other dideoxynucleosides or dideoxyfluoronucleosides), inhibitors of viral mRNA capping, such as ribavirin, inhibitors of proteases such HIV protease inhibitors (e.g., amprenavir, indinavir, nelfinavir, ritonavir, and saquinavir,), amphotericin B, castanospermine as an inhibitor of glycoprotein processing, inhibitors of neuraminidase such as influenza virus neuraminidase inhibitors (e.g., zanamivir and oseltamivir), topoisomerase I inhibitors (e.g., camptothecins and analogs thereof), amantadine, and rimantadine. Examples of anti-inflammatory agents include, but are not limited to, nonsteroidal anti-inflammatory drugs such as COX-2 inhibitors (e.g., meloxicam, celecoxib, rofecoxib, flosulide, and SC-58635, and MK-966), ibuprofen and indomethacin, and steroids (e.g., deflazacort, dexamethasone and methylprednisolone).

In a specific embodiment, antibodies administered to an animal are of a species origin or species reactivity that is the same species as that of the animal. Thus, in a preferred embodiment, human or humanized antibodies, or nucleic acids encoding human or human, are administered to a human patient for therapy or prophylaxis.

In some embodiments, immunoglobulins having decreased in vivo half-lives are used in passive immunotherapy (for either therapy or prophylaxis). In one embodiment, the therapeutic/prophylactic is an antibody that binds RSV, for example, SYNAGIS®, NUMAX™, or other anti-RSV antibody. Such anti-RSV antibodies, and methods of administration are disclosed in U.S. patent application Ser. Nos. 09/724,396 and 09/724,531, both entitled "Methods of Administering/Dosing Anti-RSV Antibodies For Prophylaxis and Treatment," both by Young et al., both filed Nov. 28, 2000, and continuation-in-part applications of U.S. 6,855,493 and 6,818,216, respectively, also entitled "Methods of Administering/Dosing Anti-RSV Antibodies for Prophylaxis and Treatment," by Young et al., all which are incorporated by reference herein in their entireties.

In a specific embodiment, fusion proteins administered to an animal are of a species origin or species reactivity that is the same species as that of the animal. Thus, in a preferred embodiment, human fusion proteins or nucleic acids encoding human fusion proteins, are administered to a human subject for therapy or prophylaxis.

### 5.7 PROPHYLACTIC AND THERAPEUTIC USES OF FUSION PROTEINS AND CONJUGATED MOLECULES

The present invention encompasses fusion protein-based and conjugated molecule-based therapies which involve administering fusion proteins or conjugated molecules to an animal, such as a mammal, e.g. a human, for preventing, treating, or ameliorating symptoms associated with a disease, disorder, or infection. Prophylactic and therapeutic compounds of the invention include, but are not limited to, fusion proteins and nucleic acids encoding fusion proteins and conjugated molecules. Fusion proteins and conjugated molecules may be provided in pharmaceutically acceptable compositions as known in the art or as described herein.

Fusion proteins and conjugated molecules of the present invention that function as antagonists of a disease, disorder, or infection can be administered to an animal, such as a mammal, e.g., a human, to treat, prevent or ameliorate one or more symptoms associated with the disease, disorder, or infection. Further, fusion proteins and conjugated molecules of the present invention that function as agonists of the immune response may be administered to an animal, such as a mammal, e.g., a human, to treat, prevent or ameliorate one or more symptoms associated with the disease, disorder, or infection.

One or more fusion proteins and conjugated molecules may be used locally or systemically in the body as a therapeutic. The fusion proteins and conjugated molecules of this invention may also be advantageously utilized in combination with monoclonal or chimeric antibodies, or with lymphokines or hematopoietic growth factors (such as, e.g., IL-2, IL-3 and IL-7), which, for example, serve to increase the number or activity of effector cells which interact with the antibodies. The fusion proteins and conjugated molecules of this invention may also be advantageously utilized in combination with monoclonal or chimeric antibodies, or with lymphokines or hematopoietic growth factors (such as, e.g., IL-2, IL-3 and IL-7), which, for example, serve to increase the immune response. The fusion proteins and conjugated molecules of this invention may also be advantageously utilized in combination with one or more drugs used to treat a disease, disorder, or infection such as, for example anti-cancer agents, anti-inflammatory agents or anti-viral agents. Examples of anti-cancer agents include, but are not limited to, isplatin, ifosfamide, paclitaxel, taxanes, topoisomerase I inhibitors (e.g., CPT-11, topotecan, 9-AC, and GG-211), gemcitabine, vinorelbine, oxaliplatin, 5-fluorouracil (5-FU), leucovorin, vinorelbine, temodal, and taxol. Examples of anti-viral agents include, but are not limited to, cytokines (e.g, IFN-α, IFN-β, IFN-.gamma.), inhibitors of reverse transcriptase (e.g., AZT, 3TC, D4T, ddC, ddI, d4T, 3TC, adefovir, efavirenz, delavirdine, nevirapine, abacavir, and other dideoxynucleosides or dideoxyfluoronucleosides), inhibitors of viral mRNA capping, such as ribavirin, inhibitors of proteases such HIV protease inhibitors (e.g., amprenavir, indinavir, nelfinavir, ritonavir, and saquinavir,), amphotericin B, castanospermine as an inhibitor of glycoprotein processing, inhibitors of neuraminidase such as influenza virus neuraminidase inhibitors (e.g., zanamivir and oseltamivir), topoisomerase I inhibitors (e.g., camptothecins and analogs thereof), amantadine, and rimantadine. Examples of anti-inflammatory agents include, but are not limited to, nonsteroidal anti-inflammatory drugs such as COX-2 inhibitors (e.g., meloxicam, celecoxib, rofecoxib, flosulide, and SC-58635, and MK-966), ibuprofen and indomethacin, and steroids (e.g., deflazacort, dexamethasone and methylprednisolone).

### 5.8. ADMINISTRATION OF ANTIBODIES OR FUSION PROTEINS

The invention provides methods of treatment, prophylaxis, and amelioration of one or more symptoms associated with a disease, disorder or infection by administering to a subject of an effective amount of an antibody, a fusion protein, or a conjugated molecule or pharmaceutical composition comprising an antibody, a fusion protein, or a conjugated molecule. In a preferred aspect, an antibody, a fusion protein or a conjugated molecule, is substantially purified (i.e., substantially free from substances that limit its effect or produce undesired side-effects). In a specific embodiment, the subject is an animal, such as a mammal, such as non-primate (e.g., cows, pigs, horses, cats, dogs, rats etc.) and a primate (e.g., monkey such as a cynomolgous monkey and a human). In a preferred embodiment, the subject is a human.

Various delivery systems are known and can be used to administer a polypeptide of the invention, e.g., encapsulation in liposomes, microparticles, microcapsules, recombinant cells capable of expressing the antibody or fusion protein, receptor-mediated endocytosis (see, e.g., Wu and Wu, J. Biol. Chem., 262:4429-4432, 1987), construction of a nucleic acid as part of a retroviral or other vector, etc. Methods of administering an antibody, a fusion protein or conjugated molecule, or pharmaceutical composition include, but are not limited to, parenteral administration (e.g., intradermal, intramuscular, intraperitoneal, intravenous and subcutaneous), epidural, and mucosal (e.g., intranasal, see e.g., WO 03/086443, which is incorporated by reference in its entirety, and oral routes). In a specific embodiment, antibodies, fusion proteins, conjugated molecules, or pharmaceutical compositions are administered intramuscularly, intravenously, or subcutaneously. The compositions may be administered by any convenient route, either systemic or local, for example by infusion or bolus injection, or by absorption through epithelial or mucocutaneous linings (e.g., oral mucosa, rectal and intestinal mucosa, etc.) and may be administered together with other biologically active agents. Administration can be systemic or local. In addition, pulmonary administration can also be employed, e.g., by use of an inhaler or nebulizer, and formulation with an aerosolizing agent. See, e.g., U.S. Pat. Nos. 6,019,968; 5,985, 320; 5,985,309; 5,934,272; 5,874,064; 5,855,913; 5,290,540; and 4,880,078; and PCT Publication Nos. WO 92/19244; WO 97/32572; WO 97/44013; WO 98/31346; WO 99/66903, WO 04/058156, WO 03/087339, WO 03/087335 and WO 03/087327, each of which is incorporated herein by reference in its entirety. Such pulmonary or intranasal or other mucosal administration may be employed to deliver the antibody or fusion protein or conjugated molecule of the invention systemically. In a preferred embodiment, an antibody, a fusion protein, conjugated molecules, or a pharmaceutical composition is administered using Alkermes AIR™ pulmonary drug delivery technology (Alkermes, Inc., Cambridge, Mass.).

The invention also provides that the polypeptide may be packaged in a hermetically sealed container such as an ampoule or sachette indicating the quantity of molecule, e.g., antibody, fusion protein, or conjugated molecule. In one embodiment, an antibody, fusion protein, or conjugated molecule is supplied as a dry sterilized lyophilized powder or water free concentrate in a hermetically sealed container and can be reconstituted, e.g., with water or saline to the appropriate concentration for administration to a subject. An antibody, fusion protein, or conjugated molecule may be supplied as a dry sterile lyophilized powder in a hermetically sealed container at a unit dosage of at least 5 mg, at least 10 mg, at least 15 mg, at least 25 mg, at least 35 mg, at least 45 mg, at least 50 mg, or at least 75 mg. A lyophilized antibody, fusion protein, or conjugated molecule may be stored at between 2 and 8 C in its original container and the antibody, fusion protein, or conjugated molecules may be administered within 12 hours, within 6 hours, within 5 hours, within 3 hours, or within 1 hour after being reconstituted. In an alternative embodiment, an antibody, fusion protein, or conjugated molecule is supplied in liquid form in a hermetically sealed container indicating the quantity and concentration of the antibody, fusion protein, or conjugated molecule. S liquid form of the antibody, fusion protein, or conjugated molecule may be supplied in a hermetically sealed container at least 1 mg/ml, at least 2.5 mg/ml, at least 5 mg/ml, at least 8 mg/ml, at least 10 mg/ml, at least 15 mg/kg, or at least 25 mg/ml.

In one embodiment, it may be desirable to administer compositions of the invention locally to the area in need of treatment; this may be achieved by, for example, and not by way of limitation, local infusion, by injection, or by means of an implant, said implant being of a porous, non-porous, or gelatinous material, including membranes, such as sialastic membranes, or fibers. When administering an antibody or a fusion protein, care must be taken to use materials to which the antibody or the fusion protein does not absorb.

In another embodiment, the composition can be delivered in a vesicle, in particular a liposome (see Langer, Science, 249:1527-1533, 1990; Treat et al., in Liposomes in the Therapy of Infectious Disease and Cancer, Lopez-Berestein and Fidler (eds.), Liss, New York, pp. 353-365 (1989); Lopez-Berestein, ibid., pp. 3 17-327; see generally ibid.).

In yet another embodiment, the composition can be delivered in a controlled release or sustained release system. Any technique known to one of skill in the art can be used to produce sustained release formulations comprising one or more antibodies, or one or more fusion proteins. See, e.g. U.S. Pat. No. 4,526,938; PCT publication WO 91/05548; PCT publication WO 96/20698; Ning et al., "Intratumoral Radioimmunotheraphy of a Human Colon Cancer Xenograft Using a Sustained-Release Gel," Radiotherapy & Oncology, 39:179-189, 1996; Song et al., "Antibody Mediated Lung Targeting of Long-Circulating Emulsions," PDA Journal of Pharmaceutical Science & Technology, 50:372-397, 1995; Cleek et al., "Biodegradable Polymeric Carriers for a bFGF Antibody for Cardiovascular Application," Pro. Intl. Symp. Control. Rel. Bioact. Mater., 24:853-854, 1997; and Lam et al., "Microencapsulation of Recombinant Humanized Monoclonal Antibody for Local Delivery," Proc. Int'l. Symp. Control Rel. Bioact. Mater., 24:759-760, 1997, each of which is incorporated herein by reference in its entirety. In one embodiment, a pump may be used in a controlled release system (see Langer, supra; Sefton, CRC Crit. Ref. Biomed. Eng., 14:20, 1987; Buchwald et al., Surgery, 88:507, 1980; and Saudek et al., N. Engl. J. Med., 321:574, 1989). In another embodiment, polymeric materials can be used to achieve controlled release of antibodies or fusion proteins (see e.g., Medical Applications of Controlled Release, Langer and Wise (eds.), CRC Pres., Boca Raton, Fla. (1974); Controlled Drug Bioavailability, Drug Product Design and Performance, Smolen and Ball (eds.), Wiley, N.Y. (1984); Ranger and Peppas, J., Macromol. Sci. Rev. Macromol. Chem., 23:61, 1983; see also Levy et al., Science, 228:190, 1985; During et al., Ann. Neurol., 25:351, 1989; Howard et al., J. Neurosurg., 7 1:105, 1989); U.S. Pat. No. 5,679,377; U.S. Pat. No. 5,916,597; U.S. Pat. No. 5,912,015; U.S. Pat. No. 5,989,463; U.S. Pat. No. 5,128,326; PCT Publication No. WO 99/15154; and PCT Publication No. WO 99/20253). In yet another embodiment, a controlled release system can be placed in proximity of the therapeutic target (e.g., the lungs), thus requiring only a fraction of the systemic dose (see, e.g., Goodson, in Medical Applications of Controlled Release, supra, vol. 2, pp. 115-138 (1984)).

Other controlled release systems are discussed in the review by Langer, Science, 249:1527-1533, 1990).

In one embodiment, if the composition of the invention is a nucleic acid encoding an antibody or fusion protein, the nucleic acid can be administered in vivo to promote expression of its encoded antibody or fusion protein, by constructing it as part of an appropriate nucleic acid expression vector and administering it so that it becomes intracellular, e.g., by use of a retroviral vector (see U.S. Pat. No. 4,980,286), or by direct injection, or by use of microparticle bombardment (e.g., a gene gun; Biolistic, Dupont), or coating with lipids or cell-surface receptors or transfecting agents, or by administering it in linkage to a homeobox-like peptide which is known to enter the nucleus (see e.g., Joliot et al., Proc. Natl. Acad. Sci. USA, 88:1864-1868, 1991), etc. Alternatively, a nucleic acid can be introduced intracellularly and incorporated within host cell DNA for expression by homologous recombination.

The present invention also provides pharmaceutical compositions. Such compositions comprise a prophylactically or therapeutically effective amount of an antibody, fusion protein or conjugated molecule, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant (e.g., Freund's complete and incomplete, mineral gels such as aluminum hydroxide, surface active substances such as lysolecithin, pluronic polyols, polyanions, peptides, oil emulsions, keyhole limpet hemocyanins, dinitrophenol, and potentially useful adjuvants for humans such as BCG (Bacille Calmette-Guerin) and Corynebacterium parvum), excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water may be a desirable carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E. W. Martin. Such compositions will contain a prophylactically or therapeutically effective amount of the antibody or fragment thereof, or fusion protein or conjugated molecule, preferably in purified form, together with a suitable amount of carrier so as to provide the form for proper administration to the patient. The formulation should suit the mode of administration.

In one embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic, such as lignocaine, to ease pain at the site of the injection.

The ingredients of compositions of the invention may be supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration. Where the composition is administered by a pulmonary (i.e., inhalation) or intranasal route, either a lyophilized powder to be subsequently reconstituted, or a stable liquid formulation can be filled into vials or a syringe or an atomizer.

The compositions of the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

The amount of the composition of the invention which will be effective in the treatment, prevention or amelioration of one or more symptoms associated with a disease, disorder, or infection can be determined by standard clinical techniques. The precise dose to be employed in the formulation will depend on the route of administration, the age of the subject, and the seriousness of the disease, disorder, or infection, and should be decided according to the judgment of the practitioner and each patient's circumstances. Effective doses may be extrapolated from dose-response curves derived from in vitro or animal model (e.g., the cotton rat or Cynomolgous monkey) test systems.

For fusion proteins, the therapeutically or prophylactically effective dosage administered to a subject may range from about 0.001 to 50 mg/kg body weight, about 0.01 to 25 mg/kg body weight, about 0.1 to 20 mg/kg body weight, or about 1 to 10 mg/kg, 2 to 9 mg/kg, 3 to 8 mg/kg, 4 to 7 mg/kg, or 5 to 6 mg/kg body weight. For antibodies, the therapeutically or prophylactically effective dosage administered to a subject may be 0.1 mg/kg to 200 mg/kg of the subject's body weight. The dosage administered to a subject may be between 0.1 mg/kg and 20 mg/kg of the subject's body weight, or between 1 mg/kg to 10 mg/kg of the subject's body weight. Generally, human antibodies and human fusion proteins have longer half-lives within the human body than antibodies of fusion proteins from other species due to the immune response to the foreign polypeptides.

Treatment of a subject with a therapeutically or prophylactically effective amount of an antibody, fusion protein, or conjugated molecule can include a single treatment or, can include a series of treatments. In one example, a subject is treated with an antibody, fusion protein, or conjugated molecule in the range of between about 0.1 to 30 mg/kg body weight, one time per week for between about 1 to 10 weeks, preferably between 2 to 8 weeks, more preferably between about 3 to 7 weeks, and even more referably for about 4, 5, or 6 weeks. In other embodiments, the pharmaceutical composition of the invention is administered once a day, twice a day, or three times a day. In other embodiments, the pharmaceutical composition is administered once a week, twice a week, once every two weeks, once a month, once every six weeks, once every two months, twice a year or once per year. It will also be appreciated that the effective dosage of the antibody, fusion protein, or conjugated molecule used for treatment may increase or decrease over the course of a particular treatment.

### 5.8.1. GENE THERAPY

In one embodiment, nucleic acids comprising sequences encoding antibodies or fusion proteins, are administered to treat, prevent or ameliorate one or more symptoms associated with a disease, disorder, or infection, by way of gene therapy. Gene therapy refers to therapy performed by the administration to a subject of an expressed or expressible nucleic acid. In this embodiment of the invention, the nucleic acids produce their encoded antibody or fusion protein that mediates a therapeutic or prophylactic effect.

Any of the methods for gene therapy available in the art can be used according to the present invention. Example methods are described below.

For general reviews of the methods of gene therapy, see Goldspiel et al., Clinical Pharmacy, 12:488-505, 1993; Wu and Wu, Biotherapy, 3:87-95, 1991; Tolstoshev, Ann. Rev. Pharmacol. Toxicol., 32:573-596, 1993; Mulligan, Science, 260:926-932, 1993; and Morgan and Anderson, Ann. Rev. biochem. 62:191-217, 1993; TIBTECH 11(5):155-215, 1993. Methods commonly known in the art of recombinant DNA technology which can be used are described in Ausubel et al. (eds.), Current Protocols in Molecular Biology, John Wiley & Sons, NY (1993); and Kriegler, Gene Transfer and Expression, A Laboratory Manual, Stockton Press, NY (1990).

In one aspect, a composition of the invention comprises nucleic acids encoding an antibody, said nucleic acids being part of an expression vector that expresses the antibody in a suitable host. In particular, such nucleic acids have promoters, preferably heterologous promoters, operably linked to the antibody coding region, said promoter being inducible or constitutive, and, optionally, tissue-specific. In another aspect, nucleic acid molecules are used in which the antibody coding sequences and any other desired sequences are flanked by regions that promote homologous recombination at a desired site in the genome, thus providing for intrachromosomal expression of the antibody encoding nucleic acids (Koller and Smithies, Proc. Natl. Acad. Sci. USA, 86:8932-8935, 1989; and Zijlstra et al., Nature, 342:435-438,1989).

In another aspect, a composition of the invention comprises nucleic acids encoding a fusion protein, said nucleic acids being a part of an expression vector that expression the fusion protein in a suitable host. In particular, such nucleic acids have promoters, preferably heterologous promoters, operably linked to the coding region of a fusion protein, said promoter being inducible or constitutive, and optionally, tissue-specific. In another particular embodiment, nucleic acid molecules are used in which the coding sequence of the fusion protein and any other desired sequences are flanked by regions that promote homologous recombination at a desired site in the genome, thus providing for intrachromosomal expression of the fusion protein encoding nucleic acids.

Delivery of the nucleic acids into a subject may be either direct, in which case the subject is directly exposed to the nucleic acid or nucleic acid-carrying vectors, or indirect, in which case, cells are first transformed with the nucleic acids in vitro, then transplanted into the subject. These two approaches are known, respectively, as in vivo or ex vivo gene therapy.

In one embodiment, the nucleic acid sequences are directly administered in vivo, where it is expressed to produce the encoded product. This can be accomplished by any of numerous methods known in the art, e.g., by constructing them as part of an appropriate nucleic acid expression vector and administering it so that they become intracellular, e.g., by infection using defective or attenuated retroviral or other viral vectors (see U.S. Pat. No. 4,980,286), or by direct injection of naked DNA, or by use of microparticle bombardment (e.g., a gene gun; Biolistic, Dupont), or coating with lipids or cell-surface receptors or transfecting agents, encapsulation in liposomes, microparticles, or microcapsules, or by administering them in linkage to a peptide which is known to enter the nucleus, by administering it in linkage to a ligand subject to receptor-mediated endocytosis (see, e.g., Wu and Wu, J. Biol. Chem., 262:4429-4432, 1987) (which can be used to target cell types specifically expressing the receptors), etc. In another embodiment, nucleic acid-ligand complexes can be formed in which the ligand comprises a fusogenic viral peptide to disrupt endosomes, allowing the nucleic acid to avoid lysosomal degradation. In yet another embodiment, the nucleic acid can be targeted in vivo for cell specific uptake and expression, by targeting a specific receptor (see, e.g., PCT Publications WO 92/06180; WO 92/22635; WO 92/20316; WO 93/14188; WO 93/20221). Alternatively, the nucleic acid can be introduced intracellularly and incorporated within host cell DNA for expression, by homologous recombination (Koller and Smithies, Proc. Natl. Acad. Sci. USA, 86:8932-8935, 1989; and Zijlstra et al., Nature, 342:435-438,1989).

In one embodiment, viral vectors that contain nucleic acid sequences encoding an antibody or a fusion protein can be used. For example, a retroviral vector can be used (see Miller et al., Meth. Enzymol., 217:581-599, 1993). These retroviral vectors contain the components necessary for the correct packaging of the viral genome and integration into the host cell DNA. The nucleic acid sequences encoding the antibody or a fusion protein to be used in gene therapy are cloned into one or more vectors, which facilitates delivery of the nucleotide sequence into a subject. More detail about retroviral vectors can be found in Boesen et al., Biotherapy, 6:291-302, 1994, which describes the use of a retroviral vector to deliver the mdr 1 gene to hematopoietic stem cells in order to make the stem cells more resistant to chemotherapy. Other references illustrating the use of retroviral vectors in gene therapy include: Clowes et al., J. Clin. Invest., 93:644-651,1994; Klein et al., Blood 83:1467-1473, 1994; Salmons and Gunzberg, Human Gene Therapy, 4:129-141, 1993; and Grossman and Wilson, Curr. Opin. in Genetics and Devel., 3:110-114, 1993.

Adenoviruses are other viral vectors that can be used in gene therapy. Adenoviruses can be attractive vehicles for delivering genes to respiratory epithelia. Adenoviruses naturally infect respiratory epithelia where they cause a mild disease. Other targets for adenovirus-based delivery systems include liver, the central nervous system, endothelial cells, and muscle. Adenoviruses have the advantage of being capable of infecting non-dividing cells. Kozarsky and Wilson, Current Opinion in Genetics and Development, 3:499-503, 1993, present a review of adenovirus-based gene therapy. Bout et al., Human Gene Therapy, 5:3-10, 1994, demonstrated the use of adenovirus vectors to transfer genes to the respiratory epithelia of rhesus monkeys. Other instances of the use of adenoviruses in gene therapy can be found in Rosenfeld et al., Science, 252:431-434, 1991; Rosenfeld et al., Cell, 68:143-155, 1992; Mastrangeli et al., J. Clin. Invest., 91:225-234, 1993; PCT Publication WO 94/12649; and Wang et al., Gene Therapy, 2:775-783, 1995. In a preferred embodiment, adenovirus vectors are used.

Adeno-associated virus (AAV) has also been proposed for use in gene therapy (see, e.g.,Walsh et al., Proc. Soc. Exp. Biol. Med., 204:289-300, 1993, and U.S. Pat. No. 5,436,146).

Another approach to gene therapy includes transferring a gene to cells in tissue culture by such methods as electroporation, lipofection, calcium phosphate mediated transfection, or viral infection. These methods of transfer may include the transfer of a selectable marker to the cells. The cells can then be placed under selection to isolate those cells that have taken up and are expressing the transferred gene. Those cells can then be delivered to a subject.

In one embodiment, the nucleic acid can be introduced into a cell prior to administration in vivo of the resulting recombinant cell. Such introduction can be carried out by any method known in the art, including but not limited to transfection, electroporation, microinjection, infection with a viral or bacteriophage vector containing the nucleic acid sequences, cell fusion, chromosome-mediated gene transfer, microcell-mediated gene transfer, spheroplast fusion, etc. Numerous techniques are known in the art for the introduction of foreign genes into cells (see, e.g., Loeffler and Behr, Meth. Enzymol., 217:599-618, 1993; Cohen et al., Meth. Enzymol., 217:618-644, 1993; and Clin. Pharma. Ther., 29:69-92, 1985) and may be used in accordance with the present invention, provided that the necessary developmental and physiological functions of the recipient cells are not disrupted. The technique should provide for the stable transfer of the nucleic acid to the cell, so that the nucleic acid is expressible by the cell. The stable transfer may also be heritable and expressible by its cell progeny.

The resulting recombinant cells can be delivered to a subject by various methods known in the art. Recombinant blood cells (e.g., hematopoietic stem or progenitor cells) are preferably administered intravenously. The amount of cells envisioned for use depends on the desired effect, patient state, etc., and can be determined by one skilled in the art.

Cells into which a nucleic acid can be introduced for purposes of gene therapy encompass any desired, available cell type, and include but are not limited to epithelial cells, endothelial cells, keratinocytes, fibroblasts, muscle cells, hepatocytes; blood cells such as T lymphocytes, B lymphocytes, monocytes, macrophages, neutrophils, eosinophils, megakaryocytes, granulocytes; various stem or progenitor cells, hematopoietic stem or progenitor cells, e.g., as obtained from bone marrow, umbilical cord blood, peripheral blood, fetal liver, etc.

Further, the cell used for gene therapy may be autologous to the subject.

In one embodiment in which recombinant cells are used in gene therapy, nucleic acid sequences encoding an antibody or a fusion protein are introduced into the cells such that they are expressible by the cells or their progeny, and the recombinant cells are then administered in vivo for therapeutic effect. In a specific embodiment, stem or progenitor cells are used. Any stem and/or progenitor cells which can be isolated and maintained in vitro can potentially be used in accordance with this embodiment of the present invention (see e.g., PCT Publication WO 94/08598; Stemple and Anderson, Cell, 7 1:973-985, 1992; Rheinwald, Meth. Cell Bio., 21A:229, 1980; and Pittelkow and Scott, Mayo Clinic Proc., 61:771, 1986).

In a specific embodiment, the nucleic acid to be introduced for purposes of gene therapy may comprise an inducible promoter operably linked to the coding region, such that expression of the nucleic acid is controllable by controlling the presence or absence of the appropriate inducer of transcription.

### 5.9 CHARACTERIZATION AND DEMONSTRATION OF THERAPEUTIC OR PROPHYLACTIC UTILITY

Antibodies, fusion proteins, and conjugated molecules of the present invention may be characterized in a variety of ways. In particular, antibodies of the invention may be assayed for the ability to immunospecifically bind to an antigen. Such an assay may be performed in solution (e.g., Houghten, Bio/Techniques, 13:412-421, 1992), on beads (Lam, Nature, 354:82-84, 1991, on chips (Fodor, Nature, 364:555-556, 1993), on bacteria (U.S. Pat. No. 5,223,409), on spores (U.S. Pat. Nos. 5,571,698; 5,403,484; and 5,223,409), on plasmids (Cull et al., Proc. Natl. Acad. Sci. USA, 89:1865-1869, 1992) or on phage (Scott and Smith, Science, 249:386-390, 1990; Devlin, Science, 249:404-406, 1990; Cwirla et al., Proc. Natl. Acad. Sci. USA, 87:6378-6382, 1990; and Felici, J. Mol. Biol., 222:301-310, 1991) (each of these references is incorporated herein in its entirety by reference). Antibodies that have been identified to immunospecifically bind to an antigen or a fragment thereof can then be assayed for their specificity affinity for the antigen.

The antibodies of the invention or fragments thereof may be assayed for immunospecific binding to an antigen and cross-reactivity with other antigens by any method known in the art. Immunoassays which can be used to analyze immunospecific binding and cross-reactivity include, but are not limited to, competitive and non-competitive assay systems using techniques such as western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, to name but a few. Such assays are routine and well known in the art (see, e.g, Ausubel et al, eds, 1994, Current Protocols in Molecular Biology, Vol. 1, John Wiley & Sons, Inc., New York, which is incorporated by reference herein in its entirety). Exemplary immunoassays are described briefly below (but are not intended by way of limitation).

Immunoprecipitation protocols generally comprise lysing a population of cells in a lysis buffer such as RIPA buffer (1% NP-40 or Triton X-100, 1% sodium deoxycholate, 0.1% SDS, 0.15 M NaCl, 0.01 M sodium phosphate at pH 7.2, 1% Trasylol) supplemented with protein phosphatase and/or protease inhibitors (e.g., EDTA, PMSF, aprotinin, sodium vanadate), adding the antibody of interest to the cell lysate, incubating for a period of time (e.g., 1 to 4 hours) at 40 C., adding protein A and/or protein G sepharose beads to the cell lysate, incubating for about an hour or more at 40 C., washing the beads in lysis buffer and resuspending the beads in SDS/sample buffer. The ability of the antibody of interest to immunoprecipitate a particular antigen can be assessed by, e.g., western blot analysis. One of skill in the art would be knowledgeable as to the parameters that can be modified to increase the binding of the antibody to an antigen and decrease the background (e.g., pre-clearing the cell lysate with sepharose beads). For further discussion regarding immunoprecipitation protocols see, e.g., Ausubel et al, eds, 1994, Current Protocols in Molecular Biology, Vol. 1, John Wiley & Sons, Inc., New York at 10.16.1.

Western blot analysis generally comprises preparing protein samples, electrophoresis of the protein samples in a polyacrylamide gel (e.g., 8%-20% SDS-PAGE depending on the molecular weight of the antigen), transferring the protein sample from the polyacrylamide gel to a membrane such as nitrocellulose, PVDF or nylon, blocking the membrane in blocking solution (e.g., PBS with 3% BSA or non-fat milk), washing the membrane in washing buffer (e.g., PBS-Tween 20), blocking the membrane with primary antibody (the antibody of interest) diluted in blocking buffer, washing the membrane in washing buffer, blocking the membrane with a secondary antibody (which recognizes the primary antibody, e.g., an anti-human antibody) conjugated to an enzymatic substrate (e.g., horseradish peroxidase or alkaline phosphatase) or radioactive molecule (e.g., .sup.32P or 125I) diluted in blocking buffer, washing the membrane in wash buffer, and detecting the presence of the antigen. One of skill in the art would be knowledgeable as to the parameters that can be modified to increase the signal detected and to reduce the background noise. For further discussion regarding western blot protocols see, e.g., Ausubel et al, eds, 1994, Current Protocols in Molecular Biology, Vol. 1, John Wiley & Sons, Inc., New York at 10.8.1.

ELISAs comprise preparing antigen, coating the well of a 96 well microtiter plate with the antigen, adding the antibody of interest conjugated to a detectable compound such as an enzymatic substrate (e.g., horseradish peroxidase or alkaline phosphatase) to the well and incubating for a period of time, and detecting the presence of the antigen. In ELISAs the antibody of interest does not have to be conjugated to a detectable compound; instead, a second antibody (which recognizes the antibody of interest) conjugated to a detectable compound may be added to the well. Further, instead of coating the well with the antigen, the antibody may be coated to the well. In this case, a second antibody conjugated to a detectable compound may be added following the addition of the antigen of interest to the coated well. One of skill in the art would be knowledgeable as to the parameters that can be modified to increase the signal detected as well as other variations of ELISAs known in the art. For further discussion regarding ELISAs see, e.g., Ausubel et al, eds, 1994, Current Protocols in Molecular Biology, Vol. 1, John Wiley & Sons, Inc., New York at 11.2.1.

The binding affinity of an antibody to an antigen and the off-rate of an antibody-antigen interaction can be determined by competitive binding assays. One example of a competitive binding assay is a radioimmunoassay comprising the incubation of labeled antigen (e.g., ³H or ¹²⁵I) with the antibody of interest in the presence of increasing amounts of unlabeled antigen, and the detection of the antibody bound to the labeled antigen. The affinity of the antibody of the present invention or a fragment thereof for the antigen and the binding off-rates can be determined from the saturation data by scatchard analysis. Competition with a second antibody can also be determined using radioimmunoassays. In this case, the antigen is incubated with an antibody of the present invention or a fragment thereof conjugated to a labeled compound (e.g., ³H or ¹²⁵I) in the presence of increasing amounts of an unlabeled second antibody.

In one embodiment, BIAcore kinetic analysis can be used to determine the binding on and off rates of antibodies to an antigen. BIAcore kinetic analysis comprises analyzing the binding and dissociation of an antigen from chips with immobilized antibodies on their surface (see the Example section infra).

Antibodies, fusion proteins, and conjugated molecules can also be assayed for their ability to inhibit the binding of an antigen to its host cell receptor using techniques known to those of skill in the art. For example, cells expressing the receptor for a viral antigen can be contacted with virus in the presence or absence of an antibody and the ability of the antibody to inhibit viral antigen's binding can measured by, for example, flow cytometry or a scintillation counter. The antigen or the antibody can be labeled with a detectable compound such as a radioactive label (e.g. sup.32P, .sup.35S, and 1251) or a fluorescent label (e.g., fluorescein isothiocyanate, rhodamine, phycoerythrin, phycocyanin, allophycocyanin, o-phthaldehyde and fluorescamine) to enable detection of an interaction between the antigen and its host cell receptor. Alternatively, the ability of antibodies to inhibit an antigen from binding to its receptor can be determined in cell-free assays. For example, virus or a viral antigen (e.g., RSV F glycoprotein) can be contacted in a cell-free assay with an antibody and the ability of the antibody to inhibit the virus or the viral antigen from binding to its host cell receptor can be determined. The antibody may be immobilized on a solid support and the antigen can be labeled with a detectable compound. Alternatively, the antigen can immobilized on a solid support and the antibody can be labeled with a detectable compound. The antigen may be partially or completely purified (e.g., partially or completely free of other polypeptides) or part of a cell lysate. Further, the antigen may be a fusion protein comprising the viral antigen and a domain such as glutathionine-S-transferase, Alternatively, an antigen can be biotinylated using techniques well known to those of skill in the art (e.g, biotinylation kit, Pierce Chemicals; Rockford, III.).

Antibodies, fusion proteins, and conjugated molecules can also be assayed for their ability to inhibit or downregulate viral or bacterial replication using techniques known to those of skill in the art. For example, viral replication can be assayed by a plaque assay such as described, e.g., by Johnson et al., Journal of Infectious Diseases, 176:1215-1224, 1997. The antibodies, fusion proteins, and conjugated molecules can also be assayed for their ability to inhibit or downregulate the expression of viral or bacterial polypeptides. Techniques known to those of skill in the art, including, but not limited to, Western blot analysis, Northern blot analysis, and RT-PCR, can be used to measure the expression of viral or bacterial polypeptides. Further, the antibodies, fusion proteins, and conjugated molecules of the invention of the invention can be assayed for their ability to prevent the formation of syncytia.

Antibodies, fusion proteins, conjugated molecules, and compositions can be tested in vitro, and then in vivo for the desired therapeutic or prophylactic activity, prior to use in humans. For example, in vitro assays which can be used to determine whether administration of a specific antibody, a specific fusion protein, a specific conjugated molecule, or a composition of the present invention is indicated, include in vitro cell culture assays in which a subject tissue sample is grown in culture, and exposed to or otherwise administered an antibody, a fusion protein, conjugated molecule, or composition, and the effect of such an antibody, a fusion protein, conjugated molecule, or a composition upon the tissue sample is observed. In various specific embodiments, in vitro assays can be carried out with representative cells of cell types involved in a disease or disorder, to determine if an antibody, a fusion protein, conjugated molecule, or composition of the present invention has a desired effect upon such cell types. Antibodies, fusion proteins, conjugated molecules, or compositions can also be tested in in vitro assays and animal model systems prior to administration to humans.

Antibodies, fusion proteins, conjugated molecules, or compositions for use in therapy can be tested for their toxicity in suitable animal model systems, including but not limited to rats, mice, cows, monkeys, and rabbits. For in vivo testing for the toxicity of an antibody, a fusion protein, a conjugated molecule, or a composition, any animal model system known in the art may be used.

Efficacy in treating or preventing viral infection may be demonstrated by detecting the ability of an antibody, a fusion protein, a conjugated molecule, or a composition to inhibit the replication of the virus, to inhibit transmission or prevent the virus from establishing itself in its host, or to prevent, ameliorate or alleviate one or more symptoms associated with viral infection. The treatment is considered therapeutic if there is, for example, a reduction is viral load, amelioration of one or more symptoms or a decrease in mortality and/or morbidity following administration of an antibody, a fusion protein, a conjugated molecule, or a composition. Antibodies, fusion proteins, conjugated molecules, or compositions can also be tested for their ability to inhibit viral replication or reduce viral load in in vitro and in vivo assays.

Efficacy in treating or preventing bacterial infection may be demonstrated by detecting the ability of an antibody, a fusion protein or a composition of the invention to inhibit the bacterial replication, or to prevent, ameliorate or alleviate one or more symptoms associated with bacterial infection. The treatment is considered therapeutic if there is, for example, a reduction is bacterial numbers, amelioration of one or more symptoms or a decrease in mortality and/or morbidity following administration of an antibody, a fusion protein or a composition of the invention.

Efficacy in treating cancer may be demonstrated by detecting the ability of an antibody, a fusion protein, a conjugated molecule, or a composition to inhibit or reduce the growth or metastasis of cancerous cells or to ameliorate or alleviate one or more symptoms associated with cancer. The treatment is considered therapeutic if there is, for example, a reduction in the growth or metastasis of cancerous cells, amelioration of one or more symptoms associated with cancer, or a decrease in mortality and/or morbidity following administration of an antibody, a fusion protein, a conjugated molecule, or a composition. Antibodies, fusion proteins or compositions can be tested for their ability to reduce tumor formation in in vitro, ex vivo, and in vivo assays.

Efficacy in treating inflammatory disorders may be demonstrated by detecting the ability of an antibody, a fusion protein, a conjugated molecule, or a composition to reduce or inhibit the inflammation in an animal or to ameliorate or alleviate one or more symptoms associated with an inflammatory disorder. The treatment is considered therapeutic if there is, for example, a reduction is in inflammation or amelioration of one or more symptoms following administration of an antibody, a fusion proteins, a conjugated molecule, or a composition.

Antibodies, fusion proteins, conjugated molecules, or compositions can be tested in vitro and in vivo for the ability to induce the expression of cytokines (e.g., IFN-α, IFN-β, IFN-γ, IL-2, IL-3, IL-4, IL-5, IL-6, IL-10, IL-12, and IL-15) and activation markers (e.g., CD28, ICOS, and SLAM). Techniques known to those of skill in the art can be used to measure the level of expression of cytokines and activation markers. For example, the level of expression of cytokines can be measured by analyzing the level of RNA of cytokines by, for example, RT-PCR and Northern blot analysis, and by analyzing the level of cytokines by, for example, immunoprecipitation followed by Western blot analysis or ELISA.

Antibodies, fusion proteins, conjugated molecules, or compositions can be tested in vitro and in vivo for their ability to modulate the biological activity of immune cells, e.g., human immune cells (e.g., T-cells, B-cells, and Natural Killer cells). The ability of an antibody, a fusion protein, a conjugated molecule, or a composition to modulate the biological activity of immune cells can be assessed by detecting the expression of antigens, detecting the proliferation of immune cells, detecting the activation of signaling molecules, detecting the effector function of immune cells, or detecting the differentiation of immune cells. Techniques known to those of skill in the art can be used for measuring these activities. For example, cellular proliferation can be assayed by ³H-thymidine incorporation assays and trypan blue cell counts. Antigen expression can be assayed, for example, by immunoassays including, but are not limited to, competitive and non-competitive assay systems using techniques such as Western blots, immunohistochemistry, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays and FACS analysis. The activation of signaling molecules can be assayed, for example, by kinase assays and electrophoretic shift assays (EMSAs).

Antibodies, fusion proteins, conjugated molecules, or compositions can also be tested for their ability to increase the survival period of animals, such as mammals, e.g., humans, suffering from a disease, disorder, or infection by at least 25%, preferably at least 50%, at least 60%, at least 75%, at least 85%, at least 95%, or at least 99%. Further, antibodies, fusion proteins, conjugated molecules, or compositions can be tested for their ability reduce the hospitalization period of animals, such as mammals, e.g., humans, suffering from a disease, disorder, or infection by at least 60%, preferably at least 75%, at least 85%, at least 95%, or at least 99%. Techniques known to those of skill in the art can be used to analyze the function of the antibodies or compositions of the invention in vivo.

### 5.10 DIAGNOSTIC USES OF ANTIBODIES AND FUSION PROTEINS

Labeled antibodies, fusion proteins, and conjugated molecules can be used for diagnostic purposes to detect, diagnose, or monitor diseases, disorders or infections. The invention provides for the detection or diagnosis of a disease, disorder or infection, comprising: (a) assaying the expression of an antigen in cells or a tissue sample of a subject using one or more antibodies that immunospecifically bind to the antigen; and (b) comparing the level of the antigen with a control level, e.g, levels in normal tissue samples, whereby an increase in the assayed level of antigen compared to the control level of the antigen is indicative of the disease, disorder or infection. The invention also provides for the detection or diagnosis of a disease, disorder or infection, comprising (a) assaying the expression of an antigen in cells or a tissue sample of a subject using one or fusion proteins or conjugated molecules of the invention that bind to the antigen; and (b) comparing the level of the antigen with a control level, e.g., levels in normal tissue samples, whereby an increase of antigen compared to the control level of the antigen is indicative of the disease, disorder or infection. Accordingly, a fusion protein or conjugated molecule comprises a bioactive molecule such as a ligand, cytokine or growth factor and the hinge-Fc region or fragments thereof, wherein the fusion protein or conjugated molecule is capable of binding to an antigen being detected.

Antibodies can be used to assay antigen levels in a biological sample using classical immunohistological methods as described herein or as known to those of skill in the art (e.g., see Jalkanen et al., J. Cell. Biol., 101:976-985, 1985; Jalkanen et al., J. Cell. Biol., 105:3087-3096, 1987). Other antibody-based methods useful for detecting protein gene expression include immunoassays, such as the enzyme linked immunosorbent assay (ELISA) and the radioimmunoassay (RIA). Suitable antibody assay labels are known in the art and include enzyme labels, such as, alkaline phosphatase, glucose oxidase; radioisotopes, such as iodine (¹²⁵I, ¹³¹I), carbon (¹⁴C), sulfur (³⁵S), tritium (³H), indium (¹²¹In), and technetium (^{99m}Tc); luminescent labels, such as luminol; and fluorescent labels, such as fluorescein and rhodamine.

Fusion proteins can be used to assay antigen levels in a biological sample using, for example, SDS-PAGE and immunoassays known to those of skill in the art.

One aspect of the invention is the detection and diagnosis of a disease, disorder, or infection in a human. In one embodiment, diagnosis comprises: a) administering (for example, parenterally, subcutaneously, or intraperitoneally) to a subject an effective amount of a labeled antibody that immunospecifically binds to an antigen; b) waiting for a time interval following the administration for permitting the labeled antibody to preferentially concentrate at sites in the subject where the antigen is expressed (and for unbound labeled molecule to be cleared to background level); c) determining background level; and d) detecting the labeled antibody in the subject, such that detection of labeled antibody above the background level indicates that the subject has the disease, disorder, or infection. In accordance with this embodiment, the antibody is labeled with an imaging moiety which is detectable using an imaging system known to one of skill in the art. Background level can be determined by various methods including, comparing the amount of labeled molecule detected to a standard value previously determined for a particular system.

In another embodiment, diagnosis comprises: a) administering (for example, parenterally, subcutaneously, or intraperitoneally) to a subject an effective amount of a labeled fusion protein or conjugated molecule that binds to an antigen or some other molecule; b) waiting for a time interval following the administration for permitting the labeled fusion protein or conjugated molecule to preferentially concentrate at sites in the subject where the antigen or other molecule is expressed (and for unbound labeled molecule to be cleared to background level); c) determining background level; and d) detecting the labeled fusion protein or conjugated molecule in the subject, such that detection of labeled fusion protein above the background level indicates that the subject has the disease, disorder, or infection. In accordance with this embodiment, the fusion protein or conjugated molecule comprises a bioactive molecule such as a ligand, cytokine or growth factor and a hinge-Fc region or a fragment thereof, wherein said fusion protein or conjugated molecule is labeled with an imaging moiety and is capable of binding to the antigen being detected.

It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of ^{99m}Tc. The labeled antibody will then preferentially accumulate at the location of cells which contain the specific protein. In vivo tumor imaging is described in S. W. Burchiel et al., "Immunopharmacokinetics of Radiolabeled Antibodies and Their Fragments," Chapter 13 in Tumor Imaging: The Radiochemical Detection of Cancer, S. W. Burchiel and B. A. Rhodes, eds., Masson Publishing Inc. (1982).

Depending on several variables, including the type of label used and the mode of administration, the time interval following the administration for permitting the labeled molecule to preferentially concentrate at sites in the subject and for unbound labeled molecule to be cleared to background level is 6 to 48 hours or 6 to 24 hours or 6 to 12 hours. In another embodiment the time interval following administration is 5 to 20 days or 5 to 10 days.

In one embodiment, monitoring of a disease, disorder or infection is carried out by repeating the method for diagnosing the disease, disorder or infection, for example, one month after initial diagnosis, six months after initial diagnosis, one year after initial diagnosis, etc.

Presence of the labeled molecule can be detected in the subject using methods known in the art for in vivo scanning. These methods depend upon the type of label used. Skilled artisans will be able to determine the appropriate method for detecting a particular label. Methods and devices that may be used in the diagnostic methods of the invention include, but are not limited to, computed tomography (CT), whole body scan such as position emission tomography (PET), magnetic resonance imaging (MRI), and sonography.

In one embodiment, the molecule is labeled with a radioisotope and is detected in the patient using a radiation responsive surgical instrument (Thurston et al., U.S. Pat. No. 5,441,050). In another embodiment, the molecule is labeled with a fluorescent compound and is detected in the patient using a fluorescence responsive scanning instrument. In another embodiment, the molecule is labeled with a positron emitting metal and is detected in the patient using positron emission-tomography. In yet another embodiment, the molecule is labeled with a paramagnetic label and is detected in a patient using magnetic resonance imaging (MRI).

### 5.11. KITS

The invention also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

The present invention provides kits that can be used in the above methods. In one embodiment, a kit comprises an antibody, fusion protein, or conjugated molecule, preferably in a purified form, in one or more containers. In a specific embodiment, the kits of the present invention may contain a substantially isolated antigen as a control. The kits of the present invention may further comprise a control antibody, fusion protein, or conjugated molecule which does not react with the antigen included in the kit. In another specific embodiment, the kits of the present invention may contain a means for detecting the binding of an antibody, fusion protein, or conjugated molecule, to an antigen (e.g., the antibody, fusion protein, or conjugated molecule, may be conjugated to a detectable substrate such as a fluorescent compound, an enzymatic substrate, a radioactive compound or a luminescent compound, or a second antibody which recognizes the first antibody may be conjugated to a detectable substrate). In specific embodiments, the kit may include a recombinantly produced or chemically synthesized antigen. The antigen provided in the kit may also be attached to a solid support. In a more specific embodiment the detecting means of the above-described kit may include a solid support to which antigen is attached. Such a kit may also include a non-attached reporter-labeled anti-human antibody. In this embodiment, binding of the antibody to the antigen can be detected by binding of the said reporter-labeled antibody.

### 5.12 IN VITRO AND IN VIVO ASSAYS FOR REDUCED HALF-LIFE OF MODIFIED IGG HINGE-FC FRAGMENTS

The binding ability of modified IgGs and molecules comprising a modified IgG constant domain that binds FcRn can be characterized by various in vitro assays. PCT publication WO 97/34631 by Ward discloses various methods in detail and is incorporated herein in its entirety by reference.

For example, in order to compare the ability of the modified IgG or fragments thereof to bind to FcRn with that of the wild type IgG, the modified IgG or fragments thereof and the wild type IgG can be radio-labeled and reacted with FcRn-expressing cells in vitro. The radioactivity of the cell-bound fractions can be then counted and compared. The cells expressing FcRn to be used for this assay may be endothelial cell lines including mouse pulmonary capillary endothelial cells (B10, D2.PCE) derived from lungs of B10.DBA/2 mice and SV40 transformed endothelial cells (SVEC) (Kim et al., J. Immunol., 40:457-465, 1994) derived from C3H/HeJ mice. However, other types of cells, such as intestinal brush borders isolated from 10- to 14-day old suckling mice, which express sufficient number of FcRn can be also used. Alternatively, mammalian cells which express recombinant FcRn of a species of choice can be also utilized. After counting the radioactivity of the bound fraction of modified IgG or that of wild type, the bound molecules can be then extracted with the detergent, and the percent release per unit number of cells can be calculated and compared.

Affinity of modified IgGs for FcRn can be measured by surface plasmon resonance (SPR) measurement using, for example, a BIAcore 2000 (BIAcore Inc.) as described previously (Popov et al., Mol. Immunol., 33:493-502, 1996; Karlsson et al., J. Immunol. Methods, 145:229-240, 1991, both of which are incorporated by reference in their entireties). In this method, FcRn molecules are coupled to a BIAcore sensor chip (e.g., CM5 chip by Pharmacia) and the binding of modified IgG to the immobilized FcRn is measured at a certain flow rate to obtain sensorgrams using BIA evaluation 2.1 software, based on which on- and off-rates of the modified IgG, constant domains, or fragments thereof, to FcRn can be calculated.

Relative affinities of modified IgGs or fragments thereof, and the wild type IgG for FcRn can be also measured by a simple competition binding assay. Unlabeled modified IgG or wild type IgG is added in different amounts to the wells of a 96-well plate in which FcRn is immobilize. A constant amount of radio-labeled wild type IgG is then added to each well. Percent radioactivity of the bound fraction is plotted against the amount of unlabeled modified IgG or wild type IgG and the relative affinity of the modified hinge-Fc can be calculated from the slope of the curve.

Furthermore, affinities of modified IgGs or fragments thereof, and the wild type IgG for FcRn can be also measured by a saturation study and the Scatchard analysis.

Transfer of modified IgG or fragments thereof across the cell by FcRn can be measured by in vitro transfer assay using radiolabeled IgG or fragments thereof and FcRn-expressing cells and comparing the radioactivity of the one side of the cell monolayer with that of the other side. Alternatively, such transfer can be measured in vivo by feeding 10- to 14-day old suckling mice with radiolabeled, modified IgG and periodically counting the radioactivity in blood samples which indicates the transfer of the IgG through the intestine to the circulation (or any other target tissue, e.g., the lungs). To test the dose-dependent inhibition of the IgG transfer through the gut, a mixture of radiolabeled and unlabeled IgG at certain ratio can be given to the mice and the radioactivity of the plasma can be periodically measured (Kim et al., Eur. J. Immunol., 24:2429-2434,1994).

The half-life of modified IgG or fragments thereof can be measured by pharmacokinetic studies according to the method described by Kim et al. (Eur. J. of Immuno. 24:542, 1994), which is incorporated by reference herein in its entirety. According to this method, radiolabeled modified IgG or fragments thereof is injected intravenously into mice and its plasma concentration is periodically measured as a function of time, for example, at 3 minutes to 72 hours after the injection. The clearance curve thus obtained should be biphasic, that is, α-phase and β-phase. For the determination of the in vivo half-life of the modified IgGs or fragments thereof, the clearance rate in β-phase is calculated and compared with that of the wild type IgG.

All publications, patents and patent applications mentioned in this specification are herein incorporated by reference into the specification to the same extent as if each individual publication, patent or patent application was specifically and individually indicated to be incorporated herein by reference.

### 6. EXAMPLES

The invention is now described with reference to the following examples. These examples are provided for the purpose of illustration only and the invention should in no way be construed as being limited to these examples but rather should be construed to encompass any and all variations which become evident as a result of the teachings provided herein.

### EXAMPLE 1: Construction, production, and purification of IgG1 variants

The variable regions of antibody MEDI-524 (a.k.a. NUMAX™ or motavizumab) were individually cloned into mammalian expression vectors encoding human cytomegalovirus major immediate early (hCMVie) enhancer, promoter and 5'-untranslated region (Boshart, M. et al., (1985) A very strong enhancer is located upstream of an immediate early gene of human cytomegalovirus. Cell 41, 521-530). In this system, a human γ1 chain is secreted along with a human κ chain (Johnson, S. et al., (1997) Development of a humanized monoclonal antibody (MEDI-493) with potent in vitro and in vivo activity against respiratory syncytial virus. J. Infect. Dis. 176, 1215-1224).

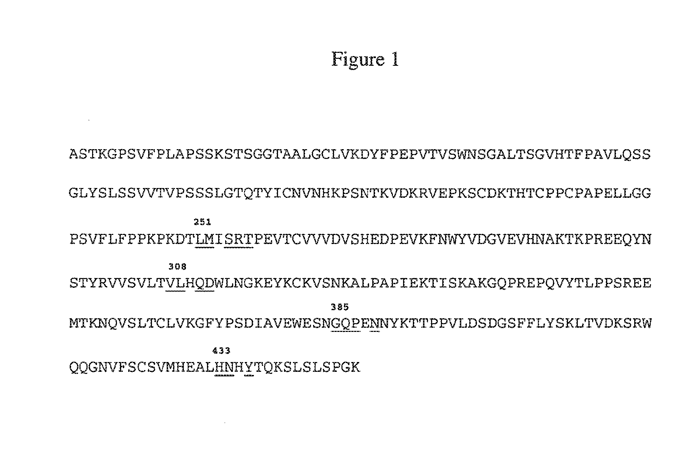

Mutations were introduced in expression vectors which would result in amino acid substitutions at positions 251, 252, 254, 255, 256, 308, 309, 311. 312, 385, 386, 387, 389,433,434, and 436 of MEDI-524. See Figure 1. The mutations were introduced using an NNS degenerate codon or a tryptophan codon (TGG). Mutagenesis was performed with the Quick Change II Site-Directed Mutagenesis Kit (Stratagene). Table 1 provides the nucleotide sequences of oligonucleotides utilized to introduce mutations in the expression vector:

**TABLE 1: Oligonucleotides**

| Oligonucleotide name | Oligonucleotide sequence (5' - 3') | SEQ ID NO. |
|---|---|---|
| G385/NNS primer 1 | CCG TGG AGT GGG AGA GCA ATN NSC AGC CGG AGA ACA ACT ACA AG | 1 |
| G385/NNS primer2 | CTT GTA GTT GTT CTC CGG CTG SNN ATT GCT CTC CCA CTC CAC GG | 2 |
| G385/TGG primer 1 | CCG TGG AGT GGG AGA GCA ATT GGC AGC CGG AGA ACA ACT ACA AG | 3 |
| G385/TGG primer 2 | CTT GTA GTT GTT CTC CGG CTG CCA ATT GCT CTC CCA CTC CAC GG | 4 |
| Q386/NNS primer 1 | GGA GTG GGA GAG CAA TGG GNN SCC GGA GAA CAA CTA CAA GAC | 5 |
| Q386/NNS primer 2 | GTC TTG TAG TTG TTC TCC GGS NNC CCA TTG CTC TCC CAC TCC | 6 |
| Q386/TGG primer 1 | GGA GTG GGA GAG CAA TGG GTT GCC GGA GAA CAA CTA CAA GAC | 7 |
| Q386/TGG primer 2 | GTC TTG TAG TTG TTC TCC GGC AAC CCA TTG CTC TCC CAC TCC | 8 |
| P387/NNS primer 1 | GAG TGG GAG AGC AAT GGG CAG NNS GAG AAC AAC TAC AAG ACC ACG | 9 |
| P387/NNS primer 2 | CGT GGT CTT GTA GTT GTT CTC SNN CTG CCC ATT GCT CTC CCA CTC | 10 |
| P387/TGG primer 1 | GAG TGG GAG AGC AAT GGG CAG TGG GAG AAC AAC TAC AAG ACC ACG | 11 |
| P387/TGG primer 2 | CGT GGT CTT GTA GTT GTT CTC CCA CTG CCC ATT GCT CTC CCA CTC | 12 |
| N389/NNS primer 1 | GAG CAA TGG GCA GCC GGA GNN SAA CTA CAA GAC CAC GCC TC | 13 |
| N389/NNS primer2 | GAG GCG TGG TCT TGT AGT TSN NCT CCG GCT GCC CAT TGC TC | 14 |
| N389/TGG primer 1 | GAG CAA TGG GCA GCC GGA GTG GAA CTA CAA GAC CAC GCC TC | 15 |
| N389/TGG primer 2 | GAG GCG TGG TCT TGT AGT TCC ACT CCG GCT GCC CAT TGC TC | 16 |
| G385+Q386/NNS primer 1 | CCG TGG AGT GGG AGA GCA ATN NSN NSC CGG AGA ACA ACT ACA AGA C | 17 |
| G385+Q386/NNS primer 2 | GTC TTG TAG TTG TTC TCC GGS NNS NNA TTG CTC TCC CAC TCC ACG G | 18 |
| G385+Q386/TGG primer 1 | CCG TGG AGT GGG AGA GCA ATT GGT GGC CGG AGA ACA ACT ACA AGA C | 19 |
| G385+Q386/TGG primer 2 | GTC TTG TAG TTG TTC TCC GGC CAC CAA TTG CTC TCC CAC TCC ACG G | 20 |
| H433/NNS primer 1 | CGT GAT GCA TGA GGC TCT GNN SAA CCA CTA CAC GCA GAA G | 21 |
| H433/NNS primer 2 | CTT CTG CGT GTA GTG GTT SNN CAG AGC CTC ATG CAT CAC G | 22 |
| H433/TGG primer 1 | CGT GAT GCA TGA GGC TCT GTG GAA CCA CTA CAC GCA GAA G | 23 |
| H433/TGG primer 2 | CTT CTG CGT GTA GTG GTT CCA CAG AGC CTC ATG CAT CAC G | 24 |
| N434/NNS primer 1 | GAT GCA TGA GGC TCT GCA CNN SCA CTA CAC GCA GAA GAG CCT C | 25 |
| N434/NNS primer 2 | GAG GCT CTT CTG CGT GTA GTG SNN GTG CAG AGC CTC ATG CAT C | 26 |
| N434/TGG primer 1 | GAT GCA TGA GGC TCT GCA CTG GCA CTA CAC GCA GAA GAG CCT C | 27 |
| N434/TGG primer 2 | GAG GCT CTT CTG CGT GTA GTG CCA GTG CAG AGC CTC ATG CAT C | 28 |
| Y436/NNS primer 1 | GAG GCT CTG CAC AAC CAC NNS ACG CAG AAG | 29 |
| | AGC CTC TCC | |
| Y436/NNS primer 2 | GGA GAG GCT CTT CTG CGT SNN GTG GTT GTG CAG AGC CTC | 30 |
| Y436/TGG primer 1 | GAG GCT CTG CAC AAC CAC TGG ACG CAG AAG AGC CTC TCC | 31 |
| Y436/TGG primer 2 | GGA GAG GCT CTT CTG CGT CCA GTG GTT GTG CAG AGC CTC | 32 |
| L251/NNS primer 1 | CCC AAA ACC CAA GGA CAC CNS SAT GAT CTC CCG GAC CCC TG | 33 |
| L251/NNS primer 2 | CAG GGG TCC GGG AGA TCA TSS NGG TGT CCT TGG GTT TTG GG | 34 |
| L251/TGG primer 1 | CCC AAA ACC CAA GGA CAC CTG GAT GAT CTC CCG GAC CCC TG | 35 |
| L251/TGG primer 2 | CAG GGG TCC GGG AGA TCA TCC AGG TGT CCT TGG GTT TTG GG | 36 |
| M252/NNS primer 1 | CAA AAC CCA AGG ACA CCC TCN SSA TCT CCC GGA CCC CTG AG | 37 |
| M252/NNS primer 2 | CTC AGG GGT CCG GGA GAT SSN GAG GGT GTC CTT GGG TTT TG | 38 |
| M252/TGG primer 1 | CAA AAC CCA AGG ACA CCC TCT GGA TCT CCC GGA CCC CTG AG | 39 |
| M252/TGG primer 2 | CTC AGG GGT CCG GGA GAT CCA GAG GGT GTC CTT GGG TTT TG | 40 |
| S254/NNS primer 1 | CAA GGA CAC CCT CAT GAT CNS SCG GAC CCC TGA GGT CAC ATG | 41 |
| S254/NNS primer 2 | CAT GTG ACC TCA GGG GTC CGS SNG ATC ATG AGG GTG TCC TTG | 42 |
| S254/TGG primer 1 | CAA GGA CAC CCT CAT GAT CTG GCG GAC CCC TGA GGT CAC ATG | 43 |
| S254/TGG primer 2 | CAT GTG ACC TCA GGG GTC CGC CAG ATC ATG AGG GTG TCC TTG | 44 |
| R255/NNS primer 1 | GGA CAC CCT CAT GAT CTC CNN SAC CCC TGA GGT CAC ATG CG | 45 |
| R255/NNS primer 2 | CGC ATG TGA CCT CAG GGG TSN NGG AGA TCA TGA GGG TGT CC | 46 |
| R255/TGG primer 1 | GGA CAC CCT CAT GAT CTC CTG GAC CCC TGA GGT CAC ATG CG | 47 |
| R255/TGG primer 2 | CGC ATG TGA CCT CAG GGG TCC AGG AGA TCA TGA GGG TGT CC | 48 |
| T256/NNS primer 1 | CAC CCT CAT GAT CTC CCG GNN SCC TGA GGT CAC ATG CGT G | 49 |
| T256/NNS primer 2 | CAC GCA TGT GAC CTC AGG SNN CCG GGA GAT CAT GAG GGT G | 50 |
| T256/TGG primer 1 | CAC CCT CAT GAT CTC CCG GTG GCC TGA GGT CAC ATG CGT G | 51 |
| T256/TGG primer 2 | CAC GCA TGT GAC CTC AGG CCA CCG GGA GAT CAT GAG GGT G | 52 |
| N434+Y436/NNS primer 1 | GTG ATG CAT GAG GCT CTG CAC NNS CAC NNS ACG CAG AAG AGC CTC TCC CTG | 53 |
| N434+Y436/NNS primer 2 | CAG GGA GAG GCT CTT CTG CGT SNN GTG SNN GTG CAG AGC CTC ATG CAT CAC | 54 |
| N434+Y436/TGG primer 1 | GTG ATG CAT GAG GCT CTG CAC TGG CAC TGG ACG CAG AAG AGC CTC TCC CTG | 55 |
| N434+Y436/TGG primer 2 | CAG GGA GAG GCT CTT CTG CGT CCA GTG CCA GTG CAG AGC CTC ATG CAT CAC | 56 |
| V308/NNS primer 1 | GGT CAG CGT CCT CAC CNN SCT GCA CCA GGA CTG GC | 57 |
| V308/NNS primer 2 | GCC AGT CCT GGT GCA GSN NGG TGA GGA CGC TGA CC | 58 |
| V308/TGG primer 1 | GGT CAG CGT CCT CAC CTG GCT GCA CCA GGA CTG GC | 59 |
| V308/TGG primer 2 | GCC AGT CCT GGT GCA GCC AGG TGA GGA CGC TGA CC | 60 |
| L309/NNS primer 1 | GTC AGC GTC CTC ACC GTC NNS CAC CAG GAC TGG CTG AAT G | 61 |
| L309/NNS primer 2 | CAT TCA GCC AGT CCT GGT GSN NGA CGG TGA GGA CGC TGA C | 62 |
| L309/TGG primer 1 | GTC AGC GTC CTC ACC GTC TGG CAC CAG GAC TGG CTG AAT G | 63 |
| L309/TGG primer 2 | CAT TCA GCC AGT CCT GGT GCC AGA CGG TGA GGA CGC TGA C | 64 |
| Q311/NNS primer 1 | GTC CTC ACC GTC CTG CAC NNS GAC TGG CTG AAT GGC AAG | 65 |
| Q311/NNS primer 2 | CTT GCC ATT CAG CCA GTC SNN GTG CAG GAC GGT GAG GAC | 66 |
| Q311/TGG primer 1 | GTC CTC ACC GTC CTG CAC TGG GAC TGG CTG AAT GGC AAG | 67 |
| Q311/TGG primer 2 | CTT GCC ATT CAG CCA GTC CCA GTG CAG GAC GGT GAG GAC | 68 |
| D312/NNS primer 1 | CTC ACC GTC CTG CAC CAG NNS TGG CTG AAT GGC AAG GAG | 69 |
| D312/NNS primer 2 | CTC CTT GCC ATT CAG CCA SNN CTG GTG CAG GAC GGT GAG | 70 |
| D312/TGG primer 1 | CTC ACC GTC CTG CAC CAG TGG TGG CTG AAT GGC AAG GAG | 71 |
| D312/TGG primer 2 | CTC CTT GCC ATT CAG CCA CCA CTG GTG CAG GAC GGT GAG | 72 |
| L309+Q311/NNS primer 1 | GTG GTC AGC GTC CTC ACC GTC NNS CAC NNS GAC TGG CTG AAT GGC AAG GAG | 73 |
| L309+Q311/NNS primer 2 | CTC CTT GCC ATT CAG CCA GTC SNN GTG SNN GAC GGT GAG GAC GCT GAC CAC | 74 |
| L309+Q311/TGG primer 1 | GTG GTC AGC GTC CTC ACC GTC TGG CAC TGG GAC TGG CTG AAT GGC AAG GAG | 75 |
| L309+Q311/TGG primer 2 | CTC CTT GCC ATT CAG CCA GTC CCA GTG CCA GAC GGT GAG GAC GCT GAC CAC | 76 |

Clones were sequenced on an ABI3730 sequencer to verify that unwanted mutations were not introduced in the heavy chain. DNA constructs were purified using Qiagen Maxi purification kits and transiently co-transfected into human embryonic kidney 293 cells along with a vector expressing wild type MEDI-524 light chain and lipofectamine 2000 transfection reagent (Invitrogen). Supernatants expressing the desired IgGs were purified on 1 mL HiTrap Protein A HP columns (GE Healthcare). Concentrations of wild type and mutant IgGs were determined with the bicinchoninic acid method (BCA). SDS page analysis confirmed that the purity of each IgG was greater than 95%.

The sixteen amino acid residues selected for substitution: L251, M252, S254, R255, T256, V308, L309, Q311, D312, G385, Q386, P387, N389, H433, N434, and Y436, have been shown to be important for interacting with human FcRn (Dall'Acqua et al., (2002) Increasing the affinity of a human IgG1 for the neonatal Fc receptor: Biological consequences. J. Immunol. 169, 5171-5180). Of all the substituted MEDI-524 immunoglobulins generated (Table 2), cysteine or proline substitutions that could potentially alter the structure were discarded. Amino acid residue substitutions that were chosen were as chemically different from the corresponding wt residue as possible. A total of 35 MEDI-524 substituted immunoglobulins were generated. See Table 2.

**Table 2: Modified MEDI-524 Immunoglobulins**

| **Position(s)** | **wt codon** | **Codon change** | **Substitution** |
|---|---|---|---|
| 251 | CTC | TCG | L251S |
| 252 | ATG | ACG | M252T |
| 252 | ATG | TCC | M252S |
| 252 | ATG | TGG | M252W |
| 254 | TCC | TGG | S254W |
| 254 | TCC | CGG | S254R |
| 255 | CGG | GTC | R255V |
| 255 | CGG | ACC | R255T |
| 256 | ACC | TTG | T256L |
| 256 | ACC | CAC | T256H |
| 256 | ACC | TGG | T256W |
| 309 | CTG | TGG | L309W |
| 309 | CTG | AAC | L309N |
| 309 | CTG | TGG | L309W |
| 311 | CAG | GGG | Q311G |
| 312 | GAC | GGC | D312G |
| 312 | GAC | ATC | D312I |
| 385 | GGG | TTC | G385P |
| 385 | GGG | TGG | G385W |
| 386 | CAG | GTG | Q386V |
| 386 | CAG | TTG | Q386L |
| 387 | CCG | GGC | P387G |
| 387 | CCG | GTG | P387V |
| 389 | AAC | GGG | N389G |
| 389 | AAC | AGC | N389S |
| 433 | CAC | CTG | H433L |
| 434 | AAC | GGG | N434G |
| 434 | AAC | TTG | N434L |
| 436 | TAC | ACC | Y436T |
| 436 | TAC | ATC | Y436I |
| 309 + 311 | CTG + CAG | TTC + TTA | L309F+0311L |
| 309 + 311 | CTG + CAG | GAG + GTG | L309E+Q311V |
| 309 + 311 | CTG + CAG | AGG + TGG | L309R+Q311W |
| 385 + 386 | GGG + CAG | TCG + ATC | G385S+Q386I |
| 434 + 436 | AAC + TAC | TCG + AGC | N434S+Y436S |

### EXAMPLE 2: Expression and purification of human FcRn for surface plasmon resonance and ELISA binding analyses

The extracellular domain of human FcRn α-chain and β2-microblobulin were expressed in *Spodoptera frugiperda* cells (Sf9) as described (Dall'Acqua, W. et al., (2002) Increasing the affinity of a human IgG1 for the neonatal Fc receptor: Biological consequences. J. Immunol 169, 5171-5180). Supernatant was adjusted to pH 6.0 with hydrochloric acid and loaded onto a 10 mL IgG Sepharose 6 Fast Flow column (APBiotech). The column was washed with 200 mL of 50 mM MES (pH 5.8) before eluting FcRn with 0.1 M Tris-Cl, pH 8.0. Purified FcRn was dialyzed against PBS and stored at -80°C. Protein concentrations were calculated by the bicinchoninic acid method (BCA). Visualization of FcRn on an SDS-PAGE gel showed it was > 95% homogenous.

### EXAMPLE 3: Surface plasmon resonance measurements

Binding of wild type and all amino acid substituted MEDI-524 immunoglobulins to human FcRn at pH 6.0 was first analyzed by using a BIAcore 3000 instrument.

Human FcRn, expressed and purified as described in Example 2, was first buffer-exchanged against 50 mM PBS, pH 6.0, containing 0.05% Tween 20 and then immobilized at high density onto a CM5 sensor chip surface using standard amine coupling chemistry. Human FcRn was coupled at a concentration of 1 µM, in 10mM NaOAc, pH 5.0. Immobilization levels ranged from approximately 6300 to 7200 RUs.

The binding experiments were carried out using 200 nM of each IgG in 50 mM PBS, pH 6.0, 0.05% Tween 20 at a flow rate of 10 µL/min for 25 minutes at 25°C. Dissociation data were collected for 5 minutes before bound IgG was removed from the FcRn surface with a 1-minute pulse of 50 mM sodium carbonate, pH 9.1. MEDI-524 (wt) was interspersed among the mutants to monitor any change in the activity of the immobilized FcRn over the course the assay.

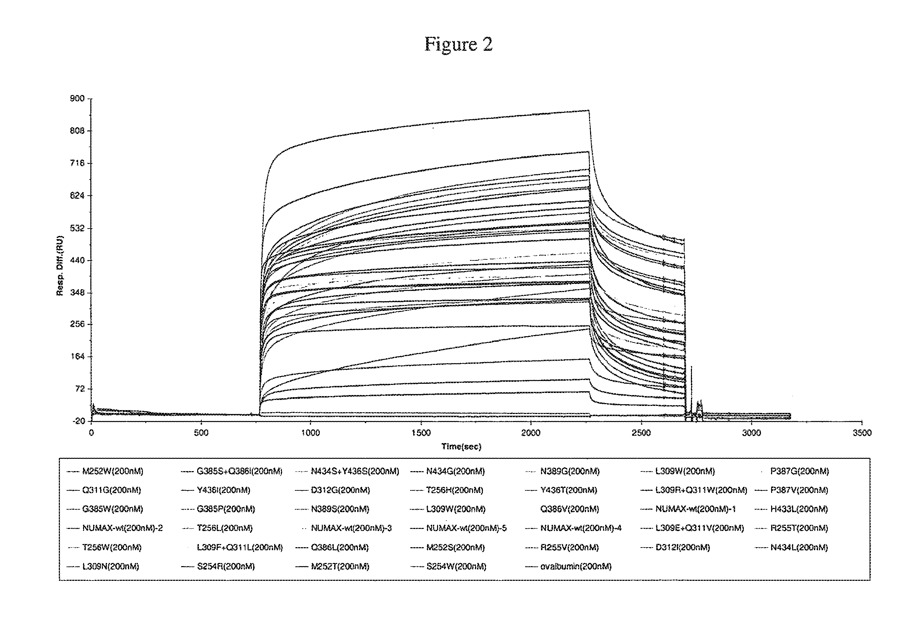

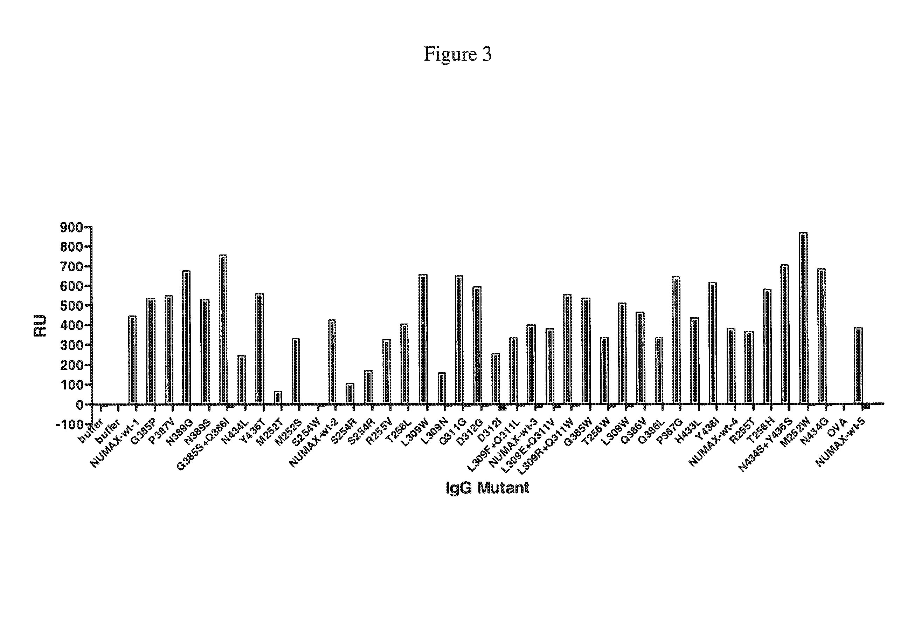

Select sensorgrams run at pH 6.0 are shown in Figure 2 and summarized in Figure 3. While many substituted MEDI-524 immunoglobulins could still bind to human FeRn, 9 substituted MEDI-524 immunoglobulins (S254R, S254W, M252T, L309N, N434L, R255V, D312I, Q386L, and L251S) demonstrated a significant reduction in binding to FcRn at pH 6.0.

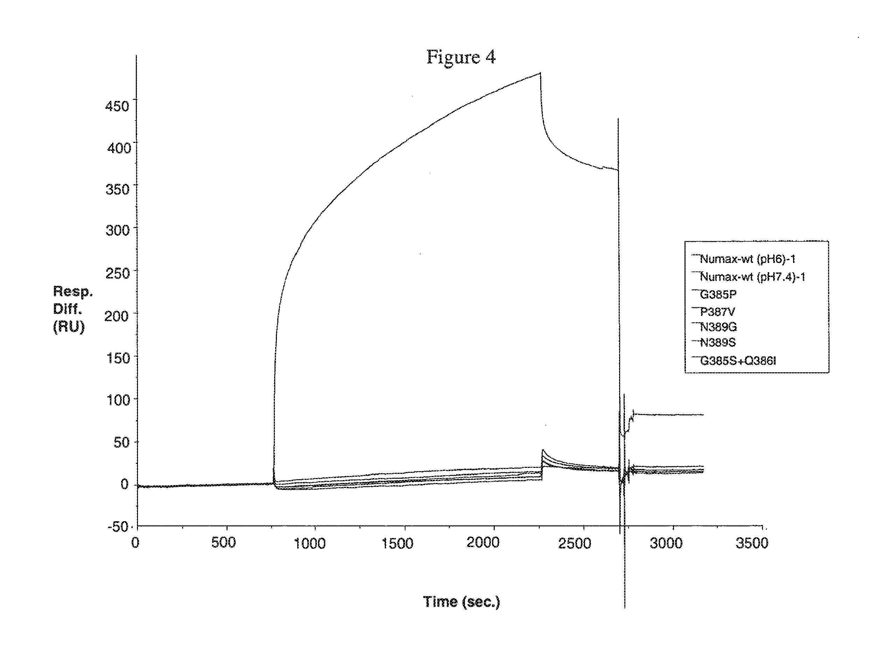

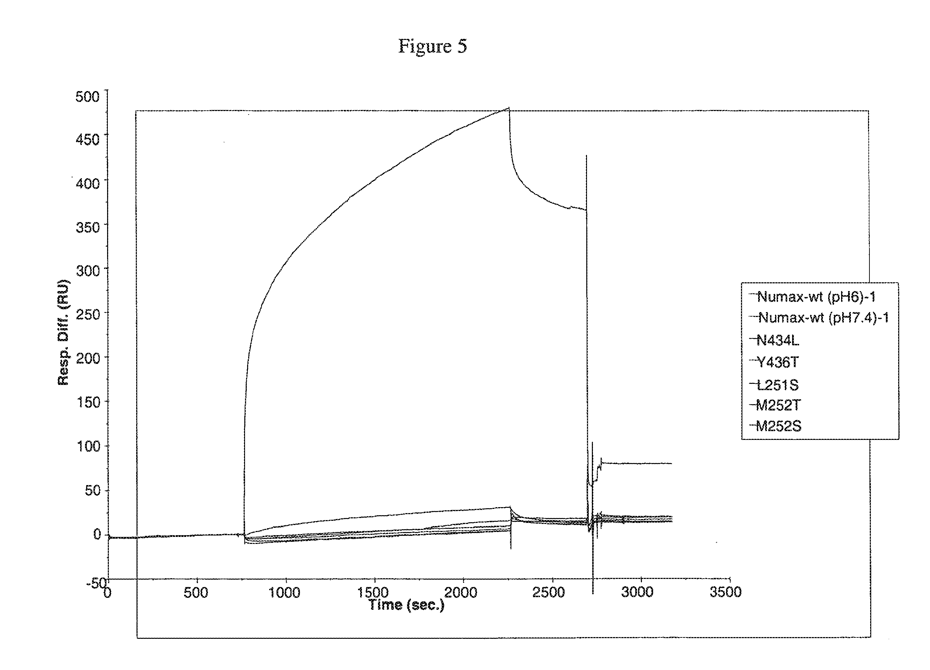

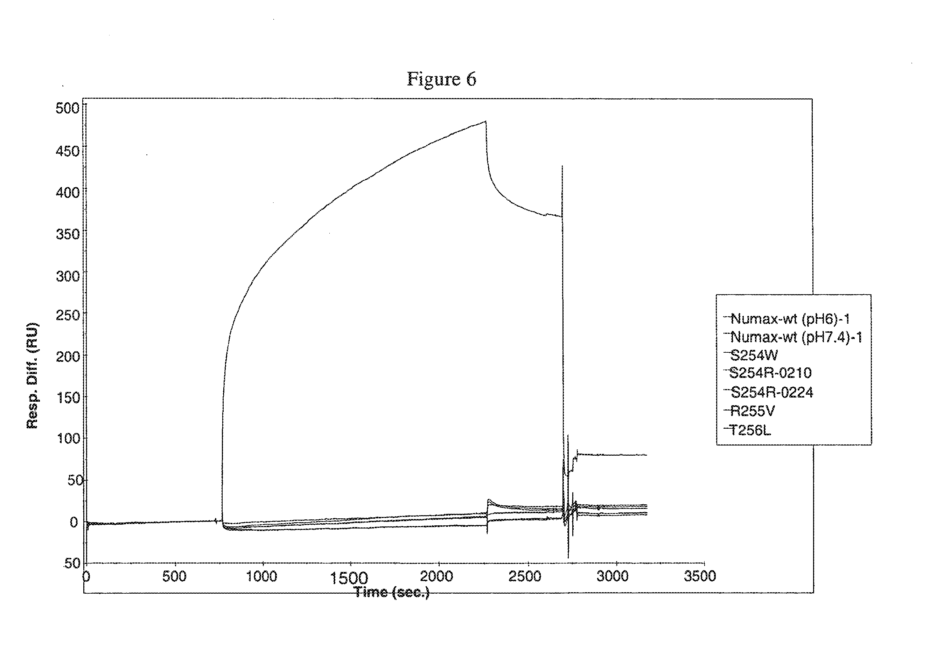

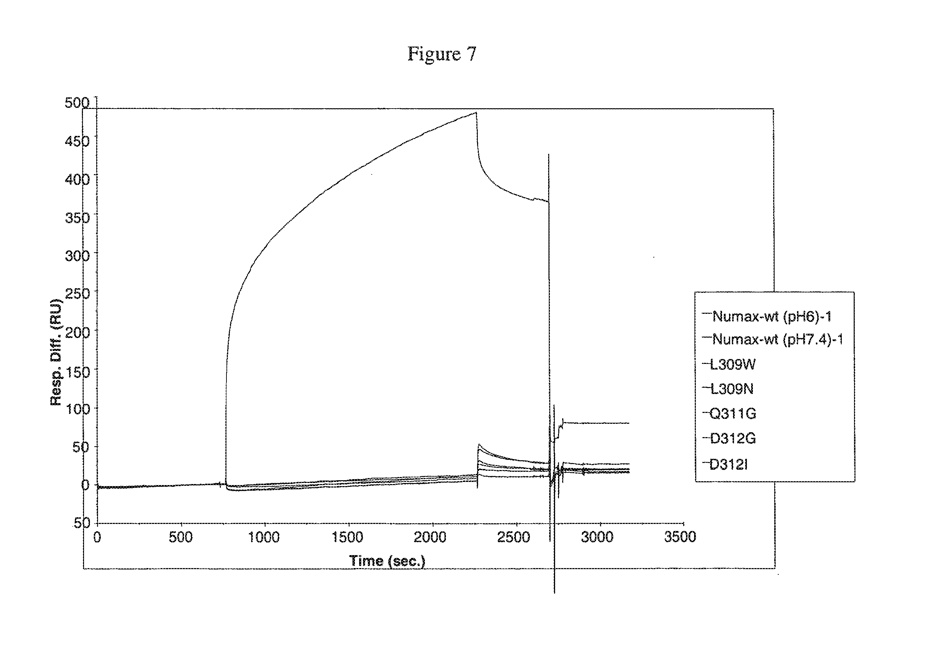

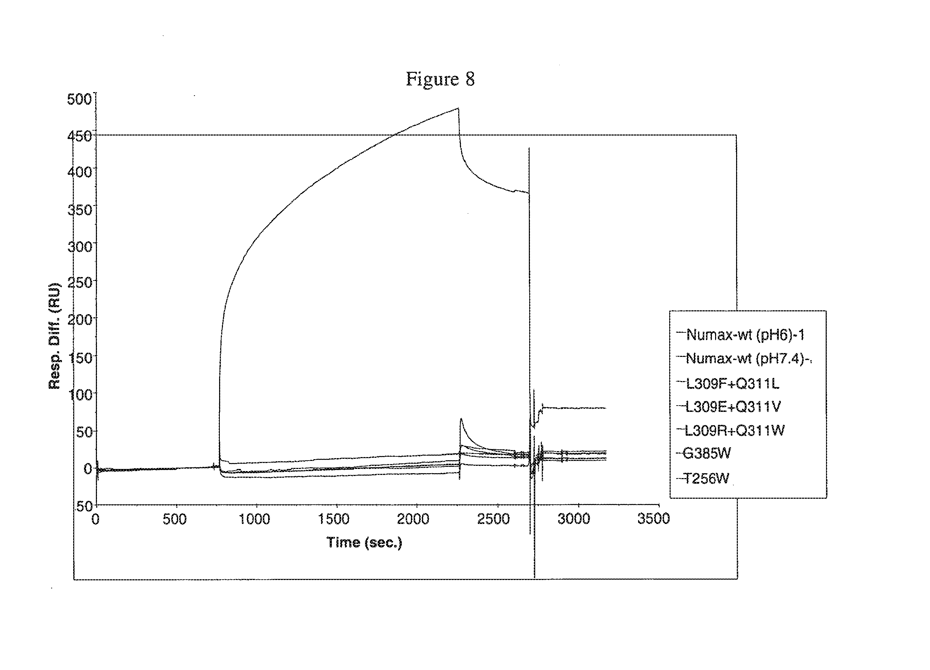

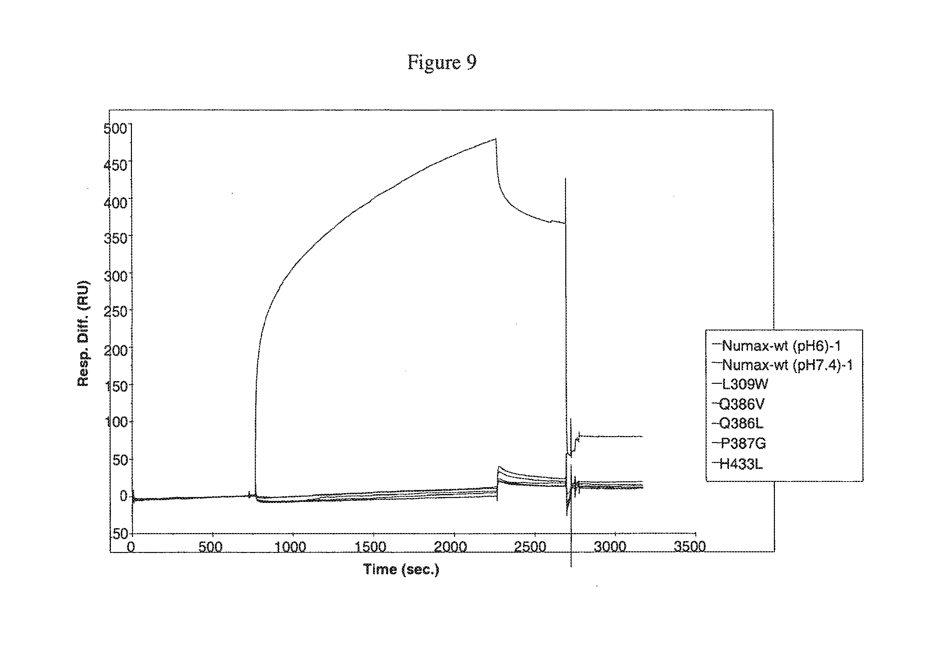

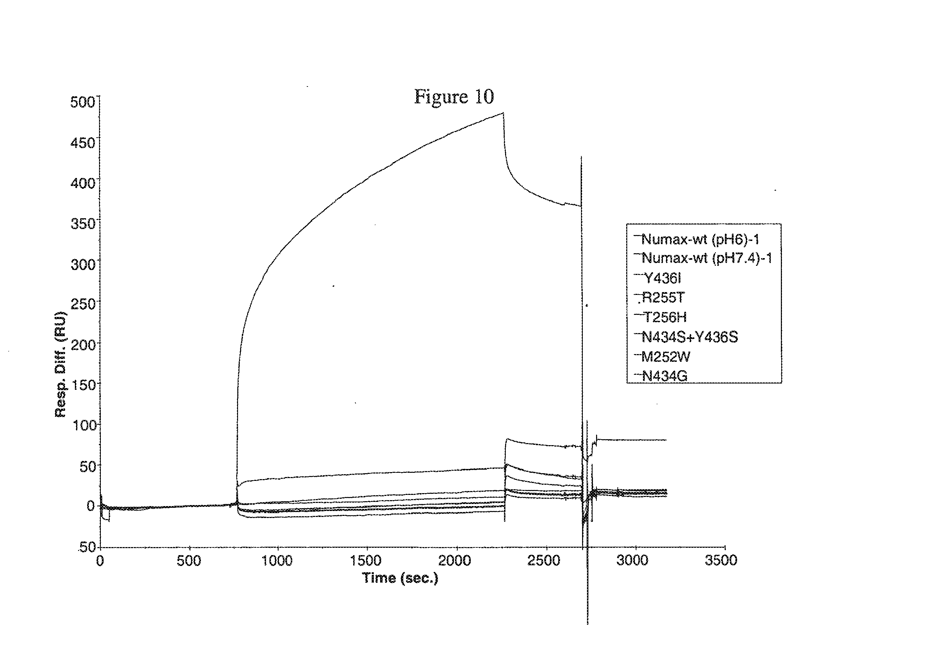

The assay was repeated in 50 mM PBS, pH 7.4, containing 0.05% Tween 20 in order to determine if any of these mutations had impacted the pH dependency of IgG binding to human FcRn. MEDI-524 (wt) binding at pH 6.0 and pH 7.4 was run as a control in the pH 7.4 assay. In all cases, IgGs were also flowed over an uncoated cell and the sensorgrams from these blank runs subtracted from those obtained with human FcRn-coupled chips. For all Fc mutants, no significant binding was observed at pH 7.4, as was observed for MEDI-524 wt (Figures 4-10).

### EXAMPLE 4: Further binding analysis of select MEDI-524 substituted immunoglobulins to human FcRn

MEDI-524 substituted immunoglobulins S254R, S254W, M252T, L309N, N434L, R255V, D312I, and L251S were further characterized by BIAcore using immobilized IgG and/or using an ELISA-based approach at both acidic and/or neutral pH.

**BIAcore.** The further BIAcore analysis was conducted in an effort to determine the equilibrium binding constants (K_{D}) for the interaction of these eight amino acid substituted MEDI-524 immunoglobulins with human FcRn. A ninth amino acid substituted MEDI-524 immunoglobulin (G385S+Q386I) was included in this Kd study.

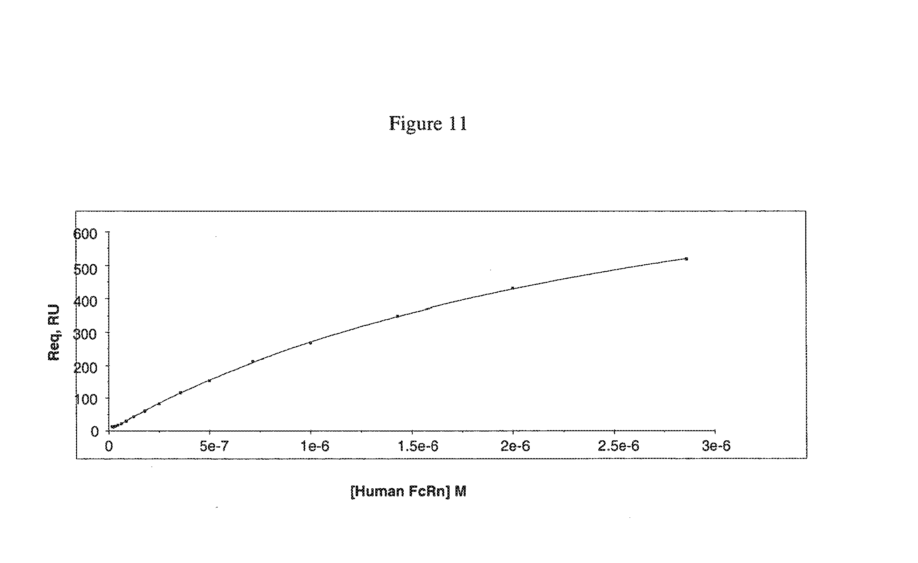

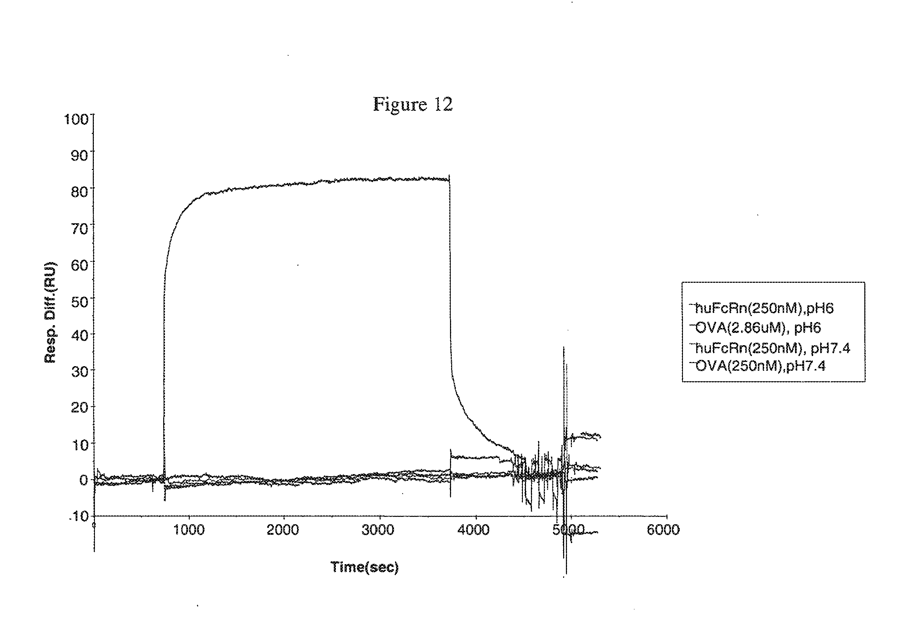

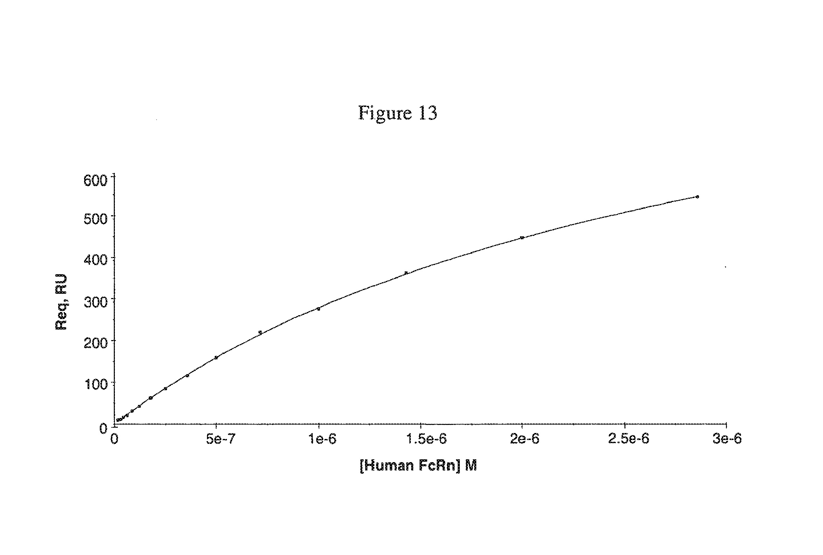

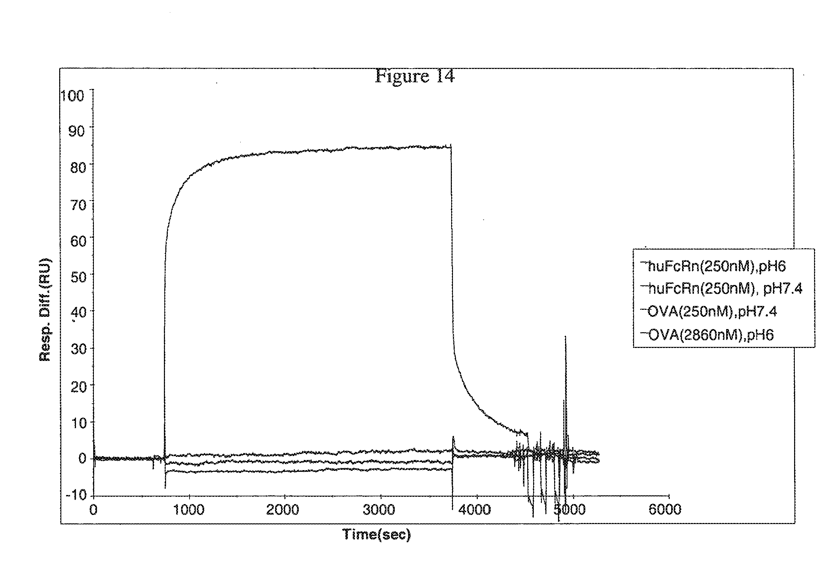

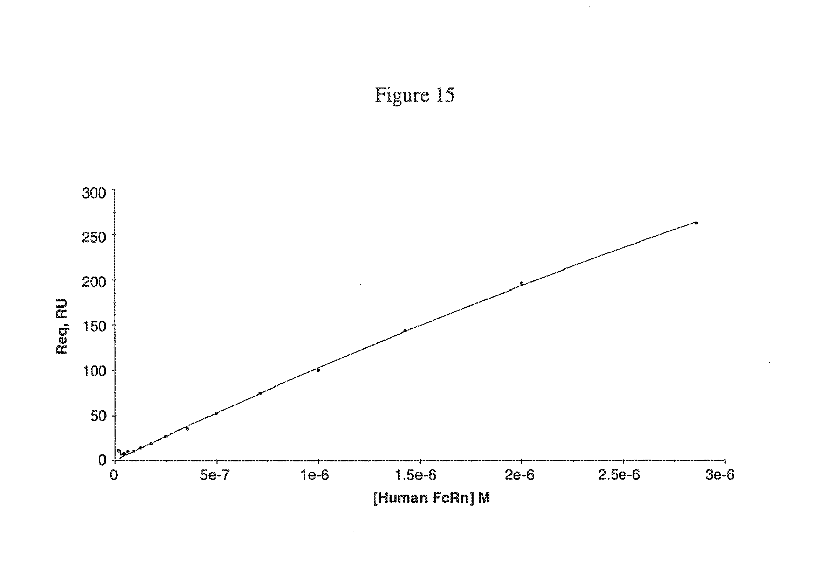

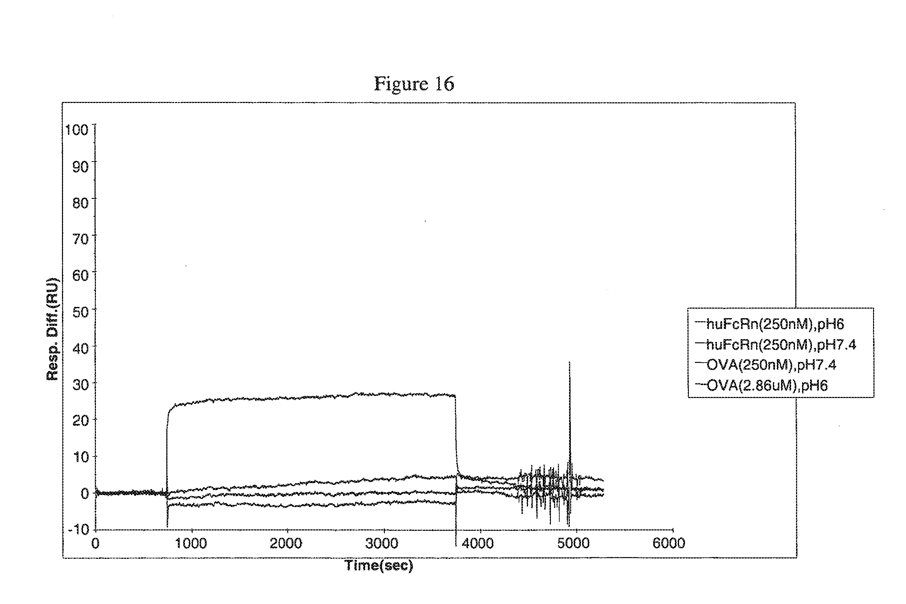

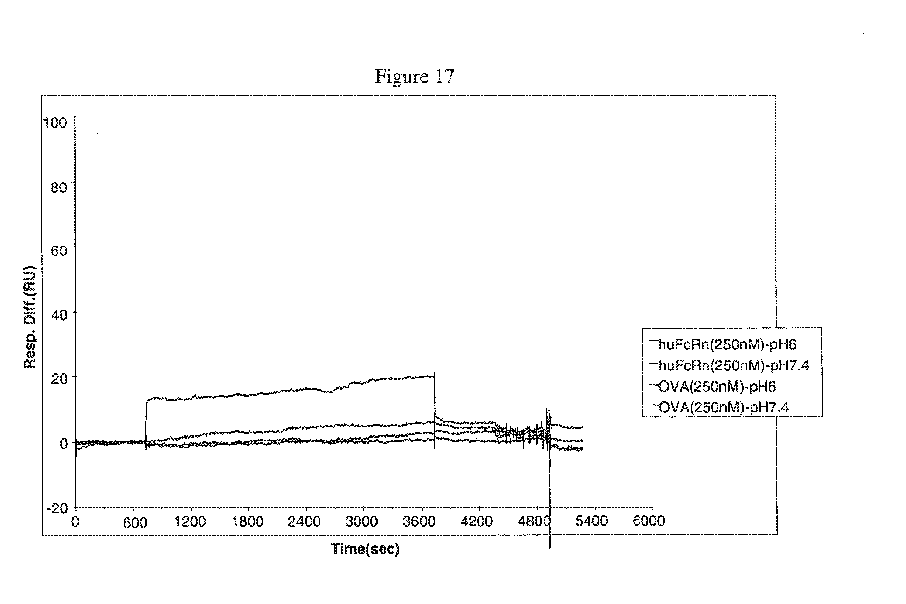

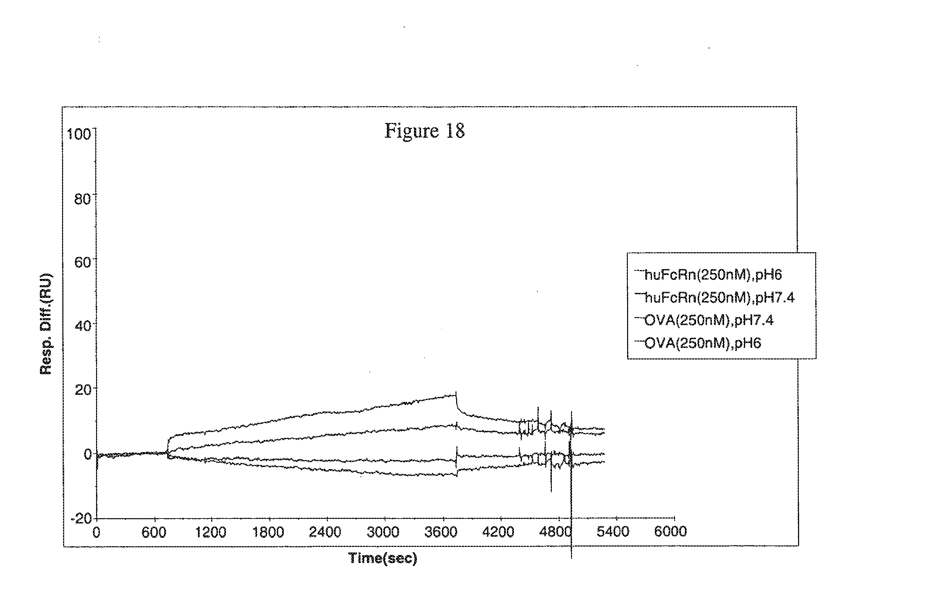

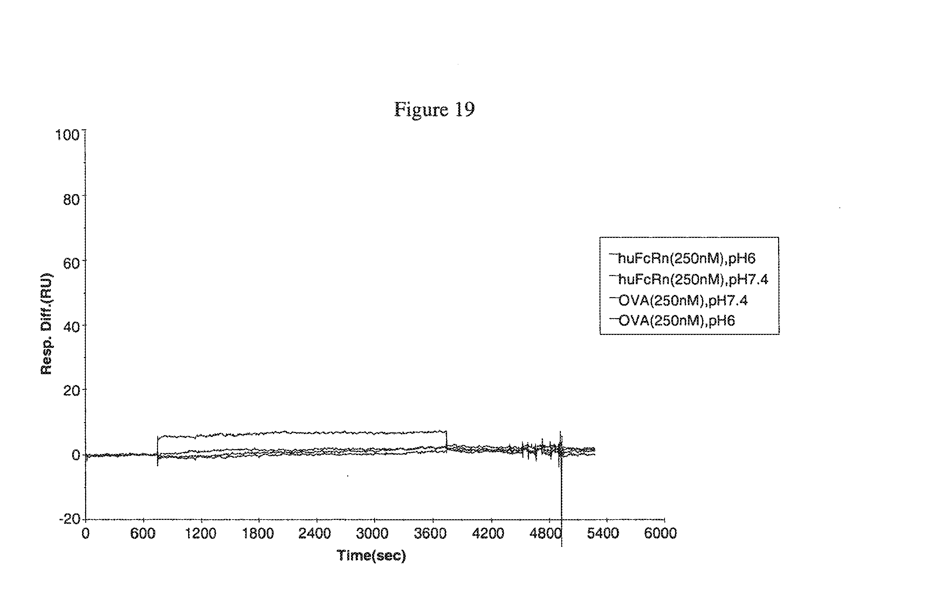

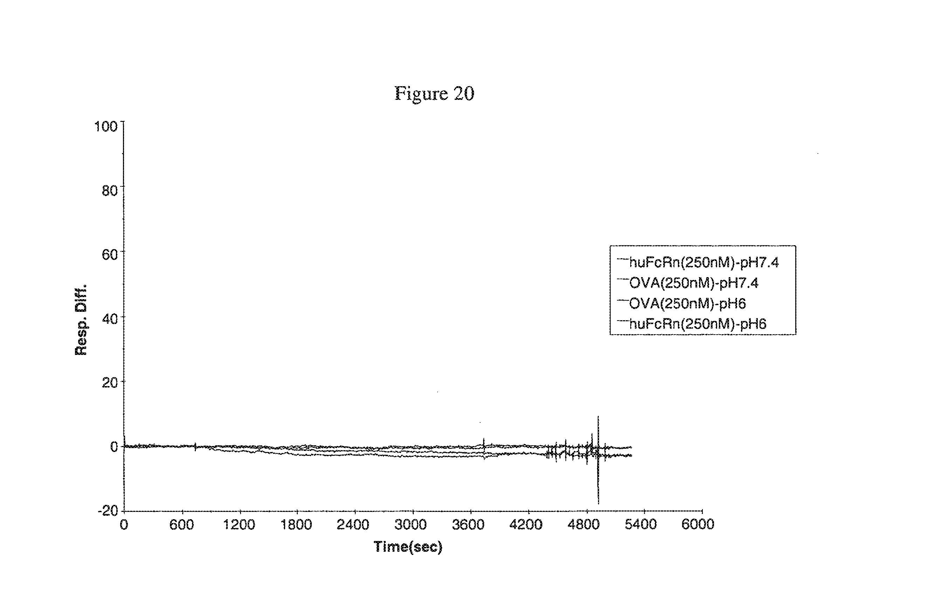

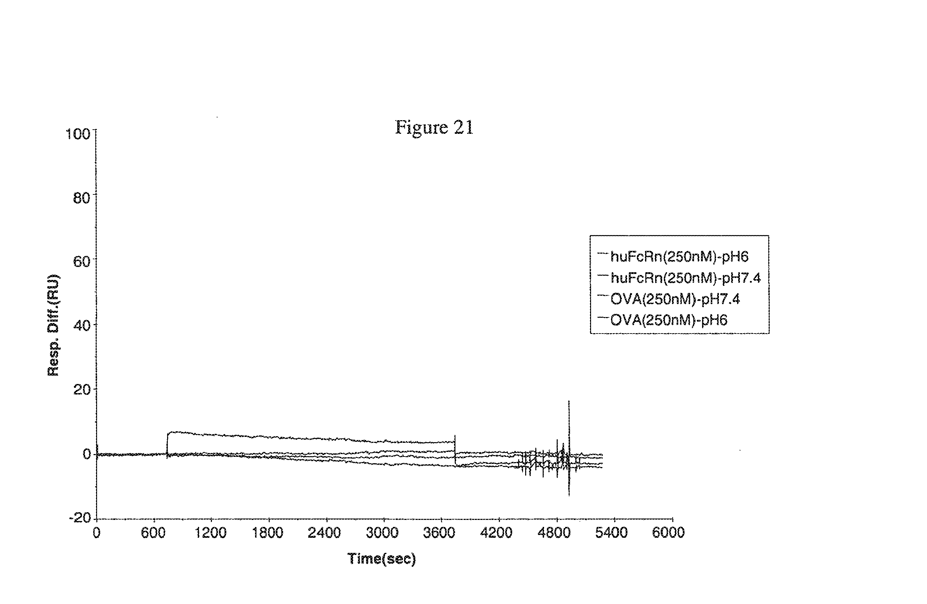

In this set of BIAcore analyses, MEDI-524 wild type and select amino acid substituted immunoglobulins were immobilized. IgGs were immobilized at 200 nM in 10 mM sodium acetate, pH 4.0, at 25°C. Immobilization levels typically ranged from approximately 2400 to 3400 RUs. In some cases, binding experiments were initially performed using 250 nM of human FcRn or ovalbumin in 50 mM PBS, pH 6.0, 0.05% Tween 20 or 50 mM PBS, pH 7.4, 0.05% Tween 20 for approximately 50 min. Three 1-min pulses of PBS pH 7.4 to regenerate the IgG surfaces. Results for select amino acid substituted immunoglobulins are shown in Figures 12, 14, 16, 17-21. Subsequent runs for Kd measurements used dilution series of 16 nM up to 2860 nM of human FcRn in 50 mM PBS, pH 6.0, 0.05% Tween 20 (results for select amino acid substituted immunoglobulins are shown in Figures 11, 13, 15). Data were typically collected for 50 min, followed by three 1-min pulses of PBS pH 7.4 to regenerate the IgG surfaces. Runs were analyzed using the BIAevaluation 4.1 software. Dissociation binding constants (K_{D}) were determined from a binding isotherm (steady-state model) by measuring the response at equilibrium (R_{eq}) for each concentration of human FcRn tested, after correction for nonspecific binding. See Table 3.

**Table 3: Dissociation constants of select substituted MEDI-524 immunoglobulins to human FcRn (pH 6.0)**

| **Amino acid substitution** | **Kd (µM)** |
|---|---|
| None (wt) | 2.68 |
| G385S+Q386I | 3.03 |
| R255V | 11.3 |
| D312I | 10.0 |
| L309N | 15.9 |
| L251S | ND* |
| N434L | ND* |
| M252T | ND* |
| S254W | ND* |
| S254R | ND* |

| | |
|---|---|
| * corresponding Kds were too high to be determined due to BIAcore's sensitivity limits. | |

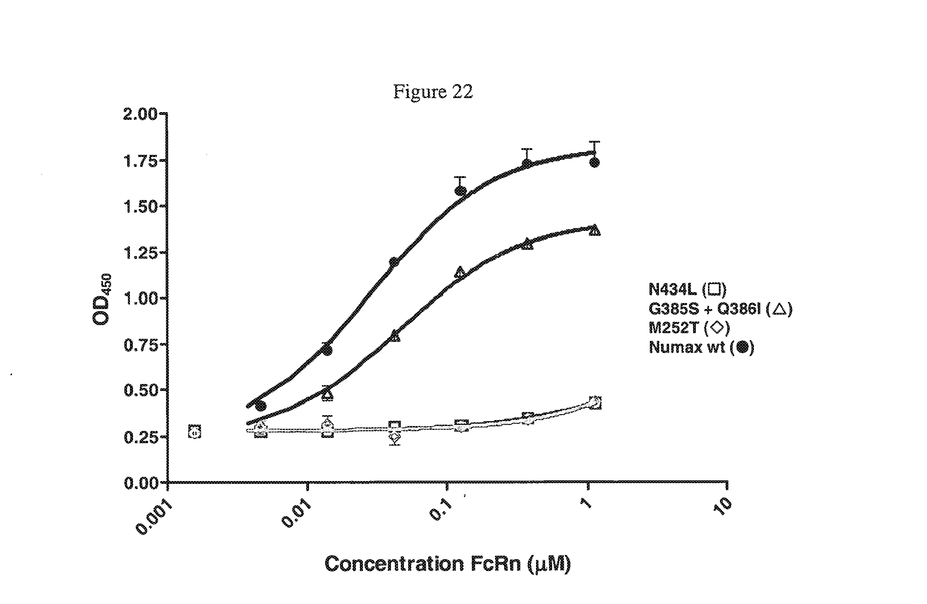

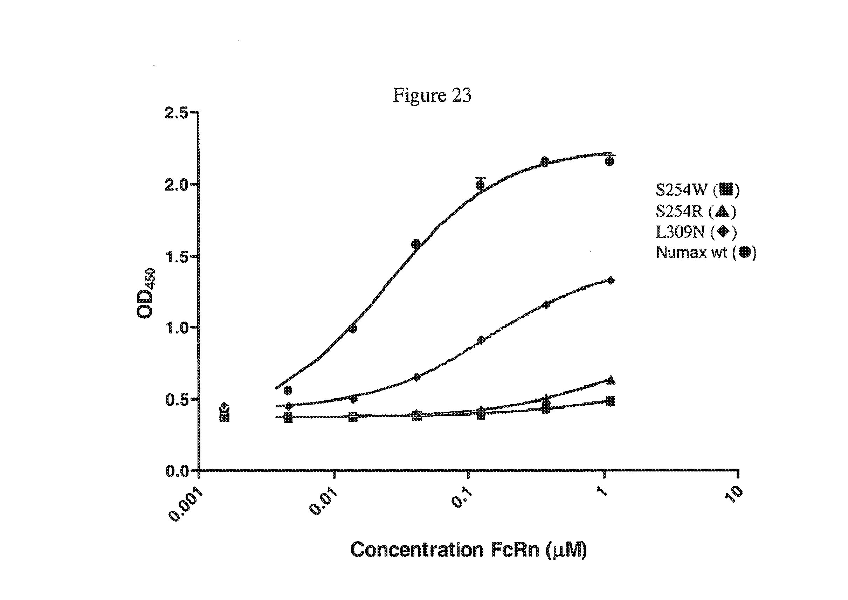

**ELISA.** Wild type MEDI-524 and MEDI-524 N434L, G385S + Q386I, M252T, S254W, S254R,and L309N immunoglobulin variants were coated on a Nunc MaxiSorp microtiter plate at a concentration of 1 µg/well in a carbonate coating buffer. Plates were washed with 50 mM sodium phosphate buffer, pH 5.8, containing 50 mM NaCl and 0.1% Tween-20 and blocked with 50% SuperBlock (Pierce), 50 mM sodium phosphate buffer, pH 5.8, and 50 mM NaCl. After subsequent washing, 3-fold dilutions of 50 µg/mL human FcRn in blocking buffer were added. Bound FcRn was detected with HRP-conjugated rabbit anti-human β2-microglobulin (AbCam). Results are shown in Figures 22-23.

The results of these BIAcore and ELISA analyses demonstrated that (i) S254R, S254W, M252T, L309N, R255V, D312I, N434L and L251S did not exhibit significant binding to human FcRn at neutral pH (7.4), and (ii) S254R, S254W, M252T, L309N, R255V, D312I, N434L and L251S exhibited varying degrees of deoptimized binding to human FcRn at pH 6.0. The affinity of the MEDI-524 and the MEDI-524substituted immunoglobulins can be summarized as follows:

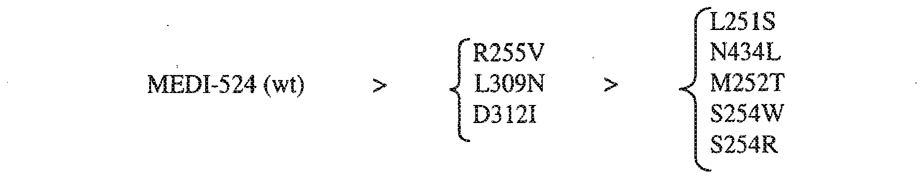

Those skilled in the art will recognize, or be able to ascertain using no more routine experimentation, many equivalents to the specific embodiments of the invention described herein. Such equivalents are intended to be encompassed by the following claims.

## Claims

1. A polypeptide comprising at least an FcRn binding portion of an Fc region of an immunoglobulin G (IgG) molecule wherein said polypeptide comprises a substitution at EU amino acid residue 312 with an isoleucine.

2. The polypeptide of claim 1 wherein the FcRn binding portion of the Fc region comprises from about amino acid residues 231-446 or from about amino acid residues 216-446 of an IgG molecule using EU numbering.

3. The polypeptide of claim 2 wherein the FcRn binding portion of the Fc region comprises an IgG molecule.

4. The polypeptide of any one of claims 1-3 wherein the IgG molecule is IgG subtype 1, or IgG subtype 2, or IgG subtype 3, or IgG subtype 4.

5. The polypeptide of any one of claims 1-4 comprising a toxic moiety.

6. The polypeptide of claim 5, wherein the toxic moiety is a radioactive element, or a cytostatic or cytocidal agent.

7. A fusion protein comprising:
a first polypeptide, wherein the first polypeptide comprises an FcRn binding portion of an Fc region of an immunoglobulin G (IgG) molecule, wherein said first polypeptide comprises a substitution at EU amino acid residue 312 with an isoleucine; and
a second polypeptide.

8. The fusion protein of claim 7 wherein the FcRn binding portion of the Fc region comprises from about amino acid residues 231-446 or from about amino acid residues 216-446 of an IgG molecule using EU numbering.

9. The fusion protein of claim 7 or 8 wherein the IgG molecule is IgG subtype 1, or IgG subtype 2, or IgG subtype 3, or IgG subtype 4.

10. The fusion protein of any one of claims 7-9 wherein the second polypeptide binds a tumor associated antigen or an epidermal growth factor receptor.

11. The fusion protein of claim 10 wherein the epidermal growth factor receptor is HER2.

12. The use of an effective amount of the polypeptide of claim 1, 5 or 6, in the manufacture of a medicament for treating a disease or disorder.

13. A method of diagnosing, monitoring, or determining the prognosis of a disease or disorder comprising:
administering the polypeptide of claim 1, wherein said polypeptide comprises a detectable substance, and wherein said polypeptide concentrates at sites afflicted by the disease or disorder; and
detecting the polypeptide.

14. The use of claim 12 or the method of claim 13, wherein the disease or disorder is cancer, infectious disease, or an inflammatory disorder.

15. A pharmaceutical composition comprising the polypeptide of claim 1 or the fusion protein of claim 7.


SEQUENCE LISTING

<110> MedImmune, LLC

<120> Molecules with reduced half-lives, compositions and uses thereof

<130> MED0004.2EP.DIV

<140> PCT/US2007/021991
<141> 2007-10-16

<150> 60/851,739
<151> 2006-10-16

<160> 77

<170> PatentIn version 3.3

<210> 1
<211> 44
<212> DNA
<213> Artificial

<220>
<223> Synthetic construct


<220>
<221> misc_feature
<222> (21)..(22)
<223> n is a, c, g, or t

<400> 1
ccgtggagtg ggagagcaat nnscagccgg agaacaacta caag 44


<210> 2
<211> 44
<212> DNA
<213> Artificial

<220>
<223> Synthetic Construct


<220>
<221> misc_feature
<222> (23)..(24)
<223> n is a, c, g, or t

<400> 2
cttgtagttg ttctccggct gsnnattgct ctcccactcc acgg 44


<210> 3
<211> 44
<212> DNA
<213> Artificial

<220>
<223> Synthetic construct

<400> 3
ccgtggagtg ggagagcaat tggcagccgg agaacaacta caag 44

<210>  4
<211>  44
<212>  DNA
<213>  Artificial

<220>
<223>  Synthetic construct

<400>  4
cttgtagttg ttctccggct gccaattgct ctcccactcc acgg                        44


<210>  5
<211>  42
<212>  DNA
<213>  Artificial

<220>
<223>  Synthetic Construct


<220>
<221>  misc_feature
<222>  (20)..(21)
<223>  n is a, c, g, or t

<400>  5
ggagtgggag agcaatgggn nsccggagaa caactacaag ac                          42


<210>  6
<211>  42
<212>  DNA
<213>  Artificial

<220>
<223>  Synthetic construct


<220>
<221>  misc_feature
<222>  (22)..(23)
<223>  n is a, c, g, or t

<400>  6
gtcttgtagt tgttctccgg snncccattg ctctcccact cc                          42


<210>  7
<211>  42
<212>  DNA
<213>  Artificial

<220>
<223>  Synthetic construct

<400>  7
ggagtgggag agcaatgggt tgccggagaa caactacaag ac                          42


<210>  8
<211>  42
<212>  DNA
<213>  Artificial

<220>
<223>  Synthetic construct

<400>  8
gtcttgtagt tgttctccgg caacccattg ctctcccact cc                          42


<210>  9
<211>  45
<212>  DNA
<213>  Artificial

<220>
<223>  Synthetic construct


<220>
<221>  misc_feature
<222>  (22)..(23)
<223>  n is a, c, g, or t

<400>  9
gagtgggaga gcaatgggca gnnsgagaac aactacaaga ccacg                       45


<210>  10
<211>  45
<212>  DNA
<213>  Artificial

<220>
<223>  Synthetic construct


<220>
<221>  misc_feature
<222>  (23)..(24)
<223>  n is a, c, g, or t

<400>  10
cgtggtcttg tagttgttct csnnctgccc attgctctcc cactc                       45


<210>  11
<211>  45
<212>  DNA
<213>  Artificial

<220>
<223>  Synthetic construct

<400>  11
gagtgggaga gcaatgggca gtgggagaac aactacaaga ccacg                       45


<210>  12
<211>  45
<212>  DNA
<213>  Artificial

<220>
<223>  Synthetic construct

<400>  12

cgtggtcttg tagttgttct cccactgccc attgctctcc cactc                    45

<210>  13
<211>  41
<212>  DNA
<213>  Artificial

<220>
<223>  Synthetic construct

<220>
<221>  misc_feature
<222>  (20)..(21)
<223>  n is a, c, g, or t

<400>  13
gagcaatggg cagccggagn nsaactacaa gaccacgcct c                          41

<210>  14
<211>  41
<212>  DNA
<213>  Artificial

<220>
<223>  Synthetic construct

<220>
<221>  misc_feature
<222>  (21)..(22)
<223>  n is a, c, g, or t

<400>  14
gaggcgtggt cttgtagtts nnctccggct gcccattgct c                          41

<210>  15
<211>  41
<212>  DNA
<213>  Artificial

<220>
<223>  Synthetic construct

<400>  15
gagcaatggg cagccggagt ggaactacaa gaccacgcct c                          41

<210>  16
<211>  41
<212>  DNA
<213>  Artificial

<220>
<223>  Synthetic construct

<400>  16
gaggcgtggt cttgtagttc cactccggct gcccattgct c                          41

<210>  17
<211>  46

<212>  DNA
<213>  Artificial

<220>
<223>  Synthetic construct


<220>
<221>  misc_feature
<222>  (21)..(22)
<223>  n is a, c, g, or t

<220>
<221>  misc_feature
<222>  (24)..(25)
<223>  n is a, c, g, or t

<400>  17
ccgtggagtg ggagagcaat nnsnnsccgg agaacaacta caagac                    46


<210>  18
<211>  46
<212>  DNA
<213>  Artificial

<220>
<223>  Synthetic construct


<220>
<221>  misc_feature
<222>  (22)..(23)
<223>  n is a, c, g, or t

<220>
<221>  misc_feature
<222>  (25)..(26)
<223>  n is a, c, g, or t

<400>  18
gtcttgtagt tgttctccgg snnsnnattg ctctcccact ccacgg                    46


<210>  19
<211>  46
<212>  DNA
<213>  Artificial

<220>
<223>  Synthetic construct

<400>  19
ccgtggagtg ggagagcaat tggtggccgg agaacaacta caagac                    46


<210>  20
<211>  46
<212>  DNA
<213>  Artificial

<220>
<223>  Synthetic construct

<400>  20

gtcttgtagt tgttctccgg ccaccaattg ctctcccact ccacgg                46

<210>  21
<211>  40
<212>  DNA
<213>  Artificial

<220>
<223>  Synthetic construct

<220>
<221>  misc_feature
<222>  (20)..(21)
<223>  n is a, c, g, or t

<400>  21
cgtgatgcat gaggctctgn nsaaccacta cacgcagaag                40

<210>  22
<211>  40
<212>  DNA
<213>  Artificial

<220>
<223>  Synthetic construct

<220>
<221>  misc_feature
<222>  (20)..(21)
<223>  n is a, c, g, or t

<400>  22
cttctgcgtg tagtggttsn ncagagcctc atgcatcacg                40

<210>  23
<211>  40
<212>  DNA
<213>  Artificial

<220>
<223>  Synthetic construct

<400>  23
cgtgatgcat gaggctctgt ggaaccacta cacgcagaag                40

<210>  24
<211>  40
<212>  DNA
<213>  Artificial

<220>
<223>  Synthettic construct

<400>  24
cttctgcgtg tagtggttcc acagagcctc atgcatcacg                40

<210>  25
<211>  43

<212>  DNA
<213>  Artificial

<220>
<223>  Synthetic construct


<220>
<221>  misc_feature
<222>  (20)..(21)
<223>  n is a, c, g, or t

<400>  25
gatgcatgag gctctgcacn nscactacac gcagaagagc ctc                    43


<210>  26
<211>  43
<212>  DNA
<213>  Artificial

<220>
<223>  Synthetic construct


<220>
<221>  misc_feature
<222>  (23)..(24)
<223>  n is a, c, g, or t

<400>  26
gaggctcttc tgcgtgtagt gsnngtgcag agcctcatgc atc                    43


<210>  27
<211>  43
<212>  DNA
<213>  Artificial

<220>
<223>  Synthetic construct

<400>  27
gatgcatgag gctctgcact ggcactacac gcagaagagc ctc                    43


<210>  28
<211>  43
<212>  DNA
<213>  Artificial

<220>
<223>  Synthetic construct

<400>  28
gaggctcttc tgcgtgtagt gccagtgcag agcctcatgc atc                    43


<210>  29
<211>  39
<212>  DNA
<213>  Artificial

<220>
<223>  Synthetic construct

<220>
<221>  misc_feature
<222>  (19)..(20)
<223>  n is a, c, g, or t

<400>  29
gaggctctgc acaaccacnn sacgcagaag agcctctcc                              39


<210>  30
<211>  39
<212>  DNA
<213>  Artificial

<220>
<223>  Synthetic construct


<220>
<221>  misc_feature
<222>  (20)..(21)
<223>  n is a, c, g, or t

<400>  30
ggagaggctc ttctgcgtsn ngtggttgtg cagagcctc                              39


<210>  31
<211>  39
<212>  DNA
<213>  Artificial

<220>
<223>  Synthetic construct

<400>  31
gaggctctgc acaaccactg gacgcagaag agcctctcc                              39


<210>  32
<211>  39
<212>  DNA
<213>  Artificial

<220>
<223>  Synthetic construct

<400>  32
ggagaggctc ttctgcgtcc agtggttgtg cagagcctc                              39


<210>  33
<211>  41
<212>  DNA
<213>  Artificial

<220>
<223>  Synthetic construct


<220>
<221>  misc_feature
<222>  (20)..(20)

<223>  n is a, c, g, or t

<400>  33
cccaaaaccc aaggacaccn ssatgatctc ccggacccct g                41


<210>  34
<211>  41
<212>  DNA
<213>  Artificial

<220>
<223>  Synthetic construct


<220>
<221>  misc_feature
<222>  (22)..(22)
<223>  n is a, c, g, or t

<400>  34
caggggtccg ggagatcats snggtgtcct tgggttttgg g                41


<210>  35
<211>  41
<212>  DNA
<213>  Artificial

<220>
<223>  Synthetic construct

<400>  35
cccaaaaccc aaggacacct ggatgatctc ccggacccct g                41


<210>  36
<211>  41
<212>  DNA
<213>  Artificial

<220>
<223>  Synthetic construct

<400>  36
caggggtccg ggagatcatc caggtgtcct tgggttttgg g                41


<210>  37
<211>  41
<212>  DNA
<213>  Artificial

<220>
<223>  Synthetic construct


<220>
<221>  misc_feature
<222>  (21)..(21)
<223>  n is a, c, g, or t

<400>  37
caaaacccaa ggacaccctc nssatctccc ggacccctga g                41

<210>  38
<211>  41
<212>  DNA
<213>  Artificial

<220>
<223>  Synthetic construct


<220>
<221>  misc_feature
<222>  (21)..(21)
<223>  n is a, c, g, or t

<400>  38
ctcaggggtc cgggagatss ngagggtgtc cttgggtttt g                41


<210>  39
<211>  41
<212>  DNA
<213>  Artificial

<220>
<223>  Synthetic construct

<400>  39
caaaacccaa ggacaccctc tggatctccc ggacccctga g                41


<210>  40
<211>  41
<212>  DNA
<213>  Artificial

<220>
<223>  Synthetic construct

<400>  40
ctcaggggtc cgggagatcc agagggtgtc cttgggtttt g                41


<210>  41
<211>  42
<212>  DNA
<213>  Artificial

<220>
<223>  Synthetic consntruct


<220>
<221>  misc_feature
<222>  (20)..(20)
<223>  n is a, c, g, or t

<400>  41
caaggacacc ctcatgatcn sscggacccc tgaggtcaca tg               42


<210>  42
<211>  42
<212>  DNA
<213>  Artificial

<220>
<223>  Synthetic construct


<220>
<221>  misc_feature
<222>  (23)..(23)
<223>  n is a, c, g, or t

<400>  42
catgtgacct caggggtccg ssngatcatg agggtgtcct tg                          42


<210>  43
<211>  42
<212>  DNA
<213>  Artificial

<220>
<223>  Synthetic construct

<400>  43
caaggacacc ctcatgatct ggcggacccc tgaggtcaca tg                          42


<210>  44
<211>  42
<212>  DNA
<213>  Artificial

<220>
<223>  Synthetic construct

<400>  44
catgtgacct caggggtccg ccagatcatg agggtgtcct tg                          42


<210>  45
<211>  41
<212>  DNA
<213>  Artificial

<220>
<223>  Synthetic construct


<220>
<221>  misc_feature
<222>  (20)..(21)
<223>  n is a, c, g, or t

<400>  45
ggacaccctc atgatctccn nsacccctga ggtcacatgc g                           41


<210>  46
<211>  41
<212>  DNA
<213>  Artificial

<220>
<223>  Synthetic construct

<220>
<221>  misc_feature
<222>  (21)..(22)
<223>  n is a, c, g, or t

<400>  46
cgcatgtgac ctcaggggts nnggagatca tgagggtgtc c                          41


<210>  47
<211>  41
<212>  DNA
<213>  Artificial

<220>
<223>  Synthetic construct

<400>  47
ggacaccctc atgatctcct ggacccctga ggtcacatgc g                          41


<210>  48
<211>  41
<212>  DNA
<213>  Artificial

<220>
<223>  Synthetic construct

<400>  48
cgcatgtgac ctcaggggtc caggagatca tgagggtgtc c                          41


<210>  49
<211>  40
<212>  DNA
<213>  Artificial

<220>
<223>  Synthetic construct


<220>
<221>  misc_feature
<222>  (20)..(21)
<223>  n is a, c, g, or t

<400>  49
caccctcatg atctcccggn nscctgaggt cacatgcgtg                            40


<210>  50
<211>  40
<212>  DNA
<213>  Artificial

<220>
<223>  Synthetic construct


<220>
<221>  misc_feature
<222>  (20)..(21)
<223>  n is a, c, g, or t

<400>  50
cacgcatgtg acctcaggsn nccgggagat catgagggtg                              40


<210>  51
<211>  40
<212>  DNA
<213>  Artificial

<220>
<223>  Synthetic construct

<400>  51
caccctcatg atctcccggt ggcctgaggt cacatgcgtg                              40


<210>  52
<211>  40
<212>  DNA
<213>  Artificial

<220>
<223>  Synthetic construct

<400>  52
cacgcatgtg acctcaggcc accgggagat catgagggtg                              40


<210>  53
<211>  51
<212>  DNA
<213>  Artificial

<220>
<223>  Synthetic construct


<220>
<221>  misc_feature
<222>  (22)..(23)
<223>  n is a, c, g, or t

<220>
<221>  misc_feature
<222>  (28)..(29)
<223>  n is a, c, g, or t

<400>  53
gtgatgcatg aggctctgca cnnscacnns acgcagaaga gcctctccct g                 51


<210>  54
<211>  51
<212>  DNA
<213>  Artificial

<220>
<223>  Synthetic construct


<220>
<221>  misc_feature
<222>  (23)..(24)
<223>  n is a, c, g, or t

<220>
<221>  misc_feature
<222>  (29)..(30)
<223>  n is a, c, g, or t

<400>  54
cagggagagg ctcttctgcg tsnngtgsnn gtgcagagcc tcatgcatca c          51


<210>  55
<211>  51
<212>  DNA
<213>  Artificial

<220>
<223>  Synthetic construct

<400>  55
gtgatgcatg aggctctgca ctggcactgg acgcagaaga gcctctccct g          51


<210>  56
<211>  51
<212>  DNA
<213>  Artificial

<220>
<223>  Synthetic construct

<400>  56
cagggagagg ctcttctgcg tccagtgcca gtgcagagcc tcatgcatca c          51


<210>  57
<211>  35
<212>  DNA
<213>  Artificial

<220>
<223>  Synthetic construct


<220>
<221>  misc_feature
<222>  (17)..(18)
<223>  n is a, c, g, or t

<400>  57
ggtcagcgtc ctcaccnnsc tgcaccagga ctggc                            35


<210>  58
<211>  35
<212>  DNA
<213>  Artificial

<220>
<223>  Synthetic construct


<220>
<221>  misc_feature
<222>  (18)..(19)
<223>  n is a, c, g, or t

<400>  58
gccagtcctg gtgcagsnng gtgaggacgc tgacc                                35


<210>  59
<211>  35
<212>  DNA
<213>  Artificial

<220>
<223>  Synthetic construct

<400>  59
ggtcagcgtc ctcacctggc tgcaccagga ctggc                                35


<210>  60
<211>  35
<212>  DNA
<213>  Artificial

<220>
<223>  Synthetic construct

<400>  60
gccagtcctg gtgcagccag gtgaggacgc tgacc                                35


<210>  61
<211>  40
<212>  DNA
<213>  Artificial

<220>
<223>  Synthetic construct


<220>
<221>  misc_feature
<222>  (19)..(20)
<223>  n is a, c, g, or t

<400>  61
gtcagcgtcc tcaccgtcnn scaccaggac tggctgaatg                           40


<210>  62
<211>  40
<212>  DNA
<213>  Artificial

<220>
<223>  Synthetic construct


<220>
<221>  misc_feature
<222>  (21)..(22)
<223>  n is a, c, g, or t

<400>  62
cattcagcca gtcctggtgs nngacggtga ggacgctgac                           40


<210>  63

<211>  40
<212>  DNA
<213>  Artificial

<220>
<223>  Synthetic construct

<400>  63
gtcagcgtcc tcaccgtctg gcaccaggac tggctgaatg                              40


<210>  64
<211>  40
<212>  DNA
<213>  Artificial

<220>
<223>  Synthettic construct

<400>  64
cattcagcca gtcctggtgc cagacggtga ggacgctgac                              40


<210>  65
<211>  39
<212>  DNA
<213>  Artificial

<220>
<223>  Synthetic construct


<220>
<221>  misc_feature
<222>  (19)..(20)
<223>  n is a, c, g, or t

<400>  65
gtcctcaccg tcctgcacnn sgactggctg aatggcaag                               39


<210>  66
<211>  39
<212>  DNA
<213>  Artificial

<220>
<223>  Synthetic construct


<220>
<221>  misc_feature
<222>  (20)..(21)
<223>  n is a, c, g, or t

<400>  66
cttgccattc agccagtcsn ngtgcaggac ggtgaggac                               39


<210>  67
<211>  39
<212>  DNA
<213>  Artificial

<220>

<223>  Synthetic construct

<400>  67
gtcctcaccg tcctgcactg ggactggctg aatggcaag                              39


<210>  68
<211>  39
<212>  DNA
<213>  Artificial

<220>
<223>  Synthetic construct

<400>  68
cttgccattc agccagtccc agtgcaggac ggtgaggac                              39


<210>  69
<211>  39
<212>  DNA
<213>  Artificial

<220>
<223>  Synthetic construct


<220>
<221>  misc_feature
<222>  (19)..(20)
<223>  n is a, c, g, or t

<400>  69
ctcaccgtcc tgcaccagnn stggctgaat ggcaaggag                              39


<210>  70
<211>  39
<212>  DNA
<213>  Artificial

<220>
<223>  Synthetic construct


<220>
<221>  misc_feature
<222>  (20)..(21)
<223>  n is a, c, g, or t

<400>  70
ctccttgcca ttcagccasn nctggtgcag gacggtgag                              39


<210>  71
<211>  39
<212>  DNA
<213>  Artificial

<220>
<223>  Synthetic construct

<400>  71
ctcaccgtcc tgcaccagtg gtggctgaat ggcaaggag                              39

<210>  72
<211>  39
<212>  DNA
<213>  Artificial

<220>
<223>  Synthetic construct

<400>  72
ctccttgcca ttcagccacc actggtgcag gacggtgag                              39


<210>  73
<211>  51
<212>  DNA
<213>  Artificial

<220>
<223>  Synthetic construct


<220>
<221>  misc_feature
<222>  (22)..(23)
<223>  n is a, c, g, or t

<220>
<221>  misc_feature
<222>  (28)..(29)
<223>  n is a, c, g, or t

<400>  73
gtggtcagcg tcctcaccgt cnnscacnns gactggctga atggcaagga g                51


<210>  74
<211>  51
<212>  DNA
<213>  Artificial

<220>
<223>  Synthetic construct


<220>
<221>  misc_feature
<222>  (23)..(24)
<223>  n is a, c, g, or t

<220>
<221>  misc_feature
<222>  (29)..(30)
<223>  n is a, c, g, or t

<400>  74
ctccttgcca ttcagccagt csnngtgsnn gacggtgagg acgctgacca c                51


<210>  75
<211>  51
<212>  DNA
<213>  Artificial

<220>

<223>  Synthetic construct

<400>  75
gtggtcagcg tcctcaccgt ctggcactgg gactggctga atggcaagga g          51

<210>  76
<211>  51
<212>  DNA
<213>  Artificial

<220>
<223>  Synthetic construct

<400>  76
ctccttgcca ttcagccagt cccagtgcca gacggtgagg acgctgacca c          51

<210>  77
<211>  330
<212>  PRT
<213>  Artificial

<220>
<223>  Synthetic construct

<400>  77

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp

145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330